# EUROPEAN PATENT APPLICATION

(11) **EP 4 082 572 A1**
(43) Date of publication of application: **02.11.2022**
(21) Application number: 20907897.1
(22) Date of filing: 25.12.2020
(51) Int. Cl.: A61K 39/395, A61P 35/00, C07K 16/28

(54) **ANTI-CTLA-4 MONOCLONAL ANTIBODY, PREPARATION METHOD THEREFOR, AND APPLICATION THEREOF**

(30) Priority: 25.12.2019 CN 201911363904; 20.05.2020 CN 202010430026
(71) Applicant: Bio-Thera Solutions, Ltd., Guangzhou, Guangdong 510530 (CN)
(72) Inventor: SU, Ziqi, Guangzhou, Guangdong 510530 (CN); QIN, Chao, Guangzhou, Guangdong 510530 (CN); YU, Jin-Chen, Guangzhou, Guangdong 510530 (CN); HUANG, Dandan, Guangzhou, Guangdong 510530 (CN); WU, Yong, Guangzhou, Guangdong 510530 (CN); CAO, Di, Guangzhou, Guangdong 510530 (CN); LIU, Cuihua, Guangzhou, Guangdong 510530 (CN); SONG, Shuqiang, Guangzhou, Guangdong 510530 (CN); LI, Shengfeng, Guangzhou, Guangdong 510530 (CN)
(74) Representative: dompatent von Kreisler Selting Werner - Partnerschaft von Patent- und Rechtsanwälten mbB
(86) International application number: PCT/CN2020/139205
(87) International publication number: WO 2021/129775

(57) **Abstract**

The present invention discloses an anti-CTLA-4 monoclonal antibody and a preparation and use thereof. The anti-CTLA-4 antibody of the present invention has the advantages of lower toxicity and better activity; in addition, the anti-CTLA-4 antibody of the present invention also has a low content of high mannose glycoforms and/or a low content of sialylated glycoforms, which helps to prolong the half-life of the antibody and/or reduce immunogenicity. In particular, a formulation comprising the antibody disclosed by the present invention has good stability.

## Description

### TECHNICAL FIELD

The present invention belongs to the technical field of bio-pharmaceuticals, and relates to an anti-CTLA-4 monoclonal antibody and a preparation method therefor and use thereof.

### BACKGROUND

The immune system is an important defense against the development and progression of cancers. Immunotherapy, such as adoptive transfer of IL-2 and autologous tumor-infiltrating lymphocytes, can induce a sustained tumor response in patient subgroups, thereby allowing long-term survival in patients with poor prognosis (Atkins MB, et al (1999), J Clin Oncol. 17(7): 2105-2116). With further understanding of the importance of the initiation and effector phases of anti-tumor immunity, checkpoint blockade-based therapies have recently emerged to provide lasting benefit to patients and appear to be applicable to a wide range of malignancies.

The immune system is tightly regulated in response to the appropriate antigen without responding to itself (Bretscher P, Cohn M. (1970), Science. 169(3950):1042-1049). T cells are one of the key mediators of the immune response, require co-stimulation for activation, and can be inactivated by inhibitory signals (Sharpe AH, Abbas AK. (2006), N Engl J Med. 355(10):973-975). Cytotoxic T lymphocyte antigen-4 (CTLA-4) is a key inhibitory receptor that affects T cell function and plays a key role in the initiation phase of the immune response (Scalapino KJ1, Daikh DI. (2008), Immunol Rev. 223:143-155). When antigens are transported to T Cell Receptors (TCRs) by MHC class I or II antigen presenting cells, signals on the TCR are amplified and counteracted by co-stimulatory molecules. Binding of CD28 on T cells to B7-1 (CD80) and B7-2 (CD86) on antigen presenting cells signals a requirement for full T cell activation. This CD28/B7 binding results in increased production of interleukin 2 and other stimulatory cytokines, increased metabolism, promotion of cell cycle progression, upregulation of cell survival genes, and ultimately proliferation and differentiation of T cells. CTLA-4 is transported and expressed on the surface of T cells after CD28 binding occurs and results in T cell proliferation (Linsley PS, et al. (1996), Immunity. 4(6):535-543). The stronger the activation signals through the TCR are, the more CTLA-4 is transported and expressed. When CTLA-4 is on the cell surface, CTLA-4 inhibitory signals are transmitted (Egen JG, Allison JP. (2002), Immunity. 16(1):23-25). CTLA-4 has a higher affinity for B7 than CD28 and can block further co-activation (Krummel MF, Allison JP. (1995), J Exp Med. 182(2):458-465). Furthermore, CTLA-4-expressing cells are able to capture and degrade B7-1 and B7-2 by endocytosis (Qureshi OS, et al. (2011), Science. 332(6029):600-603).

In addition to CTLA-4, other stimulatory or inhibitory receptors and ligands that can modulate T cell responses have been reported. The stimulatory receptors include inducible T cell costimulator (ICOS) (Tremble LF, et al. (2018), Cancer Lett. 420:109-115), glucocorticoid-induced TNF receptor (GITR) (Schaer DA, et al. (2013), Cancer Immunol Res. 1(5):320-331), CD27 (Buchan SL, et al. (2018), Blood. 131(1):39-48), 4-1BB (Eun SY, et al. (2015), J Immunol. 194(1):134-141), CD40 (Richman LP, Vonderheide RH. (2014), Cancer Immunol Res. 2(1):19-26), and DNAX accessory molecule-1 (DNAM-1) (Kearney CJ, et al. (2016), Oncoimmunology. 5(8):E1196308); and the inhibitory receptors include programmed cell death receptor-1 (PD-1) (Dong H, et al. (1999), Nat Med. 5(12):1365-1369), lymphocyte-activation gene 3 (LAG-3) (Di CE, et al. (2005), J Pathol. 205:82-91), T-cell immunoglobulin and mucin domain 3 (TIM3) (Sanchez-Fueyo A, et al. (2003), Nat Immunol. 4(11):1093-1101), T-cell activated V-domain Ig inhibitor (VISTA) (Lines JL, et al. (2014), Cancer Res. 74(7):1924-1932), CD47 (Liu X, et al. (2015), Nat Med. 21(10):1209-1215), indoleamine 2,3-dioxygenase (IDO) (Friberg M, et al. (2002), Cancer. 101:151-155), CD94/NKG2A (Muntasell A, et al. (2017), Curr Opin Immunol. 45:73-81), Killer-cell immunoglobulin-like receptor (KIR) (Tu MM, et al. (2016), Front Immunol. 7:116), and T cell immunoglobulin and ITIM domain (TIGIT) (Johnston RJ, et al. (2014), Cancer Cell. 26:923-937). These stimulatory receptors and ligands, i.e., "immune checkpoints" that are currently hotly discussed in the research field, play a crucial role in the self-regulation of the immune system, so that the function of the immune system is kept within a normal range without being over-activated. In cancer organisms, the immune response can be modulated by targeting stimulatory receptors with agonists or blocking inhibitory receptors with inhibitors, so that the immune system can recognize and attack tumor cells to achieve the therapeutic effect. For the above immune checkpoints, some therapeutic drugs in clinical or on the market (Elad S, et al. (2014), Chin J Cancer. 33(9): 434-444) have been developed, wherein CTLA-4 blocker is the first monoclonal antibody drug applied in clinical cancer treatment.

Currently, the known anti-CTLA-4 monoclonal antibodies are lpilimumab (Yervoy^{®}) from Bristol-Myers Squibb Company and Tremelimumab from AstraZeneca Company which is still in clinical stage. The two anti-CTLA-4 monoclonal antibodies all have a mechanism of enhancing the proliferation response of T cells by blocking CTLA-4 binding to B7 and liberating B7 protein to bind to CD28. Blockade of the CTLA-4/B7 interaction in several preclinical established mouse models has been shown to reject transplantable tumors, including colon cancer, prostate cancer, lymphoma and renal cancer (Korman AJ, et al. (2006), Adv Immunol. 90:297-339). However, when this mechanism of action is generalized to the human body, it only provides limited clinical benefit to a small fraction of patients treated. Therefore, it is believed that there should be an unknown mechanism associated with host-dependent factors (Romano E, et al. (2015), Proc Natl Acad Sci USA. 112(19):6140-6145).

### SUMMARY OF THE INVENTION

Based on the above background, there is a need to provide a novel anti-CTLA-4 monoclonal antibody drug with lower toxicity and better activity. The above purpose of the present invention is implemented by the following technical means.

The present invention provides an anti-CTLA-4 antibody with a low content of high mannose glycoform and/or a low content of sialylated glycoform, which helps to prolong the half-life of the antibody and/or reduce immunogenicity. In some embodiments, the anti-CTLA-4 antibody has an enhanced ADCC effect, using for targeting a cell or tissue of a patient to increase the anti-tumor effect of the drug.

Specifically, in one aspect, the present invention provides a monoclonal antibody against CTLA-4 comprising:
(1) a heavy chain CDR1 (HCDR1) comprising or consisting of an amino acid sequence set forth in SEQ ID NO: 12, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 12, or an amino acid sequence having one or more (preferably about 1, about 2 or about 3) conservative amino acid mutations (preferably substitutions, insertions or deletions) compared to SEQ ID NO: 12,
   a heavy chain CDR2 (HCDR2) comprising or consisting of an amino acid sequence set forth in SEQ ID NO: 13, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 13, or an amino acid sequence having one or more (preferably about 1, about 2 or about 3) conservative amino acid mutations (preferably substitutions, insertions or deletions) compared to SEQ ID NO: 13,
   a heavy chain CDR3 (HCDR3) comprising or consisting of an amino acid sequence set forth in SEQ ID NO: 14, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 14, or an amino acid sequence having one or more (preferably about 1, about 2 or about 3) conservative amino acid mutations (preferably substitutions, insertions or deletions) compared to SEQ ID NO: 14,
   a light chain CDR1 (LCDR1) comprising or consisting of an amino acid sequence set forth in SEQ ID NO: 15, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 15, or an amino acid sequence having one or more (preferably 1, 2 or 3) conservative amino acid mutations (preferably substitutions, insertions or deletions) compared to SEQ ID NO: 15,
   a light chain CDR2 (LCDR2) comprising or consisting of an amino acid sequence set forth in SEQ ID NO: 16, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 16, or an amino acid sequence having one or more (preferably about 1, about 2 or about 3) conservative amino acid mutations (preferably substitutions, insertions or deletions) compared to SEQ ID NO: 16, and
   a light chain CDR3 (LCDR3) comprising or consisting of an amino acid sequence set forth in SEQ ID NO: 17, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 17, or an amino acid sequence having one or more (preferably about 1, about 2 or about 3) conservative amino acid mutations (preferably substitutions, insertions or deletions) compared to SEQ ID NO: 17;
      or
(2) a heavy chain variable region (VH) comprising or consisting of an amino acid sequence set forth in SEQ ID NO: 18, an amino acid sequence having at least about 80%, preferably at least about 85%, about 90%, about 91%, about 92%, about 93%, about 94%, about 95%, about 96%, about 97%, about 98% or about 99% (or a range between any two values (inclusive) or any value therein) sequence identity to SEQ ID NO: 18, or an amino acid sequence having one or more (preferably about 1, about 2 or about 3) conservative amino acid mutations (preferably substitutions, insertions or deletions) compared to SEQ ID NO: 18, and
   a light chain variable region (VL) comprising or consisting of an amino acid sequence set forth in SEQ ID NO: 19, an amino acid sequence having at least about 80%, preferably at least about 85%, about 90%, about 91%, about 92%, about 93%, about 94%, about 95%, about 96%, about 97%, about 98% or about 99% (or a range between any two values (inclusive) or any value therein) sequence identity to SEQ ID NO: 19, or an amino acid sequence having one or more (preferably about 1, about 2 or about 3) conservative amino acid mutations (preferably substitutions, insertions or deletions) compared to SEQ ID NO: 19;
   the monoclonal antibody has a reduced fucosylation level.

In a specific embodiment, the monoclonal antibody comprises a heavy chain and a light chain, wherein the heavy chain comprises or consists of an amino acid sequence set forth in SEQ ID NO: 1, or an amino acid sequence having at least about 80%, preferably at least about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% (or a range between any two values (inclusive) or any value therein) homology to the amino acid sequence set forth in SEQ ID NO: 1;
the light chain comprises or consists of an amino acid sequence set forth in SEQ ID NO: 2, or an amino acid sequence having at least about 80%, preferably at least about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% (or a range between any two values (inclusive) or any value therein) homology to the amino acid sequence set forth in SEQ ID NO: 2.

In a specific embodiment, the heavy chain is encoded by a nucleotide sequence set forth in SEQ ID NO: 3 or a nucleotide sequence having at least about 80%, preferably at least about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% (or a range between any two values (inclusive) or any value therein) homology to the nucleotide sequence set forth in SEQ ID NO: 3, and the light chain is encoded by the nucleotide sequence set forth in SEQ ID NO: 4 or a nucleotide sequence having at least about 80%, preferably at least about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% (or a range between any two values (inclusive) or any value therein) homology to the nucleotide sequence set forth in SEQ ID NO: 4.

In a specific embodiment, the fucosylation level is 0%-20%, e.g., about 0%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19% or about 20%, or a range between any two values (inclusive) or any value therein.

In some embodiments, the Fc region of the monoclonal antibody has high mannose glycoforms with a total amount of < 5% and/or sialylated glycoforms with a total amount of < 3%. In some embodiments, the Fc region of the monoclonal antibody has high mannose glycoforms with a total amount of about 0.1%, about 0.3%, about 0.9%, about 1.18%, about 1.7%, about 2.6%, about 3.3%, about 4.1%, about 4.9%, about 4.99%, or a range between any two values (inclusive) or any value therein. In some embodiments, the Fc region of the monoclonal antibody has sialylated glycoforms with a total amount of about 0.1%, about 0.2%, about 0.36%, about 0.8%, about 1.5%, about 2.2%, about 2.7%, about 2.9%, 2.99%, or a range between any two values (inclusive) or any value therein. In some embodiments, the monoclonal antibody is IgG1, and the CH2 region of the monoclonal antibody has high mannose glycoforms with a total amount of < 5% and/or sialylated glycoforms with a total amount of < 3%.

In some embodiments, the Fc region of the monoclonal antibody has high mannose glycoforms with a total amount of < 3% and/or sialylated glycoforms with a total amount of < 2%. In some embodiments, the monoclonal antibody is IgG1, and the CH2 region of the monoclonal antibody has high mannose glycoforms with a total amount of < 3% and/or sialylated glycoforms with a total amount of < 2%.

In some embodiments, the Fc region of the monoclonal antibody has high mannose glycoforms with a total amount of < 2% and/or sialylated glycoforms with a total amount of < 1%. In some embodiments, the monoclonal antibody is IgG1, and the CH2 region of the monoclonal antibody has high mannose glycoforms with a total amount of < 2% and/or sialylated glycoforms with a total amount of < 1%.

In some embodiments, the Fc region of the monoclonal antibody has a fucosylation level of 0%-10%. In some embodiments, the monoclonal antibody is IgG1, and the CH2 region of the monoclonal antibody has a fucosylation level of 0%-10%.

In some embodiments, the Fc region of the monoclonal antibody has a fucosylation level of 0%-5%. In some embodiments, the Fc region of the monoclonal antibody has a fucosylation level of about 0, about 0.1%), about 0.3%, about 0.4%, about 0.6%, about 1.3%, about 1.9%, about 2.2%, about 2.8%, about 3.3%, about 3.7%, about 4.1%, about 4.5%, about 5%, or a range between any two values (inclusive) or any value therein. In some embodiments, the monoclonal antibody is IgG1, and the CH2 region of the monoclonal antibody has a fucosylation level of 0%-5%.

In some embodiments, the fucosylation level is achieved by:
(1) expressing a monoclonal antibody comprising a heavy chain CDR1 set forth in SEQ ID NO: 12, a heavy chain CDR2 set forth in SEQ ID NO: 13, a heavy chain CDR3 set forth in SEQ ID NO: 14, a light chain CDR1 set forth in SEQ ID NO: 15, a light chain CDR2 set forth in SEQ ID NO: 16 and a light chain CDR3 set forth in SEQ ID NO: 17 in a CHO host cell in which the FUT8 gene (α-(1,6)-fucosyltransferase gene) is knocked down or knocked out, wherein the monoclonal antibody has a low content of high mannose glycoforms, sialylated glycoforms and/or fucose glycoforms compared to a monoclonal antibody expressed in a normal CHO host cell;
(2) expressing a monoclonal antibody comprising a heavy chain CDR1 set forth in SEQ ID NO: 12, a heavy chain CDR2 set forth in SEQ ID NO: 13, a heavy chain CDR3 set forth in SEQ ID NO: 14, a light chain CDR1 set forth in SEQ ID NO: 15, a light chain CDR2 set forth in SEQ ID NO: 16 and a light chain CDR3 set forth in SEQ ID NO: 17 in a CHO-DG44 cell in which GDP-mannose 4,6-dehydratase (GMD) is knocked down, wherein the monoclonal antibody has a low content of high mannose glycoforms, sialylated glycoforms and/or fucose glycoforms compared to a monoclonal antibody expressed in a normal CHO-DG44 host cell; or
(3) expressing a monoclonal antibody comprising a heavy chain CDR1 set forth in SEQ ID NO: 12, a heavy chain CDR2 set forth in SEQ ID NO: 13, a heavy chain CDR3 set forth in SEQ ID NO: 14, a light chain CDR1 set forth in SEQ ID NO: 15, a light chain CDR2 set forth in SEQ ID NO: 16 and a light chain CDR3 set forth in SEQ ID NO: 17 in a CHO cell, and then defucosylating with a fucosyltransferase inhibitor such as 2F-peracetyl-fucose, wherein the monoclonal antibody has a low content of high mannose glycoforms, sialylated glycoforms and/or fucose glycoforms compared to a monoclonal antibody without defucosylating with the fucosyltransferase inhibitor such as 2F-peracetyl-fucose.

In some embodiments, the monoclonal antibody is expressed by a cell line in which an α-(1,6)-fucosyltransferase gene is knocked out, such as the CHO-BAT-KF cell line disclosed in PCT/CN2018/100008.

In another aspect, the present invention provides a pharmaceutical composition or fusion protein comprising the monoclonal antibody of the present invention.

In some embodiments, the pharmaceutical composition is in a form suitable for administration by oral, injection, infusion, parenteral, intravenous, mucosal, sublingual, intramuscular, intradermal, nasal, intraperitoneal, intra-arterial, subcutaneous absorption. In some embodiments, the pharmaceutical composition is in a form suitable for systemic administration.

In another aspect, the present invention provides an antibody conjugate comprising the monoclonal antibody of the present invention and a conjugated moiety coupled thereto, wherein the conjugated moiety is a purification tag (e.g., a His tag), a cytotoxic agent, a moiety that prolongs the half-life of the monoclonal antibody or a detectable label, preferably the conjugated moiety is a radioisotope, a chemiluminescent agent, an enzyme, serum albumin (e.g., human or murine serum albumin) or polyethylene glycol.

In some embodiments, the anti-CTLA-4 antibody of the present invention is a monovalent antibody or a multivalent antibody. In another aspect, the present invention provides a multispecific antibody (e.g., a bispecific antibody) comprising the monoclonal antibody of the present invention, and an antibody or an antigen-binding fragment against another antigen and/or another antigenic epitope. In yet another aspect, the present invention provides a kit comprising the monoclonal antibody, the antibody conjugate, the multispecific antibody or the fusion protein of the present invention.

In a specific embodiment, the kit further comprises a second antibody, a cytotoxic agent, a chemotherapeutic agent and a radiotherapeutic agent, wherein the second antibody specifically recognizes the monoclonal antibody; optionally, the second antibody further comprises a detectable label, such as fluorescein, a metal ion, biotin, a radioisotope, a chemiluminescent substance, an enzyme or polyethylene glycol.

In yet another aspect, the present invention provides use of the monoclonal antibody, the fusion protein, the antibody conjugate or the multispecific antibody in the treatment of a cancer or in the rejection of a transplantable tumor, such as melanoma, non-small cell lung cancer, bladder cancer, liver cancer, colon cancer, prostate cancer, lymphoma or renal cancer.

In yet another aspect, the present invention provides a method for preparing a monoclonal antibody having improved ADCC activity comprising:
(1) expressing a monoclonal antibody comprising a heavy chain CDR1 set forth in SEQ ID NO: 12, a heavy chain CDR2 set forth in SEQ ID NO: 13, a heavy chain CDR3 set forth in SEQ ID NO: 14, a light chain CDR1 set forth in SEQ ID NO: 15, a light chain CDR2 set forth in SEQ ID NO: 16 and a light chain CDR3 set forth in SEQ ID NO: 17 in a CHO host cell (e.g., a CHO-BAT-KF cell) in which the FUT8 gene (α-(1,6)-fucosyltransferase gene) is knocked down or knocked out;
(2) expressing a monoclonal antibody comprising a heavy chain CDR1 set forth in SEQ ID NO: 12, a heavy chain CDR2 set forth in SEQ ID NO: 13, a heavy chain CDR3 set forth in SEQ ID NO: 14, a light chain CDR1 set forth in SEQ ID NO: 15, a light chain CDR2 set forth in SEQ ID NO: 16 and a light chain CDR3 set forth in SEQ ID NO: 17 in a CHO-DG44 cell in which GDP-mannose 4,6-dehydratase (GMD) is knocked down; or
(3) expressing a monoclonal antibody comprising a heavy chain CDR1 set forth in SEQ ID NO: 12, a heavy chain CDR2 set forth in SEQ ID NO: 13, a heavy chain CDR3 set forth in SEQ ID NO: 14, a light chain CDR1 set forth in SEQ ID NO: 15, a light chain CDR2 set forth in SEQ ID NO: 16 and a light chain CDR3 set forth in SEQ ID NO: 17 in a CHO cell, and then defucosylating with a fucosyltransferase inhibitor (e.g., 2F-peracetyl-fucose).

In yet another aspect, the present invention provides a method for treating a cancer or for rejecting a transplantable tumor, such as melanoma, non-small cell lung cancer, bladder cancer, liver cancer, colon cancer, prostate cancer, lymphoma or renal cancer, comprising administering to a patient an effective amount of the monoclonal antibody, the fusion protein, the antibody conjugate or the multispecific antibody.

In yet another aspect, the present invention provides a method for enhancing a proliferative response of a T cell comprising blocking CTLA-4 binding to B7 using the monoclonal antibody, the fusion protein, the antibody conjugate or the multispecific antibody of the present invention.

In yet another aspect, the present invention provides a method for reducing tumor-infiltrating Treg cells expressing CTLA-4 in a patient comprising administering to the patient a therapeutically effective amount of the monoclonal antibody, the fusion protein, the antibody conjugate or the multispecific antibody.

In yet another aspect, the present invention provides use of the monoclonal antibody, the fusion protein, the antibody conjugate or the multispecific antibody in the preparation of a medicament for treating a cancer or for rejecting a transplantable tumor, such as melanoma, non-small cell lung cancer, bladder cancer, liver cancer, colon cancer, prostate cancer, lymphoma or renal cancer.

In yet another aspect, the present invention provides a method or use of an anti-CTLA-4 antibody or antigen-binding fragment for treating a tumor or cancer. In some embodiments, the anti-CTLA-4 antibody or antigen-binding fragment is for use in treating a tumor or cancer.

In some embodiments, the anti-CTLA-4 antibody or antigen-binding fragment comprises at least one or more of HCDR1 set forth in SEQ ID NO: 12, HCDR2 set forth in SEQ ID NO: 13, HCDR3 set forth in SEQ ID NO: 14, LCDR1 set forth in SEQ ID NO: 15, LCDR2 set forth in SEQ ID NO: 16, and LCDR3 set forth in SEQ ID NO: 17.

In some embodiments, the anti-CTLA-4 antibody or antigen-binding fragment comprises HCDR1 set forth in SEQ ID NO: 12, HCDR2 set forth in SEQ ID NO: 13, HCDR3 set forth in SEQ ID NO: 14, LCDR1 set forth in SEQ ID NO: 15, LCDR2 set forth in SEQ ID NO: 16, and LCDR3 set forth in SEQ ID NO: 17.

In some embodiments, the heavy chain variable region of the anti-CTLA-4 antibody or antigen-binding fragment comprises an amino acid sequence set forth in SEQ ID NO: 18, an amino acid sequence having at least 80% identity to the sequence set forth in SEQ ID NO: 18, or an amino acid sequence having one or more conservative amino acid substitutions compared to the sequence set forth in SEQ ID NO: 18; and/or
the light chain variable region of the anti-CTLA-4 antibody or antigen-binding fragment comprises an amino acid sequence set forth in SEQ ID NO: 19, an amino acid sequence having at least 80% identity to the sequence set forth in SEQ ID NO: 19, or an amino acid sequence having one or more conservative amino acid substitutions compared to the sequence set forth in SEQ ID NO: 19.

In some embodiments, a heavy chain variable region of the anti-CTLA-4 antibody or antigen-binding fragment comprises an amino acid sequence set forth in SEQ ID NO: 18, and a light chain variable region of the anti-CTLA-4 antibody or antigen-binding fragment comprises an amino acid sequence set forth in SEQ ID NO: 19.

In some embodiments, a heavy chain of the anti-CTLA-4 antibody comprises an amino acid sequence set forth in SEQ ID NO: 1, an amino acid sequence having at least 80% identity to the sequence set forth in SEQ ID NO: 1, or an amino acid sequence having one or more conservative amino acid substitutions compared to the sequence set forth in SEQ ID NO: 1; and/or
a light chain of the anti-CTLA-4 antibody comprises an amino acid sequence set forth in SEQ ID NO: 2, an amino acid sequence having at least 80% identity to the sequence set forth in SEQ ID NO: 2, or an amino acid sequence having one or more conservative amino acid substitutions compared to the sequence set forth in SEQ ID NO: 2.

In some embodiments, the anti-CTLA-4 antibody is antibody 1 or antibody 2, the heavy chains of both antibody 1 and antibody 2 comprise an amino acid sequence set forth in SEQ ID NO: 1, and the light chains of both antibody 1 and antibody 2 comprise an amino acid sequence set forth in SEQ ID NO: 2; antibody 1 and antibody 2 comprise two heavy chains with the same sequence and two light chains with the same sequence.

In some embodiments, the Fc region in the anti-CTLA-4 antibody (e.g., antibody 1 or antibody 2) has high mannose glycoforms with a total amount of < 5% and/or sialylated glycoforms with a total amount of < 3%. In some embodiments, the Fc region in the anti-CTLA-4 antibody (e.g., antibody 1 or antibody 2) has high mannose glycoforms with a total amount of about 0.1%, about 0.3%, about 0.9%, about 1.18%, about 1.7%, about 2.6%, about 3.3%, about 4.1%, about 4.9%, about 4.99%, or a range between any two values (inclusive) or any value therein. In some embodiments, the Fc region in the anti-CTLA-4 antibody (e.g., antibody 1 or antibody 2) has sialylated glycoforms with a total amount of about 0.1%, about 0.2%, about 0.36%, about 0.8%, about 1.5%, about 2.2%, about 2.7%, about 2.9%, 2.99%, or a range between any two values (inclusive) or any value therein. In some embodiments, the Fc region in the anti-CTLA-4 antibody (e.g., antibody 1 or antibody 2) has high mannose glycoforms with a total amount of < 3% and/or sialylated glycoforms with a total amount of < 2%. In some embodiments, the Fc region in the anti-CTLA-4 antibody (e.g., antibody 1 or antibody 2) has high mannose glycoforms with a total amount of < 2% and/or sialylated glycoforms with a total amount of < 1%.

In a specific embodiment, the anti-CTLA-4 antibody (e.g., antibody 2) or antigen-binding fragment has a fucosylation level of 0%-20%, e.g., about 0%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19% or about 20%, or a range between any two values (inclusive) or any value therein. In some embodiments, the anti-CTLA-4 antibody (e.g., antibody 2) or antigen-binding fragment has a fucosylation level of 0%-10%. In some embodiments, the anti-CTLA-4 antibody (e.g., antibody 2) or antigen-binding fragment has a fucosylation level of 0%-5%. In some embodiments, the anti-CTLA-4 antibody (e.g., antibody 2) or antigen-binding fragment has a fucosylation level of 0%-1%. In some embodiments, the anti-CTLA-4 antibody (e.g., antibody 2) or antigen-binding fragment has a fucosylation level of 0, about 0.1%, about 0.3%, about 0.4%, about 0.5%, about 0.6%, about 0.8%, about 1%, about 1.3%, about 1.6%, about 2.1%, about 2.2%, about 2.8%, about 2.9%, about 3%, about 3.3%, about 3.8%, about 4%, about 4.1%, about 4.2%, about 4.3%, about 4.6% or about 5%, or a range between any two of these values (inclusive) or any value therein. In some embodiments, the anti-CTLA-4 antibody (e.g., antibody 2) or antigen-binding fragment has no fucose binding. In some embodiments, the anti-CTLA-4 antibody (e.g., antibody 2) or antigen-binding fragment has an enhanced ADCC (anti-dependent cell-mediated cytotoxicity) effect.

The anti-CTLA-4 antibody or antigen-binding fragment can be expressed in a CHO cell or a 293 cell by genetic engineering and obtained by purification; the purification can be performed by conventional methods, such as performing centrifugation on the cell suspension and collecting the supernatant, and then performing centrifugation again to further remove impurities. Protein A affinity column, ion exchange column and other methods can be used for purifying antibody protein.

In some embodiments, the anti-CTLA-4 antibody (e.g., antibody 2) or antigen-binding fragment is expressed by a cell line in which an α-(1,6)-fucosyltransferase gene is knocked out, such as the CHO-BAT-KF cell line disclosed in PCT/CN2018/100008. In some embodiments, the antibody 2 is expressed by a CHO-BAT-KF cell line.

In some embodiments, the method or use comprises: administering to a patient in need thereof an effective amount of an anti-CTLA-4 antibody or antigen-binding fragment. In some embodiments, the anti-CTLA-4 antibody is antibody 1 or antibody 2. In some embodiments, the anti-CTLA-4 antibody (e.g., antibody 2) is expressed by a cell line in which an α-(1,6)-fucosyltransferase gene is knocked out. In some embodiments, the effective dose of anti-CTLA-4 antibody administered is about 7 mg to 180 mg per dose.

In some embodiments, the patient has a tumor or cancer. In some embodiments, the tumor and cancer include, but are not limited to, hematological cancer and solid tumor. In some embodiments, the hematologic cancer includes, but is not limited to, leukemia, lymphoma and myeloma. In some embodiments, the leukemia includes acute lymphocytic leukemia (ALL), acute myelogenous leukemia (AML), chronic lymphocytic leukemia (CLL), chronic myelogenous leukemia (CML), and myeloproliferative disorders(MPDs)/tumors. In some embodiments, the lymphoma includes Hodgkin's lymphoma, indolent and aggressive non-Hodgkin's lymphoma, Burkitt's lymphoma and follicular lymphoma (both small and large cell lymphomas). In some embodiments, the myeloma includes multiple myeloma (MM), giant cell myeloma, heavy chain myeloma, and light chain (or Bence-Jones) myeloma. In some embodiments, the solid tumor includes breast cancer, ovarian cancer, pancreatic cancer, prostate cancer, melanoma, colorectal cancer, lung cancer, head and neck cancer, bladder cancer, esophageal cancer, liver cancer and renal cancer. In some embodiments, the tumor and cancer are pathologically confirmed locally advanced or metastatic malignant solid tumors that have no effective treatment.

In some embodiments, the present invention discloses a method for treating a tumor or cancer in a patient in need comprising administering an effective amount of an anti-CTLA-4 antibody, wherein the effective amount of the anti-CTLA-4 antibody administered is about 7 mg to 180 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the anti-CTLA-4 antibody is antibody 1 and antibody 2. In some embodiments, the antibody 2 is expressed by a cell line in which an α-(1,6)-fucosyltransferase gene is knocked out. In some embodiments, the anti-CTLA-4 antibody can be formulated into a pharmaceutical composition and administered to a patient in a variety of forms suitable for the chosen route of administration, such as parenteral, intravenous (iv), intramuscular, topical, or subcutaneous. In some embodiments, the anti-CTLA-4 antibody can be intravenously infused. The dose of anti-CTLA-4 antibody will depend on the properties of the drug, the extent to which the internalization, transport and release of the drug is triggered at the cell surface, and the disease being treated and the conditions of the patient (e.g., age, sex and body weight).

In some embodiments, the dose of the anti-CTLA-4 antibody (e.g., antibody 1 or antibody 2) for each administration is about 0.1 mg/kg to 16 mg/kg, or a formulation containing the dose of the anti-CTLA-4 antibody is administered. In some embodiments, the dose of the anti-CTLA-4 antibody for each administration is about 0.1 mg/kg, about 0.2 mg/kg, about 0.3 mg/kg, about 0.4 mg/kg, about 0.5 mg/kg, about 0.9 mg/kg, about 1 mg/kg, about 1.3 mg/kg, about 1.5 mg/kg, about 1.8 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, or a range between any two of these values (inclusive) or any value therein, or a formulation containing the dose of the anti-CTLA-4 antibody is administered. In some embodiments, the dose of the anti-CTLA-4 antibody (e.g., antibody 1 or antibody 2) for each administration is about 0.1 mg/kg to 2.5 mg/kg, or a formulation containing the dose of the anti-CTLA-4 antibody is administered. In some embodiments, the dose of the anti-CTLA-4 antibody for each administration is about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2 mg/kg, about 2.1 mg/kg, about 2.2 mg/kg, about 2.3 mg/kg, about 2.4 mg/kg, about 2.5 mg/kg, or a range between any two of these values (inclusive) or any value therein, or a formulation containing the dose of the anti-CTLA-4 antibody is administered. In some embodiments, the drug is administered about once every 1 week, about once every 2 weeks, about once every 3 weeks or about once every 4 weeks. In some embodiments, the treatment cycle is 1 cycle, 2 cycles, 3 cycles, 4 cycles, 5 cycles, 6 cycles, 7 cycles, 8 cycles, 9 cycles, 10 cycles, or a range between any two of these values (inclusive) or any value therein. In some embodiments, the drug is administered about once every 3 weeks, and the treatment cycle is 1 cycle, 2 cycles, 3 cycles, 4 cycles, 5 cycles, 6 cycles, 7 cycles, 8 cycles, 9 cycles or 10 cycles. In some embodiments, the drug is administered about once every 4 weeks, and the treatment cycle is 1 cycle, 2 cycles, 3 cycles, 4 cycles, 5 cycles, 6 cycles, 7 cycles, 8 cycles, 9 cycles or 10 cycles. In some embodiments, the drug is administered about once every 5 weeks, and the treatment cycle is 1 cycle, 2 cycles, 3 cycles, 4 cycles, 5 cycles, 6 cycles, 7 cycles, 8 cycles, 9 cycles or 10 cycles.

In some embodiments, the present invention discloses a method for treating a tumor or cancer, and the method comprises: administrating, every 3 weeks, to a patient in need thereof about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2 mg/kg, about 2.1 mg/kg, about 2.2 mg/kg, about 2.3 mg/kg, about 2.4 mg/kg, about 2.5 mg/kg of the anti-CTLA-4 antibody, or a formulation containing the dose of the anti-CTLA-4 antibody.

In some embodiments, the present invention discloses a method for treating a tumor or cancer, and the method comprises: administrating, every 3 weeks, to a patient in need thereof about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1. 8 mg/kg, about 1.9 mg/kg, about 2 mg/kg, about 2.1 mg/kg, about 2.2 mg/kg, about 2.3 mg/kg, about 2.4 mg/kg, about 2.5 mg/kg of antibody 1 or antibody 2, or a formulation containing the dose of the antibody 1 or antibody 2; the treatment cycle is 4 cycles.

In some embodiments, the present invention discloses a method for treating a tumor or cancer comprising administering to a patient in need thereof an effective amount of an anti-CTLA-4 antibody (or formulation); wherein the effective amount of the anti-CTLA-4 antibody is about 7 mg to 180 mg (or a formulation containing the amount of the anti-CTLA-4 antibody) in a single administration. The dosage schedule and administration mode depend on benefit-risk assessment and general clinical practice guidelines for the anti-CTLA-4 antibody (or formulation) in certain patient populations.

In some embodiments, the patient is administered the anti-CTLA-4 antibody with an effective amount of about 7 mg to 180 mg (or a formulation containing the amount of the anti-CTLA-4 antibody) per treatment cycle. In some embodiments, one treatment cycle is 1 week, 2 weeks, 3 weeks, 4 weeks, 5 weeks, 6 weeks, 7 weeks, or a range between any two of these values (inclusive) or any value therein. In some embodiments, the treatment cycle is 1 cycle, 2 cycles, 3 cycles, 4 cycles, 5 cycles or 6 cycles. In some embodiments, the patient is administered the anti-CTLA-4 antibody (e.g., antibody 1 or antibody 2) with an effective amount of about 6 mg, about 10 mg, about 12 mg, about 18 mg, about 24 mg, about 30 mg, about 40 mg, about 50 mg, about 60 mg, about 100 mg, about 120 mg, about 180 mg, about 200 mg, about 250 mg, about 290 mg, about 300 mg, about 330 mg, about 380 mg, about 400 mg, about 434 mg, about 480 mg, about 500 mg, about 567 mg, about 580 mg, about 600 mg, about 700 mg, about 800 mg, about 900 mg, about 960 mg, or a range between any two of these values (inclusive) or any value therein, or a formulation containing the amount of the anti-CTLA-4 antibody per treatment cycle. In some embodiments, the patient is administered the anti-CTLA-4 antibody (e.g., antibody 1 or antibody 2) with an effective amount of about 7 mg, about 8 mg, about 10 mg, about 12 mg, about 18 mg, about 24 mg, about 30 mg, about 40 mg, about 50 mg, about 60 mg, about 68 mg, about 70 mg, about 80 mg, about 90 mg, about 100 mg, about 110 mg, about 120 mg, about 130 mg, about 140 mg, about 150 mg, about 160 mg, about 170 mg, about 180 mg, or a range between any two of these values (inclusive) or any value therein, or a formulation containing the amount of the anti-CTLA-4 antibody per treatment cycle. In some embodiments, one treatment cycle is administering once every 1 to 7 weeks. In some embodiments, the treatment cycle is 1 cycle, 2 cycles, 3 cycles, 4 cycles, 5 cycles, 6 cycles, or a range between any two of these values (inclusive) or any value therein.

In some embodiments, the patient is administered the anti-CTLA-4 antibody (e.g., antibody 1 or antibody 2) with an effective amount of about 7 mg to about 120 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles. In some embodiments, the patient is administered the anti-CTLA-4 antibody (e.g., antibody 1 or antibody 2) with an effective amount of about 7 mg, about 10 mg, about 12 mg, about 18 mg, about 20 mg, about 24 mg, about 28 mg, about 30 mg, about 40 mg, about 50 mg, about 60 mg, about 70 mg, about 80 mg, about 90 mg, about 100 mg, about 120 mg, or a range between any two of these values (inclusive) or any value therein, or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 120 mg to 180 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles. In some embodiments, the patient is administered the anti-CTLA-4 antibody (e.g., antibody 1 or antibody 2) with an effective amount of about 120 mg, about 130 mg, about 140 mg, about 150 mg, about 160 mg, about 170 mg, about 180 mg, or a range between any two of these values (inclusive) or any value therein, or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 6.9 mg to 7.2 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 7 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 10 mg to 13 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 12 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 20 mg to 24 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 22 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 28 mg to 33 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 30 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 59 mg to 64 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 60 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 95 mg to 113 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 100 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 110 mg to 123 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 120 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 127 mg to 133 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 130 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 132 mg to 144 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 140 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 140 mg to 155 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 150 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 177 mg to 180 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 180 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the anti-CTLA-4 antibody is administered at an effective amount of about 7 mg to 120 mg once every 3 weeks for 4 treatment cycles. In some embodiments, the anti-CTLA-4 antibody is administered at an effective amount of about 7 mg, about 10 mg, about 12 mg, about 20 mg, about 26 mg, about 30 mg, about 35 mg, about 40 mg, about 44 mg, about 50 mg, about 58 mg, about 60 mg, about 69 mg, about 70 mg, about 77 mg, about 85 mg, about 99 mg, about 110 mg, about 114 mg, or about 120 mg once every 3 weeks for 4 treatment cycles. In some embodiments, the anti-CTLA-4 antibody is administered at an effective amount of about 7 mg, about 12 mg, about 30 mg, about 60 mg or about 120 mg once every 3 weeks for 4 treatment cycles. In some embodiments, the anti-CTLA-4 antibody is administered at an effective amount of about 120 mg to 180 mg once every 3 weeks. In some embodiments, the anti-CTLA-4 antibody is administered at an effective amount of about 120 mg, about 130 mg, about 134 mg, about 140 mg, about 146 mg, about 150 mg, about 154 mg, about 160 mg, about 164 mg, about 170 mg, about 173 mg, about 176 mg, about 180 mg once every 3 weeks for 4 treatment cycles. In some embodiments, the anti-CTLA-4 antibody is administered at an effective amount of about 130 mg, about 140 mg, about 160 mg or about 180 mg once every 3 weeks for 4 treatment cycles.

In some embodiments, the anti-CTLA-4 antibody (or formulation) is administered to the patient once per treatment cycle. In some embodiments, the anti-CTLA-4 antibody (or formulation) is administered multiple times, e.g., 2, 3, 4 or 5 times, per treatment cycle. In some embodiments, the patient is administered only 1 or 4 times per treatment cycle.

In some embodiments, the patient receives one treatment cycle of treatment. In some embodiments, the patient receives multiple (e.g., 2, 3, 4, 5 or 6) treatment cycles of treatment. In some embodiments, the patient receives 4 treatment cycles of treatment. In some embodiments, the patient receives treatment until the condition is alleviated and no treatment is required.

In some embodiments, the present invention discloses a method for treating a tumor or cancer, and the method comprises: administrating, every 3 weeks, to a patient in need thereof about 7 mg to 30 mg, about 30 mg to 60 mg, about 60 mg to 120 mg, about 120 mg to 180 mg, e.g., about 7 mg, about 10 mg, about 12 mg, about 18 mg, about 30 mg, about 60 mg, about 80 mg, about 100 mg, about 120 mg, about 150 mg, about 160 mg, about 180 mg of the anti-CTLA-4 antibody, or a formulation containing the amount of the anti-CTLA-4 antibody. In some embodiments, the anti-CTLA-4 antibody is antibody 1. In some embodiments, the anti-CTLA-4 antibody is antibody 2.

In some embodiments, the patient is relieved of symptoms after a single dose is administered. In some embodiments, after a single dose is administered, the patient has not had desired relief of symptoms, and then about 7 mg to 180 mg of the anti-CTLA-4 antibody is administered to the patient until the patient has had relief of symptoms.

In some embodiments, the anti-CTLA-4 antibody (or formulation) is administered by subcutaneous (s.c.) injection, intraperitoneal (i.p.) injection, parenteral injection, intra-arterial injection, intravenous (i.v.) injection, or the like. In some embodiments, the anti-CTLA-4 antibody (or formulation) is administered by infusion. In some embodiments, the anti-CTLA-4 antibody (or formulation) is administered by bolus injection.

In some embodiments, the anti-CTLA-4 antibody (or formulation) is administered by intravenous infusion (i.e., i.v. infusion). In some embodiments, the intravenous infusion duration is about 50 minutes, about 55 minutes, about 60 minutes, about 65 minutes, about 70 minutes, about 75 minutes, about 81 minutes, about 87 minutes, about 90 minutes, about 95 minutes, or a range between any two of these values (inclusive) or any value therein. In some embodiments, the intravenous infusion duration is greater than or equal to 60 minutes.

In some embodiments, the anti-CTLA-4 antibody (or formulation) is used in combination with other treatment methods for the treatment of a tumor or cancer, e.g., chemotherapy, radiotherapy, immunotherapy, hormonal therapy, targeted therapy, biological therapy and surgical therapy. In some embodiments, the anti-CTLA-4 antibody (or formulation) is used in combination with other tumor or cancer therapeutic agents for the treatment of a tumor or cancer, e.g., a hormone and an antibody treating a tumor or cancer.

In yet another aspect, the present invention provides use of an anti-CTLA-4 antibody in the preparation of a medicament for treating a tumor or cancer. In some embodiments, the medicament for treating a tumor or cancer comprises an anti-CTLA-4 antibody. In some embodiments, the anti-CTLA-4 antibody is antibody 1 or antibody 2. In some embodiments, the antibody 2 is expressed by a cell line in which an α-(1,6)-fucosyltransferase gene is knocked out.

In yet another aspect, the present invention also provides a kit comprising an anti-CTLA-4 antibody (or formulation) and an instruction for directing administration of the anti-CTLA-4 antibody (or formulation) to a patient in need thereof. In some embodiments, the anti-CTLA-4 antibody is antibody 1 or antibody 2. In some embodiments, the antibody 2 is expressed by a cell line in which an α-(1,6)-fucosyltransferase gene is knocked out.

In yet another aspect, the present invention also provides a pharmaceutical composition which comprises an anti-CTLA-4 antibody and is suitable for injection, e.g., a bolus injection or infusion (drip) pharmaceutical composition. The pharmaceutical composition suitable for injectable use includes a sterile aqueous solution (herein water-soluble solution) or dispersion and sterile powders for the instant preparation of a sterile injectable solution or dispersion. For intravenous administration, a suitable carrier includes solvent or dispersion medium such as normal saline, bacteriostatic water or phosphate-buffered saline (PBS), ethanol, polyol (e.g., glycerol, propylene glycol and liquid polyethylene glycol), and a suitable mixture thereof. In some embodiments, the pharmaceutical composition further comprises a pharmaceutically acceptable carrier. In some embodiments, the pharmaceutically acceptable carrier may include an antibacterial and/or antifungal agent, e.g., *p-*hydroxylbenzoate, chlorobutanol, phenol, ascorbic acid and thimerosal. In some embodiments, the pharmaceutically acceptable carrier may include an isotonic agent, such as a sugar, a polyol (such as mannitol and sorbitol), and sodium chloride. In some embodiments, the pharmaceutical composition comprises at least 0.1% anti-CTLA-4 antibody. The percentage of antibody may vary and is between about 2% and 90% by weight of a given dosage form. The amount of anti-CTLA-4 antibody in such a therapeutically useful pharmaceutical composition may be an effective amount for administration. In yet another aspect, the present invention also provides a method for preparing the above pharmaceutical composition: mixing the anti-CTLA-4 antibody described herein with a pharmaceutically acceptable carrier suitable for injection (e.g., water for injection and normal saline). The method for mixing the anti-CTLA-4 antibody described above with the pharmaceutically acceptable carrier is generally known in the art.

In yet another aspect, the present invention also provides a liquid formulation comprising an anti-CTLA-4 antibody that is stable in storage and delivery, and the liquid formulation comprises the following components: 4-80 mg/mL anti-CTLA-4 antibody or fragment thereof, a buffer, a stabilizer, a chelating agent, a surfactant and water; the liquid formulation has a pH value of 5-7.

In some embodiments, the liquid formulation comprises the following components:
(1) 4-80 mg/mL anti-CTLA-4 antibody or fragment thereof,
(2) 8-45 mM buffer,
(3) 180-290 mM stabilizer,
(4) 0.01-0.3 mg/mL chelating agent,
(5) 0.1-0.5 mg/mL surfactant, and
(6) water;
the liquid formulation has a pH value of 5.2-6.5.

In some embodiments, the liquid formulation comprises the following components:
(1) 5-40 mg/mL anti-CTLA-4 antibody or fragment thereof,
(2) 10-30 mM buffer,
(3) 200-260 mM stabilizer,
(4) 0.02-0.2 mg/mL chelating agent,
(5) 0.1-0.4 mg/mL surfactant, and
(6) water;
the liquid formulation has a pH value of 5.4-6.2.

In some embodiments, the buffer does not include Tris buffer.

In some embodiments, the buffer is selected from a histidine buffer, an acetate buffer and a combination thereof. In some embodiments, the histidine buffer comprises histidine and histidine hydrochloride (e.g., L-histidine and L-histidine hydrochloride), and the acetate buffer comprises acetic acid and sodium acetate. In some embodiments, the buffer is selected from a histidine buffer and a combination of the histidine buffer and an acetate buffer. In some embodiments, the stabilizer is selected from trehalose, sucrose, mannitol or a combination thereof. In some embodiments, the stabilizer is selected from trehalose and sucrose. In some embodiments, the chelating agent comprises disodium edetate. In some embodiments, the chelating agent is disodium edetate. In some embodiments, the surfactant is selected from polysorbate 20, polysorbate 80 and a combination thereof. In some embodiments, the surfactant is polysorbate 80.

In some embodiments, the anti-CTLA-4 antibody or fragment thereof has a concentration of about 5 mg/mL, about 9 mg/mL, about 13 mg/mL, about 17 mg/mL, about 24 mg/mL, about 29 mg/mL, about 31 mg/mL, about 36 mg/mL, about 40 mg/mL, or a range between any two of these values (inclusive) or any value therein. In some embodiments, the buffer has a concentration of about 10 mM, about 14 mM, about 17 mM, about 21 mM, about 25 mM, about 29 mM, about 30 mM, or a range between any two of these values (inclusive) or any value therein. In some embodiments, the stabilizer has a concentration of about 200 mM, about 210 mM, about 219 mM, about 227 mM, about 235 mM, about 244 mM, about 251 mM, about 260 mM, or a range between any two of these values (inclusive) or any value therein. In some embodiments, the chelating agent has a concentration of about 0.02 mg/mL, about 0.09 mg/mL, about 0.12 mg/mL, about 0.17 mg/mL, about 0.2 mg/mL, or a range between any two of these values (inclusive) or any value therein. In some embodiments, the surfactant has a concentration of about 0.1 mg/mL, about 0.18 mg/mL, about 0.24 mg/mL, about 0.3 mg/mL, about 0.36 mg/mL, about 0.4 mg/mL, or a range between any two of these values (inclusive) or any value therein. In some embodiments, when the buffer is a histidine buffer or a combination of the histidine buffer and an acetate buffer, the liquid formulation has a pH value of about 5.4, about 5.5, about 5.7, about 5.9, about 6.1, about 6.2, or a range between any two of these values (inclusive) or any value therein. In some embodiments, when the buffer is an acetate buffer, a histidine buffer or a combination of the histidine buffer and the acetate buffer, the liquid formulation has a pH value of about 5.7, about 5.74, about 5.81, about 5.86, about 5.9, or a range between any two of these values (inclusive) or any value therein.

In some embodiments, the liquid formulation comprises 7-15 mg/mL anti-CTLA-4 antibody or fragment thereof. In some embodiments, the anti-CTLA-4 antibody or fragment thereof in the liquid formulation has a concentration of about 7 mg/mL, about 8 mg/mL, about 9 mg/mL, about 10 mg/mL, about 11 mg/mL, about 12 mg/mL, about 13 mg/mL, about 14 mg/mL, about 15 mg/mL, or a range between any two of these values (inclusive) or any value therein.

In some embodiments, the liquid formulation comprises 17-23 mM histidine buffer. In some embodiments, the histidine buffer in the liquid formulation has a concentration of about 17 mM, about 18 mM, about 19 mM, about 20 mM, about 21 mM, about 22 mM, about 23 mM, or a range between any two of these values (inclusive) or any value therein.

In some embodiments, the liquid formulation comprises 220-250 mM sucrose. In some embodiments, the sucrose in the liquid formulation has a concentration of about 220 mM, about 224 mM, about 228 mM, about 232 mM, about 237 mM, about 239 mM, about 241 mM, about 243 mM, about 247 mM, about 250 mM, or a range between any two of these values (inclusive) or any value therein.

In some embodiments, the liquid formulation comprises 0.02-0.05 mg/mL disodium edetate. In some embodiments, the disodium edetate in the liquid formulation has a concentration of about 0.02 mg/mL, about 0.03 mg/mL, about 0.032 mg/mL, about 0.033 mg/mL, about 0.038 mg/mL, about 0.04 mg/mL, about 0.043 mg/mL, about 0.048 mg/mL, about 0.05 mg/mL, or a range between any two of these values (inclusive) or any value therein.

In some embodiments, the liquid formulation comprises 0.1-0.3 mg/mL polysorbate 80. In some embodiments, the polysorbate 80 in the liquid formulation has a concentration of about 0.1 mg/mL, about 0.15 mg/mL, about 0.19 mg/mL, about 0.21 mg/mL, about 0.26 mg/mL, about 0.3 mg/mL, or a range between any two of these values (inclusive) or any value therein.

In some embodiments, the liquid formulation has a pH value of 5.7-5.9. In some embodiments, the liquid formulation has a pH value of about 5.7, about 5.73, about 5.75, about 5.78, about 5.8, about 5.83, about 5.87, about 5.9, or a range between any two of these values (inclusive) or any value therein.

In some embodiments, the liquid formulation comprises the following components:
(1) 10 mg/mL anti-CTLA-4 antibody or fragment thereof,
(2) 20 mM histidine buffer,
(3) 240 mM sucrose,
(4) 0.033 mg/mL disodium edetate,
(5) 0.2 mg/mL polysorbate 80, and
(6) water;
wherein the liquid formulation has a pH value of 5.8.

In some embodiments, the liquid formulation comprises sterile water or water for injection.

In some embodiments, the anti-CTLA-4 antibody or fragment thereof comprises HCDR1 set forth in SEQ ID NO: 12, HCDR2 set forth in SEQ ID NO: 13, HCDR3 set forth in SEQ ID NO: 14, LCDR1 set forth in SEQ ID NO: 15, LCDR2 set forth in SEQ ID NO: 16, and LCDR3 set forth in SEQ ID NO: 17.

In some embodiments, the heavy chain variable region of the anti-CTLA-4 antibody or fragment thereof comprises an amino acid sequence set forth in SEQ ID NO: 18 or an amino acid sequence having at least about 90% identity to the sequence set forth in the SEQ ID NO: 18, and the light chain variable region of the anti-CTLA-4 antibody or fragment thereof comprises an amino acid sequence set forth in SEQ ID NO: 19 or an amino acid sequence having at least about 90% identity to the sequence set forth in the SEQ ID NO: 19.

In some embodiments, the heavy chain variable region of the anti-CTLA-4 antibody or fragment thereof comprises a sequence having at least about 90%, about 92%, about 94%, about 95%, about 97%, about 99%, about 100% identity to the SEQ ID NO: 18, or a range between any two of these values (inclusive) or any value therein, and the light chain variable region of the anti-CTLA-4 antibody or fragment thereof comprises a sequence having at least about 90%, about 92%, about 94%, about 95%, about 97%, about 99%, about 100% identity to the SEQ ID NO: 19, or a range between any two of these values (inclusive) or any value therein.

In some embodiments, the heavy chain variable region of the anti-CTLA-4 antibody or fragment thereof comprises an amino acid sequence set forth in SEQ ID NO: 18, and the light chain variable region of the anti-CTLA-4 antibody or fragment thereof comprises an amino acid sequence set forth in SEQ ID NO: 19.

In some embodiments, the heavy chain of the anti-CTLA-4 antibody or fragment thereof comprises an amino acid sequence set forth in SEQ ID NO: 1 or an amino acid sequence having at least about 90% identity to the sequence set forth in the SEQ ID NO: 1, and the light chain of the anti-CTLA-4 antibody or fragment thereof comprises an amino acid sequence set forth in SEQ ID NO: 2 or an amino acid sequence having at least about 90% identity to the sequence set forth in the SEQ ID NO: 2. In some embodiments, the heavy chain of the anti-CTLA-4 antibody or fragment thereof comprises a sequence having at least about 90%, about 92%, about 94%, about 95%, about 97%, about 99%, about 100% identity to the SEQ ID NO: 1, or a range between any two of these values (inclusive) or any value therein, and the light chain of the anti-CTLA-4 antibody or fragment thereof comprises a sequence having at least about 90%, about 92%, about 94%, about 95%, about 97%, about 99%, about 100% identity to the SEQ ID NO: 2, or a range between any two of these values (inclusive) or any value therein.

In some embodiments, the heavy chain of the anti-CTLA-4 antibody or fragment thereof comprises an amino acid sequence set forth in SEQ ID NO: 1, and the light chain of the anti-CTLA-4 antibody or fragment thereof comprises an amino acid sequence set forth in SEQ ID NO: 2.

In some embodiments, the anti-CTLA-4 antibody or fragment thereof is a monoclonal antibody. In some embodiments, the anti-CTLA-4 antibody or fragment thereof is a fully human monoclonal antibody. In some embodiments, the anti-CTLA-4 antibody or fragment thereof is expressed by a CHO cell. In some embodiments, the anti-CTLA-4 antibody or fragment thereof is expressed by a CHO cell in which an α-(1,6)-fucosyltransferase gene is knocked out. In some embodiments, the anti-CTLA-4 antibody or fragment thereof is expressed by the CHO-BAT-KF fut8(-/-) cell (disclosed in WO2019029713A1). The anti-CTLA-4 antibody or fragment thereof is purified by a conventional method, such as low-speed centrifugation to separate cells from the culture medium, high-speed centrifugation on the supernatant, and subsequent protein A chromatography and ion exchange chromatography.

In some embodiments, the anti-CTLA-4 antibody or fragment thereof has a fucosylation level of ≤ 5%. In some embodiments, the anti-CTLA-4 antibody or fragment thereof has a fucosylation level of about 0, about 0.1%, about 0.3%, about 0.4%, about 0.6%, about 1.3%, about 1.9%, about 2.2%, about 2.8%, about 3.3%, about 3.7%, about 4.1%, about 4.5%, about 5%, or a range between any two of these values (inclusive) or any value therein.

In some embodiments, the anti-CTLA-4 antibody or fragment thereof has high mannose glycoforms with a total amount of < 5%. In some embodiments, the anti-CTLA-4 antibody or fragment thereof has high mannose glycoforms with a total amount of about 0.1%, about 0.3%, about 0.9%, about 1.18%, about 1.7%, about 2.6%, about 3.3%, about 4.1%, about 4.9%, about 4.99%, or a range between any two of these values (inclusive) or any value therein.

In some embodiments, the anti-CTLA-4 antibody or fragment thereof has sialylated glycoforms with a total amount of < 3%. In some embodiments, the anti-CTLA-4 antibody or fragment thereof has sialylated glycoforms with a total amount of about 0.1%, about 0.2%, about 0.36%, about 0.8%, about 1.5%, about 2.2%, about 2.7%, about 2.9%, about 2.99%, or a range between any two of these values (inclusive) or any value therein.

In yet another aspect, the present invention also provides use of the liquid formulation in the preparation of a medicament for treating a CTLA-4-associated disease. In another aspect, the present invention provides a method for treating a CTLA-4-associated disease comprising administering to a patient an effective dose of the liquid formulation.

In some embodiments, the CTLA-4-associated disease includes a cancer, and the liquid formulation can be used to treat the cancer, for example, to reject a transplantable tumor. In some embodiments, the CTLA-4-associated disease is melanoma, non-small cell lung cancer, bladder cancer, liver cancer, colon cancer, prostate cancer, lymphoma or renal cancer.

In some embodiments, the liquid formulation may be administered by injection or infusion; the administration site is parenteral, intravenous, mucosal, sublingual, intramuscular, intradermal, nasal, intraperitoneal, intra-arterial or subcutaneous.

In some embodiments, the patient is a mammal. In some embodiments, the patient is a human.

In yet another aspect, the present invention also discloses a method for preparing the liquid formulation comprising mixing all components in a solvent to prepare the liquid formulation. In some embodiments, the method comprises the following steps:
(1) preparing a buffer, and performing sterile filtration;
(2) performing UF/DF ultrafiltration, performing buffer exchange by ultrafiltration on an antibody solution by adopting the buffer prepared in the step (1), and then concentrating to obtain an antibody concentrate; and
(3) preparing an excipient mother liquor containing a buffer, a stabilizer, a surfactant and a chelating agent, and adding the excipient mother liquor subjected to sterile filtration into the antibody concentrate prepared in the step (2) to obtain a liquid formulation.

In order to maintain the stability of the anti-CTLA-4 antibody or fragment thereof, the present invention develops a liquid formulation that can significantly inhibit the formation of an acidic variant, a dimer, a polymer, a degradant and insoluble microparticles during freeze/thaw cycles, long term storage and temperature changes by selecting an appropriate buffer system, optimizing a stabilizer and a chelating agent and adding a surfactant. The anti-CTLA-4 antibody or fragment thereof of the present invention has good stability after repeated freeze-thaw for at least 5 times in the formulation, and can be stably stored for at least 6 months at room temperature. The liquid formulation of the present invention may be used in storing clinically effective anti-CTLA-4 antibody and fragment thereof stably and is significant in treating a CTLA-4-associated disease.

The anti-CTLA-4 antibody (or formulation) of the present invention can be used for treating a tumor or cancer.

### BRIEF DESCRIPTION OF THE DRAWINGS

FIG. 1 shows glycoforms of antibody 2 and antibody 1 for comparison; wherein triangles represent fucose, and antibody 2 is substantially free of fucose.
FIG. 2 shows ELISA binding curves of antibody 2, antibody 1 and CTLA-4 antigen; wherein "sample" represents an antibody sample.
FIG. 3 shows binding curves of Yervoy^{®}, antibody 2, antibody 1 to CTLA-4 antigen on Biacore.
FIG. 4 shows a schematic diagram of a model of *in vitro* biological activity of the anti-CTLA-4 antibody; wherein "promoter" represents a promoter, and "luciferase" represents a luciferase gene.
FIG. 5 shows a plasmid pattern of pCMV2-OKT3.
FIG. 6 shows that both antibody 2 and antibody 1 have the activity in enhancing T cell function in an activity model; wherein sample represents an antibody sample.
FIG. 7 shows the association-dissociation sensing curves of antibody 2, antibody 1 and FcγRIIIa 158V.
FIG. 8 shows a plasmid pattern of pCDH-FcγRIIIa 158V FL-Puro.
FIG. 9 shows that antibody 2 has higher *in vitro* ADCC activity than antibody 1 on an ADCC reporter model; wherein "sample" represents an antibody sample.
FIGs. 10A and 10B show the change trend of body weight of mice and the tumor inhibition effect, respectively, of antibody 2 and antibody 1 on CTLA-4 humanized MC38 tumor-bearing mice after administration; wherein arrows represent a time of each administration.
FIGs. 11A, 11B, 11C and 11D show a proportion of CD4⁺, CD8⁺, Treg cells in peripheral blood mononuclear cells in CD45 cells and a ratio of CD8⁺ to Treg cells, respectively, of Yervoy^{®}, antibody 1 and antibody 2 on CTLA-4 humanized MC38 tumor-bearing mice after administration.

### Terms

Unless otherwise specified, each of the following terms shall have the meaning described below. "Antibody" (Ab) including but not limited to an immunoglobulin is a large "Y"-shaped protein produced primarily by a plasma cell and is used by the immune system to neutralize a pathogen such as pathogenic bacteria and viruses. The antibody recognizes a unique molecule on the pathogen referred to as an "antigen" through the variable region of Fab. Each tip of the "Y"-shaped structure of the antibody comprises a paratope to a specific epitope on the antigen, allowing the paratope and the epitope to bind together precisely. The antibody can label microorganisms or infected cells by the binding mechanism, allowing the immune system to attack the microorganisms or infected cells, or can directly neutralize the microorganisms or infected cells. The communication of the antibody with other components of the immune system is mediated through its Fc region that comprises conservative glycosylation sites involved in these interactions. The production of the antibody is a major function of humoral immune system.

"Antibody fragment" comprises a portion of a full-length antibody, typically comprising the antigen-binding region thereof. Examples of antibody fragments include Fab, Fab', F(ab')2 and Fv fragments; a diabody; a linear antibody; a single chain antibody molecule; and a multispecific antibody formed from fragments of the antibody.

"Identity" and "homology" refer to sequence similarity between two peptides or between two nucleic acid molecules. Homology can be determined by comparing the positions that can be aligned in the sequences. When a position of the compared sequences is occupied by the same base or amino acid, then the molecules are homologous at that position. The degree of homology between the sequences is a function of the number of matching or homologous positions shared by the sequences.

"At least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. An amino acid may be encoded by different codons, which is known as "codon degeneracy". Amino acids include natural amino acids and non-natural amino acids. Natural amino acids include alanine (three-letter code: ala, one-letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

"Conservative amino acid substitution" refers to the replacement of one amino acid residue with another amino acid residue having a side chain (R group) of similar chemical nature (e.g., charge or hydrophobicity). Generally, conservative amino acid substitutions do not substantially alter the functional properties of the protein. Examples of amino acid classes with a side chain of similar chemical nature include: 1) an aliphatic side chain: glycine, alanine, valine, leucine and isoleucine; 2) an aliphatic hydroxyl side chain: serine and threonine; 3) an amide-containing side chain: asparagine and glutamine; 4) an aromatic side chain: phenylalanine, tyrosine and tryptophan; 5) a basic side chain: lysine, arginine and histidine; and 6) an acidic side chain: aspartic acid and glutamic acid.

A number of amino acids of "conservative amino acid substitutions of VL or VH" may be about 1, about 2, about 3, about 4, about 5, about 6, about 8, about 9, about 10, about 11, about 13, about 14, about 15 conservative amino acid substitutions, or a range between any two of these values (inclusive) or any value therein. A number of amino acids of "conservative amino acid substitutions of a heavy chain or a light chain" may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

Antibodies can be of different types referred to as isotypes or classes. In placental mammals, there are five antibody isotypes referred to as IgA, IgD, IgE, IgG and IgM. Each antibody isotype is named after the "Ig" prefix, and represents an immunoglobulin (sometimes used interchangeably with "antibody"), which differs in its biological property, functional position, and ability to process different antigens. The different suffixes of antibody isotypes represent different types of heavy chains comprised in an antibody, each heavy chain class is named alphabetically as α, γ, δ, ε and µ that correspond to the production of IgA, IgG, IgD, IgE and IgM, respectively. While there are two types of immunoglobulin light chains in mammals, referred to as λ and κ. In general, each antibody comprises two identical light chains; only one type of light chain κ or λ is present in each antibody in mammals.

Immunoglobulin g (IgG) is one of antibodies. IgG represents approximately 75% of serum antibodies in humans, and is the most common type of antibody in blood circulation. There are four IgG subclasses in humans (IgG1, 2, 3, and 4) named in the order of their abundance in serum (IgG1 is the most abundant).

Certain portions of the antibody have the same function. For example, the arm of the "Y"-shaped structure comprises a site that can bind to an antigen to recognize a specific foreign object. This antibody region is referred to as Fab region. The Fab region consists of one constant region and one variable region of each heavy chain and light chain of the antibody. The variable domain is also referred to as Fv region, and is the most important region for binding to the antigen. Specifically, three regions each of the light chain variable region (VL) and the heavy chain variable region (VH) are responsible for binding to the antigen, and these regions are referred to as complementarity determining regions (CDR). The base of the "Y"-shaped structure plays a role in regulating immune cell activity. The base region is referred to as Fc region, and consists of two heavy chains which contribute two or three constant regions depending on the class of antibody. Thus, the Fc region ensures that each antibody generates the appropriate immune response to a given antigen by binding to a specific class of Fc receptors and other immune molecules (e.g., complement proteins). In this way, the Fc region mediates diverse physiological effects including recognizing opsonized particles (binding to FcγR), cell lysis (binding to complement), and degranulating mast cells, basophils and eosinophils (binding to FcεR).

CDRs defined according to Kabat and Chothia systems include overlaps or subsets of amino acid residues when compared to each other. Nevertheless, it is within the scope of the present invention to apply any definition to refer to the CDRs of an antibody or a variant thereof. The exact number of residues comprising a specific CDR will vary according to the sequence and size of the CDR. Those skilled in the art can generally determine which specific residues a CDR comprises based on the variable region amino acid sequence of an antibody.

Kabat et al. also defines a numbering system applicable to the variable region sequence of any antibody. One of ordinary skill in the art can apply the "Kabat numbering" system to any variable region sequence without depending on other experimental data beyond the sequence itself. "Kabat numbering" refers to the numbering system proposed by Kabat et al., U.S. Dept. of Health and Human Services in "Sequence of Proteins of Immunological Interest" (1983). EU or Chothia numbering system can be also applicable to the antibody.

In immunology, an "antigen" refers to a substance that specifically binds to an antibody or a T lymphocyte antigen receptor. The antigen is a substance that stimulates the production of an antibody or is recognized by the antibody. The antigen sometimes is a portion of the host itself in an autoimmune disease. The term antigen is originally described as a structural molecule that specifically binds to an antibody. The antigen is later extended to refer to any molecule or linear molecule fragment that can be recognized by a highly variable antigen receptor (B cell receptor or T cell receptor) of the adaptive immune system.

A "monoclonal antibody" (mAb) is an antibody prepared from the same immune cell which is all clones of a single parent cell. The monoclonal antibody can have monovalent affinity because it binds to the same epitope (the antigen portion that the antibody recognizes). In contrast, a polyclonal antibody binds to multiple epitopes, and are usually secreted by several different plasma cells. A bispecific monoclonal antibody can also be engineered by adding therapeutic targets of single monoclonal antibody to two epitopes. The monoclonal antibody can be made by hybridoma, recombinant, transgene technology, or other technologies known to those skilled in the art.

A "humanized" antibody refers to an antibody from non-human species whose protein sequences have been modified to increase their similarity to naturally occurring antibody variants of humans. The "humanization" process is generally applied to an monoclonal antibody developed for human administration (e.g., the antibody developed as a anti-cancer drug). Humanization is also desirable when the process of developing a specific antibody occurs in the non-human immune system (e.g., in mice). Humanized mice are mice that carry functional human genes, cells, tissues and/or organs. Humanized mice are commonly used as small animal models in biological and medical research for human therapy. Immunodeficient mice are generally used as recipients of human cells or tissues because they can relatively easily receive heterologous cells due to lack of host immunity. This humanized mouse model can be used to simulate the human immune system in both healthy and pathological situations, and can evaluate therapeutic candidates in an *in vivo* environment related to human physiology.

"Antibody-dependent cell-mediated cytotoxicity" (ADCC) is a mechanism of cell-mediated immune defense in which effector cells of the immune system actively lyse target cells whose membrane surface antigens are bound to by specific antibodies. ADCC is a mechanism through which an antibody, as part of the humoral immune response, can act to limit and contain infection.

ADCC is independent of the immune complement system that also lyses targets but does not require any other cell. ADCC requires an effector cell which is known to be natural killer (NK) cells that typically interact with the IgG antibody. However, macrophages, neutrophils and eosinophils can also mediate ADCC, such as eosinophils killing certain parasitic worms known as helminths via the IgE antibody. In addition, ADCC is part of the adaptive immune response because it is dependent on previous antibody responses.

An "Fc region" is the tail region of an antibody which interacts with cell surface receptors referred to as Fc receptors and some proteins of the complement system. This property allows the antibody to activate the immune system. In the IgG, IgA and IgD antibody isotypes, the Fc region consists of two identical protein fragments, and is derived from a second and third constant regions of the two heavy chains of the antibody; in the IgM and IgE antibody isotypes, Fc region comprises three heavy chain constant domains (CH domains 2-4) in each polypeptide chain. The Fc region in IgG has highly conservative N-glycosylation sites. Glycosylation of the Fc fragment is essential for Fc receptor-mediated activity, and different glycoforms have different effects on the pharmaceutical properties of the therapeutic antibody. High mannose glycoforms causes rapid clearance of antibodies in the blood, leading to a shortened half-life; G0F promotes the function of complement pathway and accelerates clearance rate; sialic acid modification has a significant influence on the inflammatory effect of intravenous immunoglobulin; the reduction of fucosylation results in a significant increase in ADCC activity. Therefore, it is necessary to design and optimize the glycochains of the antibody according to the main action mechanism and pharmaceutical use of the therapeutic antibody. In the present patent, "Fc", "Fc fragment" or "Fc domain" has the same meaning as "Fc region".

An "Fc receptor" or "FcR" is the protein found on the surface of certain cells including B lymphocytes, follicular dendritic cells, natural killer cells, macrophages, neutrophils, eosinophils, basophils, human platelets, mast cells and the like, which all contribute to the protection of immune system function. The name Fc receptor derives from its binding specificity to the Fc region of an antibody. The Fc receptors bind to antibodies that attaches to infected cells or invading pathogens. The activity of the Fc receptors is exerted by destruction of the microorganism by phagocytic and cytotoxic cells, or by de-infection of the cells by antibody-mediated phagocytosis, or by antibody-dependent cell-mediated cytotoxicity. Some viruses, such as flaviviruses, use Fc receptors to help them infect cells through a mechanism known as antibody-dependent infection enhancement. There are several different types of Fc receptors which are classified based on the type of antibody they recognize. The Latin letters used to identify the antibody type are converted to the corresponding Greek letters following the "Fc" portion of the name. For example, those binding to the most common class of antibody IgG are referred to as Fc-γ receptors (FcyRs), those binding to IgA are referred to as Fc-α receptors (FcaRs), and those binding to IgE are referred to as Fc-ε receptors (FcεRs). The class of FcRs is also distinguished by the signaling properties of the cells (macrophages, granulocytes, natural killer cells, T cells and B cells) expressing the FcRs and of each receptor.

All Fcγ receptors (FcyRs) belong to the immunoglobulin superfamily and are the most important Fc receptors for inducing phagocytosis in microorganisms. This family includes several members, FcγRI (CD64), FcγRIIA (CD32), FcγRIIB (CD32), FcγRIIIA (CD16a), FcγRIIIB (CD16b), which differ in antibody affinity due to their different molecular structures. For example, FcγRI has a greater binding ability to IgG than FcγRII or FcγRIII. FcγRI also has an extracellular portion consisting of three immunoglobulin (Ig)-like domains, and has more domains than FcγRII or FcγRIII. This property allows FcγRI to bind to the only IgG molecule (or monomer), while all Fcγ receptors must bind to multiple IgG molecules in an immune complex to be activated. Fcγ receptors differ in their affinity for IgG, and different IgG subclasses have unique affinity for each Fcγ receptor. These interactions are further regulated by glycans (oligosaccharides) at position CH2-84.4 of IgG. For example, fucose containing glycans at the position CH2-84.4 reduces IgG affinity for FcγRIIIA by generating steric hindrance.

"Cancer" is a group of diseases involving abnormal cell growth with the potential to invade or spread to other parts of the body. The cancer contrasts with benign tumors which do not spread to other parts of the body. Possible signs and symptoms include a lump, abnormal bleeding, prolonged cough, unexplained weight loss, and a change in bowel movements.

The tumor-bearing mice are divided into primary models and xenograft tumor models. The most commonly used in the study are the xenograft tumor models, mainly including orthotopic and subcutaneous xenograft tumors. Briefly, the relevant tumor cells are injected into mice orthotopically or subcutaneously to result in tumorigenicity on the mice.

"Administration" means that the drug may be administered by oral, injection, infusion, parenteral, intravenous, mucosal, sublingual, intramuscular, intradermal, nasal, intraperitoneal, intra-arterial, subcutaneous absorption, or by any other administration modes in combination with the prior art. In one embodiment of the present invention, the administration is systemic.

An "effective amount" refers to an amount needed to achieve a therapeutic goal. The effective amount is related to the binding affinity of the antibody to its specific antigen, the severity of the disease, disorder or condition, the route of administration, the rate at which the antibody administered in the subject is depleted from free volume, and the like. As a non-limiting example, a therapeutically effective amount of an antibody of the present invention can be from about 0.01 mg/kg to about 15 mg/kg of patient body weight, from about 0.1 mg/kg to about 10 mg/kg of patient body weight, or from about 1 mg/kg to about 5 mg/kg of patient body weight, such as about 0.3 mg/kg, about 1 mg/kg or about 3 mg/kg of patient body weight. The frequency of administration may be, for example, from once every week to once every month, such as once every three weeks.

"Patient" generally refers to any patient, particularly a mammalian patient, in need of diagnosis, prognosis or treatment, including humans, cats, guinea pigs, rabbits, rats, mice, horses, cattle and the like.

A "glycoform" is an isoform of a protein that differs only with respect to the number or type of attached glycan. Glycoproteins generally consist of a number of different glycoforms, with alterations in the attached saccharide or oligosaccharide. These modifications may result from differences in biosynthesis during the process of glycosylation, or due to the action of glycosidases or glycosyltransferases. Glycoforms may be detected through detailed chemical analysis of separated glycoforms or through lectin affinity chromatography and lectin affinity electrophoresis and the like.

"Glycosylation" is the reaction in which a carbohydrate (i.e., a glycosyl donor) is attached to a hydroxyl or other functional group of another molecule (a glycosyl acceptor). In biology, glycosylation mainly refers to the enzymatic process of attaching glycans to proteins or other organic molecules. This enzymatic process produces a basic biopolymer (as well as DNA, RNA and proteins) found in cells. Glycosylation is a form of co-translational and post-translational modification. Glycans play a variety of structural and functional roles in membrane and secreted proteins. The majority of proteins synthesized in the rough endoplasmic reticulum undergo glycosylation. Glycosylation is an enzyme-directed site-specific process, as opposed to the non-enzymatic chemical reaction of glycation. Glycosylation is also present in the cytoplasm and nucleus as the O-GlcNAc modification. Common glycosylation include O-glycosylation and N-glycosylation.

As used herein, the term "in need" means that the patient has been identified as in need of a particular method or treatment. In some embodiments, identification may be made by any diagnostic mode. The patient may be in need of any methods and treatments described herein.

"About" refers to a general error range for corresponding values as readily understood by those skilled in the relevant art. In some embodiments, "about" mentioned herein refers to the values described and ranges thereof of ± 10%, ± 5% or ± 1%.

The amount of the buffer in the present invention means the total amount of the buffer pair in the buffer system constituting the buffer. In some embodiments, molar concentration is taken as a unit of the amount of the buffer, the value of which refers to the molar concentration of the buffer pair in the buffer system of the buffer. For example, where histidine buffer consisting of histidine and histidine hydrochloride is used as the buffer, a given concentration of histidine buffer (e.g., 20 mM) is the combined concentration of histidine and histidine hydrochloride (e.g., 8 mM histidine, 12 mM histidine hydrochloride; or 6 mM histidine, 14 mM histidine hydrochloride; or 8.46 mM histidine, 11.54 mM histidine hydrochloride).

The formulation of the present invention can be prepared by the excipients or the hydrate or salt thereof. For example, histidine hydrochloride can be anhydrous histidine hydrochloride, or histidine hydrochloride hydrate, such as histidine hydrochloride monohydrate; for example, "12 mM histidine hydrochloride" may mean that 1 L liquid formulation contains 12 mmol histidine hydrochloride or 12 mmol histidine hydrochloride monohydrate; for example, the addition of "2.52 g histidine hydrochloride monohydrate" into the formulation is equivalent to the addition of 2.3 g histidine hydrochloride into the formulation. For another example, both trehalose and a corresponding amount of trehalose dihydrate may be added into the formulation; for example, "225 mM trehalose" may mean that 1 L liquid formulation contains 225 mmol trehalose or 225 mmol trehalose dihydrate; for example, the addition of "85 g trehalose dihydrate" into the formulation is equivalent to the addition of 76.9 g trehalose into the formulation. For another example, both disodium edetate (EDTA) and a corresponding amount of disodium edetate dihydrate or edetic acid may be added into the formulation; for example, "0.033 mg/mL disodium edetate" can be prepared by adding 0.037 g disodium edetate dihydrate or 0.033 g disodium edetate or 0.029 g edetic acid to 1 L liquid formulation.

The "treatment" of a patient's disease means: (1) preventing a disease from occurring in a patient who is prone to the disease or has not shown symptoms of the disease; (2) inhibiting the disease or symptom or preventing its development; or (3) alleviating or eliminating the disease or symptom or making it regress.

"Stability" means that the antibody or fragment thereof does not, or only minimally, undergo aggregation, degradation or fragmentation under given conditions of manufacture, preparation, transportation and/or storage in a liquid formulation comprising the antibody. A "stable" formulation maintains biological activity under given conditions of manufacture, preparation, transportation and/or storage. The stability of the antibody can be evaluated by measuring the extent of aggregation, degradation or fragmentation and the like of the formulation through technologies such as SEC-HPLC, IEC-HPLC, CE-SDS (NR), visual inspection and turbidity, insoluble particles, and detection of particle size by DLS, etc. In some embodiments, a "stable" formulation refers to a formulation that can be stored stably at room temperature for at least 3 months, at least 6 months, or at least 12 months.

### DETAILED DESCRIPTION OF THE INVENTION

The technical solution of the present invention will be further illustrated below through specific examples, which are not intended to limit the protection scope of the present invention. Some nonessential modifications and adjustments made by other people according to the concept of the present invention still fall within the protection scope of the present invention.

In one aspect, the present invention provides a monoclonal antibody against CTLA-4 comprising:
(1) a heavy chain CDR1 (HCDR1) comprising or consisting of an amino acid sequence set forth in SEQ ID NO: 12, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 12, or an amino acid sequence having one or more (preferably about 1, about 2 or about 3) conservative amino acid mutations (preferably substitutions, insertions or deletions) compared to SEQ ID NO: 12,
   a heavy chain CDR2 (HCDR2) comprising or consisting of an amino acid sequence set forth in SEQ ID NO: 13, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 13, or an amino acid sequence having one or more (preferably about 1, about 2 or about 3) conservative amino acid mutations (preferably substitutions, insertions or deletions) compared to SEQ ID NO: 13,
   a heavy chain CDR3 (HCDR3) comprising or consisting of an amino acid sequence set forth in SEQ ID NO: 14, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 14, or an amino acid sequence having one or more (preferably about 1, about 2 or about 3) conservative amino acid mutations (preferably substitutions, insertions or deletions) compared to SEQ ID NO: 14,
   a light chain CDR1 (LCDR1) comprising or consisting of an amino acid sequence set forth in SEQ ID NO: 15, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 15, or an amino acid sequence having one or more (preferably 1, 2 or 3) conservative amino acid mutations (preferably substitutions, insertions or deletions) compared to SEQ ID NO: 15,
   a light chain CDR2 (LCDR2) comprising or consisting of an amino acid sequence set forth in SEQ ID NO: 16, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 16, or an amino acid sequence having one or more (preferably about 1, about 2 or about 3) conservative amino acid mutations (preferably substitutions, insertions or deletions) compared to SEQ ID NO: 16, and
   a light chain CDR3 (LCDR3) comprising or consisting of an amino acid sequence set forth in SEQ ID NO: 17, an amino acid sequence having at least about 80%, about 90%, preferably at least about 91%, about 92%, about 93%, about 94%, about 95%, about 96%, about 97%, about 98% or about 99% (or a range between any two values (inclusive) or any value therein) sequence identity to SEQ ID NO: 17, or an amino acid sequence having one or more (preferably about 1, about 2 or about 3) conservative amino acid mutations (preferably substitutions, insertions or deletions) compared to SEQ ID NO: 17;
      or
(2) a heavy chain variable region (VH) comprising or consisting of an amino acid sequence set forth in SEQ ID NO: 18, an amino acid sequence having at least about 80%, preferably at least about 85%, about 90%, about 91%, about 92%, about 93%, about 94%, about 95%, about 96%, about 97%, about 98% or about 99% (or a range between any two values (inclusive) or any value therein) sequence identity to SEQ ID NO: 18, or an amino acid sequence having one or more (preferably about 1, about 2 or about 3) conservative amino acid mutations (preferably substitutions, insertions or deletions) compared to SEQ ID NO: 18, and
   a light chain variable region (VL) comprising or consisting of an amino acid sequence set forth in SEQ ID NO: 19, an amino acid sequence having at least about 80%, preferably at least about 85%, about 90%, about 91%, about 92%, about 93%, about 94%, about 95%, about 96%, about 97%, about 98% or about 99% (or a range between any two values (inclusive) or any value therein) sequence identity to SEQ ID NO: 19, or an amino acid sequence having one or more (preferably about 1, about 2 or about 3) conservative amino acid mutations (preferably substitutions, insertions or deletions) compared to SEQ ID NO: 19;
   the monoclonal antibody has a reduced fucosylation level.

In a specific embodiment, the monoclonal antibody comprises a heavy chain and a light chain, wherein the heavy chain comprises or consists of an amino acid sequence set forth in SEQ ID NO: 1, or an amino acid sequence having at least about 80%, preferably at least about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% (or a range between any two values (inclusive) or any value therein) homology to the amino acid sequence set forth in SEQ ID NO: 1;
the light chain comprises or consists of an amino acid sequence set forth in SEQ ID NO: 2, or an amino acid sequence having at least about 80%, preferably at least about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% (or a range between any two values (inclusive) or any value therein) homology to the amino acid sequence set forth in SEQ ID NO: 2.

In a specific embodiment, the heavy chain is encoded by a nucleotide sequence set forth in SEQ ID NO: 3 or a nucleotide sequence having at least about 80%, preferably at least about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% (or a range between any two values (inclusive) or any value therein) homology to the nucleotide sequence set forth in SEQ ID NO: 3, and the light chain is encoded by the nucleotide sequence set forth in SEQ ID NO: 4 or a nucleotide sequence having at least about 80%, preferably at least about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% (or a range between any two values (inclusive) or any value therein) homology to the nucleotide sequence set forth in SEQ ID NO: 4.

In a specific embodiment, the fucosylation level is 0%-20%, e.g., about 0%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19% or about 20%, or a range between any two values (inclusive) or any value therein.

In some embodiments, the Fc region of the monoclonal antibody has high mannose glycoforms with a total amount of < 5% and/or sialylated glycoforms with a total amount of < 3%. In some embodiments, the Fc region of the monoclonal antibody has high mannose glycoforms with a total amount of about 0.1%, about 0.3%, about 0.9%, about 1.18%, about 1.7%, about 2.6%, about 3.3%, about 4.1%, about 4.9%, about 4.99%, or a range between any two values (inclusive) or any value therein. In some embodiments, the Fc region of the monoclonal antibody has sialylated glycoforms with a total amount of about 0.1%, about 0.2%, about 0.36%, about 0.8%, about 1.5%, about 2.2%, about 2.7%, about 2.9%, about 2.99%, or a range between any two values (inclusive) or any value therein.

In some embodiments, the monoclonal antibody is IgG1, and the CH2 region of the monoclonal antibody has high mannose glycoforms with a total amount of < 5% and/or sialylated glycoforms with a total amount of < 3%.

In some embodiments, the Fc region of the monoclonal antibody has high mannose glycoforms with a total amount of < 3% and/or sialylated glycoforms with a total amount of < 2%. In some embodiments, the monoclonal antibody is IgG1, and the CH2 region of the monoclonal antibody has high mannose glycoforms with a total amount of < 3% and/or sialylated glycoforms with a total amount of < 2%.

In some embodiments, the Fc region of the monoclonal antibody has high mannose glycoforms with a total amount of < 2% and/or sialylated glycoforms with a total amount of < 1%. In some embodiments, the monoclonal antibody is IgG1, and the CH2 region of the monoclonal antibody has high mannose glycoforms with a total amount of < 2% and/or sialylated glycoforms with a total amount of < 1%.

In some embodiments, the Fc region of the monoclonal antibody has a fucosylation level of 0%-10%. In some embodiments, the monoclonal antibody is IgG1, and the CH2 region of the monoclonal antibody has a fucosylation level of 0%-10%.

In some embodiments, the Fc region of the monoclonal antibody has a fucosylation level of 0%-5%. In some embodiments, the Fc region of the monoclonal antibody has a fucosylation level of about 0, about 0.1%, about 0.3%, about 0.4%, about 0.6%, about 1.3%, about 1.9%, about 2.2%, about 2.8%, about 3.3%, about 3.7%, about 4.1%, about 4.5%, about 5%, or a range between any two values (inclusive) or any value therein. In some embodiments, the monoclonal antibody is IgG1, and the CH2 region of the monoclonal antibody has a fucosylation level of 0%-5%.

In some embodiments, the monoclonal antibody is expressed by a cell line in which an α-(1,6)-fucosyltransferase gene is knocked out, such as the CHO-BAT-KF cell line disclosed in PCT/CN2018/100008.

In another aspect, the present invention provides a pharmaceutical composition or fusion protein comprising the monoclonal antibody of the present invention.

In some embodiments, the pharmaceutical composition is in a form suitable for administration by oral, injection, infusion, parenteral, intravenous, mucosal, sublingual, intramuscular, intradermal, nasal, intraperitoneal, intra-arterial, subcutaneous absorption. In some embodiments, the pharmaceutical composition is in a form suitable for systemic administration.

In another aspect, the present invention provides an antibody conjugate comprising the monoclonal antibody of the present invention and a conjugated moiety coupled thereto, wherein the conjugated moiety is a purification tag (e.g., a His tag), a cytotoxic agent, a moiety that prolongs the half-life of the monoclonal antibody or a detectable label, preferably the conjugated moiety is a radioisotope, a chemiluminescent agent, an enzyme, serum albumin (e.g., human or murine serum albumin) or polyethylene glycol.

In some embodiments, the anti-CTLA-4 antibody of the present invention is a monovalent antibody or a multivalent antibody. In another aspect, the present invention provides a multispecific antibody (e.g., a bispecific antibody) comprising the monoclonal antibody of the present invention, and an antibody or an antigen-binding fragment against another antigen and/or another antigenic epitope. In yet another aspect, the present invention provides a kit comprising the monoclonal antibody, the antibody conjugate, the multispecific antibody or the fusion protein of the present invention.

In a specific embodiment, the kit further comprises a second antibody, a cytotoxic agent, a chemotherapeutic agent and a radiotherapeutic agent, wherein the second antibody specifically recognizes the monoclonal antibody; optionally, the second antibody further comprises a detectable label, such as fluorescein, a metal ion, biotin, a radioisotope, a chemiluminescent substance, an enzyme or polyethylene glycol.

In yet another aspect, the present invention provides use of the monoclonal antibody, the fusion protein, the antibody conjugate or the multispecific antibody in the treatment of a cancer or in the rejection of a transplantable tumor, such as melanoma, non-small cell lung cancer, bladder cancer, liver cancer, colon cancer, prostate cancer, lymphoma or renal cancer.

In yet another aspect, the present invention provides a method for treating a cancer or for rejecting a transplantable tumor, such as melanoma, non-small cell lung cancer, bladder cancer, liver cancer, colon cancer, prostate cancer, lymphoma or renal cancer, comprising administering to a patient an effective amount of the monoclonal antibody, the fusion protein, the antibody conjugate or the multispecific antibody.

In yet another aspect, the present invention provides a method for enhancing a proliferative response of a T cell comprising blocking CTLA-4 binding to B7 using the monoclonal antibody, the fusion protein, the antibody conjugate or the multispecific antibody of the present invention.

In yet another aspect, the present invention provides a method for reducing tumor-infiltrating Treg cells expressing CTLA-4 in a patient comprising administering to the patient a therapeutically effective amount of the monoclonal antibody, the fusion protein, the antibody conjugate or the multispecific antibody.

In yet another aspect, the present invention provides use of the monoclonal antibody, the fusion protein, the antibody conjugate or the multispecific antibody in the preparation of a medicament for treating a cancer or for rejecting a transplantable tumor, such as melanoma, non-small cell lung cancer, bladder cancer, liver cancer, colon cancer, prostate cancer, lymphoma or renal cancer.

In yet another aspect, the present invention discloses a method or use of an anti-CTLA-4 antibody or antigen-binding fragment for treating a tumor or cancer. In some embodiments, the anti-CTLA-4 antibody or antigen-binding fragment is for use in treating a tumor or cancer.

In some embodiments, the anti-CTLA-4 antibody (e.g., antibody 2) or antigen-binding fragment is expressed by a cell line in which an α-(1,6)-fucosyltransferase gene is knocked out, such as the CHO-BAT-KF cell line disclosed in PCT/CN2018/100008. In some embodiments, the antibody 2 is expressed by a CHO-BAT-KF cell line.

In some embodiments, the method or use comprises: administering to a patient in need thereof an effective amount of an anti-CTLA-4 antibody or antigen-binding fragment. In some embodiments, the anti-CTLA-4 antibody is antibody 1 or antibody 2. In some embodiments, the anti-CTLA-4 antibody (e.g., antibody 2) is expressed by a cell line in which an α-(1,6)-fucosyltransferase gene is knocked out. In some embodiments, the effective dose of anti-CTLA-4 antibody administered is about 7 mg to 180 mg per dose.

In some embodiments, the patient has a tumor or cancer. In some embodiments, the tumor and cancer include, but are not limited to, hematological cancer and solid tumor. In some embodiments, the hematologic cancer includes, but is not limited to, leukemia, lymphoma and myeloma. In some embodiments, the leukemia includes acute lymphocytic leukemia (ALL), acute myelogenous leukemia (AML), chronic lymphocytic leukemia (CLL), chronic myelogenous leukemia (CML), and myeloproliferative disorders(MPDs)/tumors. In some embodiments, the lymphoma includes Hodgkin's lymphoma, indolent and aggressive non-Hodgkin's lymphoma, Burkitt's lymphoma and follicular lymphoma (both small and large cell lymphomas). In some embodiments, the myeloma includes multiple myeloma (MM), giant cell myeloma, heavy chain myeloma, and light chain/Bence-Jones myeloma. In some embodiments, the solid tumor includes breast cancer, ovarian cancer, pancreatic cancer, prostate cancer, melanoma, colorectal cancer, lung cancer, head and neck cancer, bladder cancer, esophageal cancer, liver cancer and renal cancer. In some embodiments, the tumor and cancer are pathologically confirmed locally advanced or metastatic malignant solid tumors that have no effective treatment.

In some embodiments, the present invention discloses a method for treating a tumor or cancer in a patient in need comprising administering an effective amount of an anti-CTLA-4 antibody, wherein the effective amount of the anti-CTLA-4 antibody administered is about 7 mg to 180 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the anti-CTLA-4 antibody is antibody 1 and antibody 2. In some embodiments, the antibody 2 is expressed by a cell line in which an α-(1,6)-fucosyltransferase gene is knocked out. In some embodiments, the anti-CTLA-4 antibody can be formulated into a pharmaceutical composition and administered to a patient in a variety of forms suitable for the chosen administration route, such as parenteral, intravenous (iv), intramuscular, topical, or subcutaneous. In some embodiments, the anti-CTLA-4 antibody can be intravenously infused. The dose of anti-CTLA-4 antibody will depend on the properties of the drug, the extent to which the internalization, transport and release of the drug is triggered at the cell surface, and the disease being treated and the conditions of the patient (e.g., age, sex and body weight).

In some embodiments, the dose of the anti-CTLA-4 antibody (e.g., antibody 1 or antibody 2) for each administration is about 0.1 mg/kg to 16 mg/kg, or a formulation containing the dose of the anti-CTLA-4 antibody. In some embodiments, the dose of the anti-CTLA-4 antibody for each administration is about 0.1 mg/kg, about 0.2 mg/kg, about 0.3 mg/kg, about 0.4 mg/kg, about 0.5 mg/kg, about 0.9 mg/kg, about 1 mg/kg, about 1.3 mg/kg, about 1.5 mg/kg, about 1.8 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, or a range between any two of these values (inclusive) or any value therein, or a formulation containing the dose of the anti-CTLA-4 antibody. In some embodiments, the dose of the anti-CTLA-4 antibody (e.g., antibody 1 or antibody 2) for each administration is about 0.1 mg/kg to 2.5 mg/kg, or a formulation containing the dose of the anti-CTLA-4 antibody. In some embodiments, the dose of the anti-CTLA-4 antibody for each administration is about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2 mg/kg, about 2.1 mg/kg, about 2.2 mg/kg, about 2.3 mg/kg, about 2.4 mg/kg, about 2.5 mg/kg, or a range between any two of these values (inclusive) or any value therein, or a formulation containing the dose of the anti-CTLA-4 antibody. In some embodiments, the drug is administered about once every 1 week, about once every 2 weeks, about once every 3 weeks or about once every 4 weeks. In some embodiments, the treatment cycle is 1 cycle, 2 cycles, 3 cycles, 4 cycles, 5 cycles, 6 cycles, 7 cycles, 8 cycles, 9 cycles, 10 cycles, or a range between any two of these values (inclusive) or any value therein. In some embodiments, the drug is administered about once every 3 weeks, and the treatment cycle is 1 cycle, 2 cycles, 3 cycles, 4 cycles, 5 cycles, 6 cycles, 7 cycles, 8 cycles, 9 cycles or 10 cycles. In some embodiments, the drug is administered about once every 4 weeks, and the treatment cycle is 1 cycle, 2 cycles, 3 cycles, 4 cycles, 5 cycles, 6 cycles, 7 cycles, 8 cycles, 9 cycles or 10 cycles. In some embodiments, the drug is administered about once every 5 weeks, and the treatment cycle is 1 cycle, 2 cycles, 3 cycles, 4 cycles, 5 cycles, 6 cycles, 7 cycles, 8 cycles, 9 cycles or 10 cycles.

In some embodiments, the present invention discloses a method for treating a tumor or cancer, and the method comprises: administrating, every 3 weeks, to a patient in need thereof about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2 mg/kg, about 2.1 mg/kg, about 2.2 mg/kg, about 2.3 mg/kg, about 2.4 mg/kg, about 2.5 mg/kg of the anti-CTLA-4 antibody, or a formulation containing the dose of the anti-CTLA-4 antibody.

In some embodiments, the present invention discloses a method for treating a tumor or cancer, and the method comprises: administrating, every 3 weeks, to a patient in need thereof about 0.1 mg/kg, about 0.2 mg/kg, about 0.3 mg/kg, about 0.4 mg/kg, about 0.5 mg/kg, about 0.6 mg/kg, about 0. 7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1. 8 mg/kg, about 1.9 mg/kg, about 2 mg/kg, about 2.1 mg/kg, about 2.2 mg/kg, about 2.3 mg/kg, about 2.4 mg/kg, about 2.5 mg/kg of antibody 1 or antibody 2, or a formulation containing the dose of the antibody 1 or antibody 2; the treatment cycle is 4 cycles.

In some embodiments, the present invention discloses a method for treating a tumor or cancer comprising administering to a patient in need thereof an effective amount of an anti-CTLA-4 antibody (or formulation); wherein the effective amount of the anti-CTLA-4 antibody is about 7 mg to 180 mg (or a formulation containing the amount of the anti-CTLA-4 antibody) in a single administration. The dosage schedule and administration mode depend on benefit-risk assessment and general clinical practice guidelines for the anti-CTLA-4 antibody (or formulation) in certain patient populations.

In some embodiments, the patient is administered the anti-CTLA-4 antibody with an effective amount of about 7 mg to 180 mg (or a formulation containing the amount of the anti-CTLA-4 antibody) per treatment cycle. In some embodiments, one treatment cycle is 1 week, 2 weeks, 3 weeks, 4 weeks, 5 weeks, 6 weeks, 7 weeks, or a range between any two of these values (inclusive) or any value therein. In some embodiments, the treatment cycle is 1 cycle, 2 cycles, 3 cycles, 4 cycles, 5 cycles or 6 cycles. In some embodiments, the patient is administered the anti-CTLA-4 antibody (e.g., antibody 1 or antibody 2) with an effective amount of about 6 mg, about 10 mg, about 12 mg, about 18 mg, about 24 mg, about 30 mg, about 40 mg, about 50 mg, about 60 mg, about 100 mg, about 120 mg, about 180 mg, about 200 mg, about 250 mg, about 290 mg, about 300 mg, about 330 mg, about 380 mg, about 400 mg, about 434 mg, about 480 mg, about 500 mg, about 567 mg, about 580 mg, about 600 mg, about 700 mg, about 800 mg, about 900 mg, about 960 mg, or a range between any two of these values (inclusive) or any value therein, or a formulation containing the amount of the anti-CTLA-4 antibody per treatment cycle. In some embodiments, the patient is administered the anti-CTLA-4 antibody (e.g., antibody 1 or antibody 2) with an effective amount of about 7 mg, about 8 mg, about 10 mg, about 12 mg, about 18 mg, about 24 mg, about 30 mg, about 40 mg, about 50 mg, about 60 mg, about 68 mg, about 70 mg, about 80 mg, about 90 mg, about 100 mg, about 110 mg, about 120 mg, about 130 mg, about 140 mg, about 150 mg, about 160 mg, about 170 mg, about 180 mg, or a range between any two of these values (inclusive) or any value therein, or a formulation containing the amount of the anti-CTLA-4 antibody per treatment cycle. In some embodiments, one treatment cycle is administering once every 1 to 7 weeks. In some embodiments, the treatment cycle is 1 cycle, 2 cycles, 3 cycles, 4 cycles, 5 cycles, 6 cycles, or a range between any two of these values (inclusive) or any value therein.

In some embodiments, the patient is administered the anti-CTLA-4 antibody (e.g., antibody 1 or antibody 2) with an effective amount of about 7 mg to about 120 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles. In some embodiments, the patient is administered the anti-CTLA-4 antibody (e.g., antibody 1 or antibody 2) with an effective amount of about 7 mg, about 10 mg, about 12 mg, about 18 mg, about 20 mg, about 24 mg, about 28 mg, about 30 mg, about 40 mg, about 50 mg, about 60 mg, about 70 mg, about 80 mg, about 90 mg, about 100 mg, about 120 mg, or a range between any two of these values (inclusive) or any value therein, or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 120 mg to 180 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles. In some embodiments, the patient is administered the anti-CTLA-4 antibody (e.g., antibody 1 or antibody 2) with an effective amount of about 120 mg, about 130 mg, about 140 mg, about 150 mg, about 160 mg, about 170 mg, about 180 mg, or a range between any two of these values (inclusive) or any value therein, or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 6.9 mg to 7.2 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 7 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 10 mg to 13 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 12 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 20 mg to 24 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 22 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 28 mg to 33 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 30 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 59 mg to 64 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 60 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 95 mg to 113 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 100 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 110 mg to 123 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 120 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 127 mg to 133 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 130 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 132 mg to 144 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 140 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 140 mg to 155 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 150 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the patient is administered the anti-CTLA-4 antibody (e.g., antibody 1 or antibody 2) with an effective amount of about 177 mg to 180 mg or a formulation containing the amount of the anti-CTLA-4 antibody per treatment cycle. In some embodiments, the patient is administered the anti-CTLA-4 antibody (e.g., antibody 1 or antibody 2) with an effective amount of about 180 mg or a formulation containing the amount of the anti-CTLA-4 antibody per treatment cycle; wherein one treatment cycle is administering about once every 1 week, about once every 2 weeks, about once every 3 weeks or about once every 4 weeks, and the treatment cycle is 2 cycles, 3 cycles, 4 cycles or 5 cycles.

In some embodiments, the anti-CTLA-4 antibody is administered at an effective amount of about 7 mg to 120 mg once every 3 weeks for 4 treatment cycles. In some embodiments, the anti-CTLA-4 antibody is administered at an effective amount of about 7 mg, about 10 mg, about 12 mg, about 20 mg, about 26 mg, about 30 mg, about 35 mg, about 40 mg, about 44 mg, about 50 mg, about 58 mg, about 60 mg, about 69 mg, about 70 mg, about 77 mg, about 85 mg, about 99 mg, about 110 mg, about 114 mg, or about 120 mg once every 3 weeks for 4 treatment cycles. In some embodiments, the anti-CTLA-4 antibody is administered at an effective amount of about 7 mg, about 12 mg, about 30 mg, about 60 mg or about 120 mg once every 3 weeks for 4 treatment cycles. In some embodiments, the anti-CTLA-4 antibody is administered at an effective amount of about 120 mg to 180 mg once every 3 weeks. In some embodiments, the anti-CTLA-4 antibody is administered at an effective amount of about 120 mg, about 130 mg, about 134 mg, about 140 mg, about 146 mg, about 150 mg, about 154 mg, about 160 mg, about 164 mg, about 170 mg, about 173 mg, about 176 mg, about 180 mg once every 3 weeks for 4 treatment cycles. In some embodiments, the anti-CTLA-4 antibody is administered at an effective amount of about 130 mg, 140 mg, about 160 mg or about 180 mg once every 3 weeks for 4 treatment cycles.

In some embodiments, the anti-CTLA-4 antibody (or formulation) is administered to the patient once per treatment cycle. In some embodiments, the anti-CTLA-4 antibody (or formulation) is administered multiple times, e.g., 2, 3, 4 or 5 times, per treatment cycle. In some embodiments, the patient is administered only 1 or 4 times per treatment cycle.

In some embodiments, the patient receives one treatment cycle of treatment. In some embodiments, the patient receives multiple (e.g., 2, 3, 4, 5 or 6) treatment cycles of treatment. In some embodiments, the patient receives 4 treatment cycles of treatment. In some embodiments, the patient receives treatment until the condition is alleviated and no treatment is required.

In some embodiments, the present invention discloses a method for treating a tumor or cancer, and the method comprises: administrating, every 3 weeks, to a patient in need thereof about 7 mg to 30 mg, about 30 mg to 60 mg, about 60 mg to 120 mg, about 120 mg to 180 mg, e.g., about 7 mg, about 10 mg, about 12 mg, about 18 mg, about 30 mg, about 60 mg, about 80 mg, about 100 mg, about 120 mg, about 150 mg, about 160 mg, about 180 mg of the anti-CTLA-4 antibody, or a formulation containing the amount of the anti-CTLA-4 antibody. In some embodiments, the anti-CTLA-4 antibody is antibody 1. In some embodiments, the anti-CTLA-4 antibody is antibody 2.

In some embodiments, the patient is relieved of symptoms after a single dose is administered. In some embodiments, after a single dose is administered, the patient has not had desired relief of symptoms, and then about 7 mg to 180 mg of the anti-CTLA-4 antibody is administered to the patient until the patient has had relief of symptoms.

In some embodiments, the anti-CTLA-4 antibody (or formulation) is administered by subcutaneous (s.c.) injection, intraperitoneal (i.p.) injection, parenteral injection, intra-arterial injection, intravenous (i.v.) injection, or the like. In some embodiments, the anti-CTLA-4 antibody (or formulation) is administered by infusion. In some embodiments, the anti-CTLA-4 antibody (or formulation) is administered by bolus injection.

In some embodiments, the anti-CTLA-4 antibody (or formulation) is administered by intravenous infusion (i.e., i.v. infusion).. In some embodiments, the intravenous infusion duration is about 50 minutes, about 55 minutes, about 60 minutes, about 65 minutes, about 70 minutes, about 75 minutes, about 81 minutes, about 87 minutes, about 90 minutes, about 95 minutes, or a range between any two of these values (inclusive) or any value therein. In some embodiments, the intravenous infusion duration is greater than or equal to 60 minutes.

In some embodiments, the anti-CTLA-4 antibody (or formulation) is used in combination with other treatment methods for the treatment of a tumor or cancer, e.g., chemotherapy, radiotherapy, immunotherapy, hormonal therapy, targeted therapy, biological therapy and surgical therapy. In some embodiments, the anti-CTLA-4 antibody (or formulation) is used in combination with other tumor or cancer therapeutic agents for the treatment of a tumor or cancer, e.g., a hormone, an antibody treating a tumor or cancer, etc.

In yet another aspect, the present invention discloses use of an anti-CTLA-4 antibody in the preparation of a medicament for treating a tumor or cancer. In some embodiments, the medicament for treating a tumor or cancer comprises an anti-CTLA-4 antibody. In some embodiments, the anti-CTLA-4 antibody is antibody 1 or antibody 2. In some embodiments, the antibody 2 is expressed by a cell line in which an α-(1,6)-fucosyltransferase gene is knocked out.

In yet another aspect, the present invention also discloses a kit comprising an anti-CTLA-4 antibody (or formulation) and an instruction for directing administration of the anti-CTLA-4 antibody (or formulation) to a patient in need thereof. In some embodiments, the anti-CTLA-4 antibody is antibody 1 or antibody 2. In some embodiments, the antibody 2 is expressed by a cell line in which an α-(1,6)-fucosyltransferase gene is knocked out.

In yet another aspect, the present invention provides a liquid formulation comprising an anti-CTLA-4 antibody or fragment thereof. In some embodiments, the liquid formulation comprises the following components: 4-80 mg/mL anti-CTLA-4 antibody or fragment thereof, a buffer, a stabilizer, a chelating agent, a surfactant and water; the liquid formulation has a pH value of 5-7.

In some embodiments, the buffer does not include Tris buffer. In some embodiments, the buffer is selected from a histidine buffer, an acetate buffer and a combination thereof. In some embodiments, the stabilizer is selected from trehalose, sucrose or mannitol. In some embodiments, the chelating agent comprises disodium edetate. In some embodiments, the chelating agent is disodium edetate. In some embodiments, the surfactant is selected from polysorbate 20, polysorbate 80 and a combination thereof.

In some embodiments, the anti-CTLA-4 antibody or fragment thereof comprises HCDR1 set forth in SEQ ID NO: 12, HCDR2 set forth in SEQ ID NO: 13, HCDR3 set forth in SEQ ID NO: 14, LCDR1 set forth in SEQ ID NO: 15, LCDR2 set forth in SEQ ID NO: 16, and LCDR3 set forth in SEQ ID NO: 17.

In some embodiments, the heavy chain variable region of the anti-CTLA-4 antibody or fragment thereof comprises an amino acid sequence set forth in SEQ ID NO: 18 or an amino acid sequence having at least about 90% identity to the sequence set forth in the SEQ ID NO: 18, and the light chain variable region of the anti-CTLA-4 antibody or fragment thereof comprises an amino acid sequence set forth in SEQ ID NO: 19 or an amino acid sequence having at least about 90% identity to the sequence set forth in the SEQ ID NO: 19.

In some embodiments, the heavy chain of the anti-CTLA-4 antibody or fragment thereof comprises an amino acid sequence set forth in SEQ ID NO: 1, and the light chain of the anti-CTLA-4 antibody or fragment thereof comprises an amino acid sequence set forth in SEQ ID NO: 2. In some embodiments, the anti-CTLA-4 antibody or fragment thereof is a fully human monoclonal antibody. In some embodiments, the anti-CTLA-4 antibody or fragment thereof is expressed by a CHO cell. In some embodiments, the anti-CTLA-4 antibody or fragment thereof is expressed by Chinese hamster ovary cells (CHO fut8(-/-), such as CHO-BAT-KF) in which the α-(1,6)-fucosyltransferase gene is knocked out. In some embodiments, the anti-CTLA-4 antibody or fragment thereof has a fucosylation level of < 5%, high mannose glycoforms with a total amount of < 5%, and sialylated glycoforms with a total amount of < 3%.

In some embodiments, the anti-CTLA-4 antibody or fragment thereof has a fucosylation level of about 0, about 0.1%, about 0.3%, about 0.4%, about 0.6%, about 1.3%, about 1.9%, about 2.2%, about 2.8%, about 3.3%, about 3.7%, about 4.1%, about 4.5%, about 5%, or a range between any two of these values (inclusive) or any value therein. In some embodiments, the anti-CTLA-4 antibody or fragment thereof has high mannose glycoforms with a total amount of about 0.1%, about 0.3%, about 0.9%, about 1.18%, about 1.7%, about 2.6%, about 3.3%, about 4.1%, about 4.9%, about 4.99%, or a range between any two of these values (inclusive) or any value therein. In some embodiments, the anti-CTLA-4 antibody or fragment thereof has sialylated glycoforms with a total amount of about 0.1%, about 0.2%, about 0.36%, about 0.8%, about 1.5%, about 2.2%, about 2.7%, about 2.9%, about 2.99%, or a range between any two of these values (inclusive) or any value therein.

In some embodiments, the liquid formulation comprises the following components: 5-40 mg/mL anti-CTLA-4 antibody or fragment thereof, 10-30 mM buffer, 200-260 mM stabilizer, 0.02-0. 2 mg/mL chelating agent, 0.1-0.4 mg/mL surfactant, and water; the liquid formulation has a pH value of 5.4-6.2.

In some embodiments, the liquid formulation comprises the following components: 5-40 mg/mL anti-CTLA-4 antibody or fragment thereof, 10-30 mM acetate buffer, 200-260 mM trehalose, 0.02-0.2 mg/mL disodium edetate, 0.1-0.4 mg/mL polysorbate 80, and water; the liquid formulation has a pH value of 5.7-5.9. In some embodiments, the stabilizer is sucrose or mannitol. In some embodiments, the surfactant is polysorbate 20.

In some embodiments, the liquid formulation comprises the following components: 5-40 mg/mL anti-CTLA-4 antibody or fragment thereof, 10-30 mM histidine buffer, 200-260 mM sucrose, 0.02-0.2 mg/mL disodium edetate, 0.1-0.4 mg/mL polysorbate 80, and water; the liquid formulation has a pH value of 5.4-6.2. In some embodiments, the stabilizer is trehalose or mannitol. In some embodiments, the surfactant is polysorbate 20.

In some embodiments, the liquid formulation comprises the following components: 5-40 mg/mL anti-CTLA-4 antibody or fragment thereof, 10-30 mM composition of histidine buffer and acetate buffer, 200-260 mM sucrose, 0.02-0.2 mg/mL disodium edetate, 0.1-0.4 mg/mL polysorbate 20, and water; the liquid formulation has a pH value of 5.7-5.9. In some embodiments, the stabilizer is trehalose or mannitol. In some embodiments, the surfactant is polysorbate 80.

In some embodiments, the liquid formulation comprises the following components: about 5 mg/mL anti-CTLA-4 antibody or fragment thereof, about 10 mM histidine buffer, about 200 mM trehalose, about 0.02 mg/mL disodium edetate, about 0.1 mg/mL polysorbate 20, and water; the liquid formulation has a pH value of about 5.4.

In some embodiments, the liquid formulation comprises the following components: about 9 mg/mL anti-CTLA-4 antibody or fragment thereof, about 14 mM acetate buffer, about 219 mM trehalose, about 0.09 mg/mL disodium edetate, about 0.24 mg/mL polysorbate 20, and water; the liquid formulation has a pH value of about 5.7.

In some embodiments, the liquid formulation comprises the following components: about 24 mg/mL anti-CTLA-4 antibody or fragment thereof, about 17 mM acetate buffer, about 227 mM sucrose, about 0.12 mg/mL disodium edetate, about 0.3 mg/mL polysorbate 20, and water; the liquid formulation has a pH value of about 5.81.

In some embodiments, the liquid formulation comprises the following components: about 29 mg/mL anti-CTLA-4 antibody or fragment thereof, about 21 mM histidine buffer, about 235 mM sucrose, about 0.17 mg/mL disodium edetate, about 0.3 mg/mL polysorbate 80, and water; the liquid formulation has a pH value of about 5.7.

In some embodiments, the liquid formulation comprises the following components: about 31 mg/mL anti-CTLA-4 antibody or fragment thereof, about 25 mM histidine buffer, about 244 mM sucrose, about 0.11 mg/mL disodium edetate, about 0.3 mg/mL composition of polysorbate 80 and polysorbate 20, and water; the liquid formulation has a pH value of about 5.9.

In some embodiments, the liquid formulation comprises the following components: about 36 mg/mL anti-CTLA-4 antibody or fragment thereof, about 29 mM histidine buffer, about 251 mM mannitol, about 0.16 mg/mL disodium edetate, about 0.36 mg/mL polysorbate 80, and water; the liquid formulation has a pH value of about 6.1.

In some embodiments, the liquid formulation comprises the following components: about 40 mg/mL anti-CTLA-4 antibody or fragment thereof, about 30 mM composition of histidine buffer and acetate buffer, about 260 mM mannitol, about 0.2 mg/mL disodium edetate, about 0.4 mg/mL polysorbate 80, and water; the liquid formulation has a pH value of about 5.9.

In some embodiments, the liquid formulation comprises the following components: about 40 mg/mL anti-CTLA-4 antibody or fragment thereof, about 30 mM acetate buffer, about 248 mM mannitol, about 0.2 mg/mL disodium edetate, about 0.3 mg/mL polysorbate 80, and water; the liquid formulation has a pH value of about 5.9.

In some embodiments, the liquid formulation comprises the following components: 7-15 mg/mL anti-CTLA-4 antibody or fragment thereof, 17-23 mM histidine buffer, 220-250 mM sucrose, 0.02-0.05 mg/mL disodium edetate, 0.1-0.3 mg/mL polysorbate 80, and water; the liquid formulation has a pH value of 5.7-5.9.

In some embodiments, the liquid formulation comprises the following components: about 7 mg/mL anti-CTLA-4 antibody or fragment thereof, about 17 mM histidine buffer, about 220 mM sucrose, about 0.02 mg/mL disodium edetate, about 0.1 mg/mL polysorbate 80, and water; the liquid formulation has a pH value of about 5.7.

In some embodiments, the liquid formulation comprises the following components: about 9 mg/mL anti-CTLA-4 antibody or fragment thereof, about 20 mM histidine buffer, about 228 mM sucrose, about 0.032 mg/mL disodium edetate, about 0.19 mg/mL polysorbate 80, and water; the liquid formulation has a pH value of about 5.75.

In some embodiments, the liquid formulation comprises the following components: about 10 mg/mL anti-CTLA-4 antibody or fragment thereof, about 20 mM histidine buffer, about 240 mM sucrose, about 0.033 mg/mL disodium edetate, about 0.2 mg/mL polysorbate 80, and water; the liquid formulation has a pH value of about 5.8.

In some embodiments, the liquid formulation comprises the following components: about 14 mg/mL anti-CTLA-4 antibody or fragment thereof, about 22 mM histidine buffer, about 243 mM sucrose, about 0.043 mg/mL disodium edetate, about 0.26 mg/mL polysorbate 80, and water; the liquid formulation has a pH value of about 5.87.

In some embodiments, the liquid formulation comprises the following components: about 15 mg/mL anti-CTLA-4 antibody or fragment thereof, about 23 mM histidine buffer, about 250 mM sucrose, about 0.05 mg/mL disodium edetate, about 0.3 mg/mL polysorbate 80, and water; the liquid formulation has a pH value of about 5.9.

In some embodiments, the water comprised in the liquid formulation is sterile water or water for injection.

In another aspect, the present invention provides use of the liquid formulation in the preparation of a medicament for treating a CTLA-4-associated disease. In another aspect, the present invention provides a method for treating a CTLA-4-associated disease comprising administering to a patient an effective dose of the liquid formulation.

In some embodiments, the CTLA-4-associated disease includes a cancer or is used to reject a transplantable tumor. In some embodiments, the CTLA-4-associated disease is melanoma, non-small cell lung cancer, bladder cancer, liver cancer, colon cancer, prostate cancer, lymphoma or renal cancer. In some embodiments, the liquid formulation may be administered by injection or infusion; the administration site is parenteral, intravenous, mucosal, sublingual, intramuscular, intradermal, nasal, intraperitoneal, intra-arterial or subcutaneous.

In some embodiments, the solution containing the anti-CTLA-4 antibody is diluted with an isotonic solution (e.g., 0.9% NaCl solution for injection) and then administered by intravenous infusion. In some embodiments, the solution containing 10 mg/mL anti-CTLA-4 antibody is diluted with an isotonic solution (e.g., 0.9% NaCl solution for injection) and then administered by intravenous infusion. In some embodiments, the diluted anti-CTLA-4 antibody has a concentration of about 0.05 mg/mL to about 5 mg/mL or about 0.5 mg/mL to about 1 mg/mL. In some embodiments, the diluted anti-CTLA-4 antibody has a concentration of about 0.05 mg/mL, about 0.07 mg/mL, about 0.09 mg/mL, about 0.1 mg/mL, about 0.2 mg/mL, about 0.3 mg/mL, about 0.5 mg/mL, about 0.7 mg/mL, about 0.8 mg/mL, about 0.9 mg/mL, about 1 mg/mL, about 1.7 mg/mL, about 2.4 mg/mL, about 2.9 mg/mL, about 3.1 mg/mL, about 3.8 mg/mL, about 4.1 mg/mL, about 4.6 mg/mL or about 5 mg/mL. In some embodiments, the effective amount is the dose described herein.

In some embodiments, the effective amount may be 0.1-100 mg/kg of patient body weight. In some embodiments, the effective amount is 0.1-20 mg/kg of patient body weight. In other embodiments, the effective amount is about 2 mg/kg of patient body weight. In other embodiments, the effective amount is about 3 mg/kg of patient body weight. In other embodiments, the effective amount is about 10 mg/kg of patient body weight. In other embodiments, the effective amount is about 20 mg/kg of patient body weight. In other embodiments, the effective amount is about 30 mg/kg of patient body weight. In other embodiments, the effective amount is about 50 mg/kg of patient body weight. In other embodiments, the effective amount is about 100 mg/kg of patient body weight. In some embodiments, the effective amount of the anti-CTLA-4 antibody or fragment thereof can be administered to the patient once or more times daily, weekly, monthly and/or yearly per hour/day/week/month as needed.

In another aspect, the present invention provides a method for preparing the liquid formulation, which comprises the following steps:
(1) preparing a buffer, and performing sterile filtration;
(2) performing UF/DF ultrafiltration, performing buffer exchange by ultrafiltration on an antibody by adopting the buffer prepared in the step (1), and then concentrating to obtain an antibody concentrate; and
(3) preparing an exicipients mother liquor containing a buffer, a stabilizer, a surfactant and a chelating agent, adjusting to a suitable pH, and adding the exicipients mother liquor subjected to sterile filtration into the antibody concentrate prepared in the step (2) to obtain a liquid formulation.

### Example 1. Preparation of Antibody 1 and Antibody 2

CHO cells (e.g., Chinese hamster ovary cells cultured from CHO-K1 cell strain and adapted to suspension growth) and CHO-BAT-KF which have been disclosed in Patent CN109096399A were prepared, and two strains of cells were transfected with linearized plasmids comprising nucleotide sequences encoding the light/heavy chains of anti-CTLA-4 antibody by electroporation, wherein the heavy chain CDR1 of the anti-CTLA-4 antibody was set force in SEQ ID NO: 12, the heavy chain CDR2 thereof was set force in SEQ ID NO: 13, the heavy chain CDR3 thereof was set force in SEQ ID NO: 14, the light chain CDR1 thereof was set force in SEQ ID NO: 15, the light chain CDR2 thereof was set force in SEQ ID NO: 16, the light chain CDR3 thereof was set force in SEQ ID NO: 17, the heavy chain variable region sequence thereof was set force in SEQ ID NO: 18, the light chain variable region sequence thereof was set force in SEQ ID NO: 19, the heavy chain sequence thereof was set force in SEQ ID NO: 1, the light chain sequence thereof was set force in SEQ ID NO: 2, and the nucleotide sequence coding the heavy chain thereof was set force in SEQ ID NO: 3, and the nucleotide sequence encoding the light chain thereof was set force in SEQ ID NO: 4. The electrotransfer conditions are as follows: 300 V, 1500 µF, 4 mm electroporation cuvette, 40 µg linearized plasmids, and 10 million cells. A cell transfecting the CHO cell is named BAT1 (which is a cell expressing "antibody 1" herein); a cell transfecting the CHO-BAT-KF cell is named BAT2 (which is a cell expressing "antibody 2" herein). After the electrotransfection, the two cells were resuspended in CHO-AGT (Gibco, Cat.# 12490025) medium containing 1× GS (Specialty Media, Cat.# GSS 1016-C) and distributed evenly in 40 96-well plates at a density of 1500 cells per well, and the volume of each well was 100 µL. The cell culture plate was cultured in an incubator at 8% CO₂, 37 °C. After 48 h, the culture was continued by replenishing 100 µL of CHO-AGT medium (1× GS and MSX (methionine sulfoximine) at a final concentration of 50 µM).

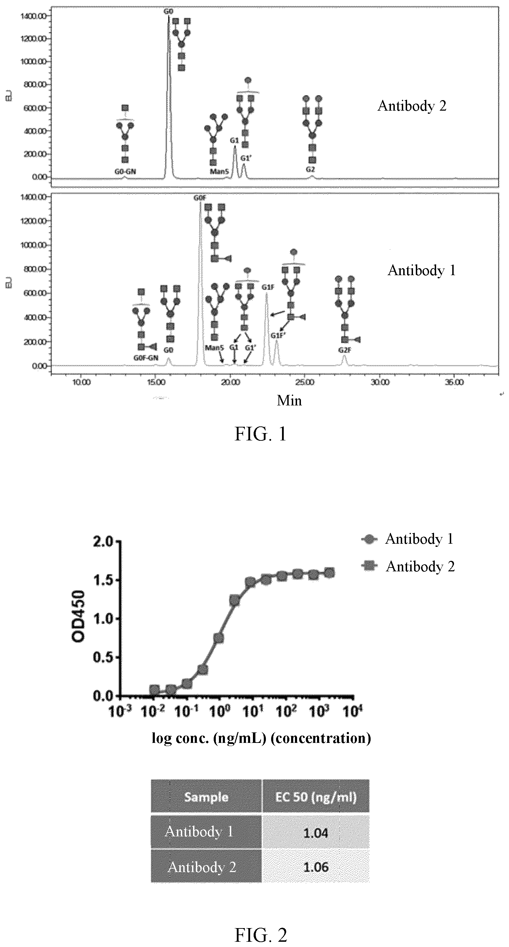

The ELISA plate (Corning, Cat.# 9018) for screening positive clones were prepared in advance. Goat-anti-human Fc antibody (Sigma, Cat.# 12136) was diluted to a concentration of 2 µg/mL by PBS, 100 µL of the diluted goat-anti-human Fc antibody was added into each well of the ELISA plate, after incubation for 2 h at 37 °C, liquid in the plate was thrown off and patted dry, 200 µL of blocking solution (PBS containing 5% BSA) was added into each well, and after incubation for 2 h at 37 °C, the plate was directly placed at -20 °C for storage. Cells in the 96-well cell culture plate formed visible cell clone clusters after about 16-20 days. 1389 clones were picked out as BAT1, and 852 clones were picked out as BAT2. The assay steps comprises: thawing the ELISA plate coated with goat-anti-human Fc antibody, washing the plate with PBST for 3 times, coating the cell culture supernatant in the 96-well plate diluted by 40 times, incubating for 2 h at 37 °C, washing the plate with PBST for 5 times, adding 1:10000 diluted goat-anti-human Kappa light chain-secondary antibody conjugating with peroxidase (Sigma, Cat.# A7164), incubating for 0.5 h at 37 °C, washing the plate with PBST for 7 times, adding TMB single-component color developing solution (InnoReagents, Cat.# TMB-S-001) for color development in the dark for 10-15 minutes, terminating the reaction with 0.1 M sulfuric acid, reading at a wavelength of 450 nm, and marking positive clones with higher expression level. Positive clones were transferred to 24-well plates and cultured for 5-7 days, and the culture supernatant was diluted 1000-fold for the above ELISA assay. Positive clones with higher expression level and good cell state were transferred to 6-well plates and cultured for 5-7 days, and the culture supernatant was diluted 2000-fold for the above ELISA assay. Positive clones with higher expression level and good cell state were transferred to 125 mL small shake flasks and cultured on shakers. When the cells had stable and good growth state and ≥ 95% viability, and the cells reach a certain quantity, the cells were inoculated into the CHO-AGT culture medium at a density of 30× 10⁵ cells/mL to 50×10⁵ cells/mL and a volume of 30 mL, and the shake flask culture was performed for 12 days. In the culture process, 0.5 mL of culture was taken on days 3, 6 and 9 to measure the cell density and the cell viability, and 3 mL of Feed C (CD EfficientFeed C AGT, Gibco) was added after taking the culture, wherein the culture on days 6 and 9 was centrifuged to take the supernatant for cryopreserving; the culture on days 10, 11 and 12 was taken to measure the cell density and the cell viability, and the culture was centrifuged to take the supernatant for cryopreserving. All cell supernatants were collected on day 12 and purified with Protein A. The antibody purified from BAT1 cells was antibody 1, and antibody purified from BAT2 cells was antibody 2. Antibody 1 and antibody 2 were sequenced, and antibody 1 and antibody 2 had the heavy chain sequence set forth in SEQ ID NO: 1 (each heavy chain comprises 448 amino acids and has a molecular weight of 51 kDa) and the light chain sequence set forth in SEQ ID NO: 2 (each light chain comprises 215 amino acids and has a molecular weight of 23 kDa). The resulting proteins were subjected to all mass analysis, wherein in analysis of protein glycoforms, since antibody 2 was expressed in a cell line CHO-BAT-KF in which an α-(1,6)-fucosyltransferase was knocked out, the glycoforms of the antibody 2 differed from those of antibody 1 (see FIG. 1 and Table 1), and the main glycoforms of antibody 2 were defucosylated glycoforms, i.e., the fucosylation level was about 0. The cryopreserved supernatant on days 6, 9, 10, 11 and 12 was subjected to HPLC to detect the protein expression level.

In addition, CH2 regions of Yervoy^{®} (lpilimumab commercially available from Bristol-Myers Squibb Company, Yervoy^{®} was also represented as _930395 herein), antibody 1 and antibody 2 were detected for glycoform content, with the result that a total amount of high mannose glycoform was < 5% and a total amount of sialylated glycoform was < 3%; among them, the measurement results of a group of high mannose glycoform and sialylated glycoform are shown in Table 1 (/ represents no detection or a amount of 0 in Table 1):

**Table 1. Percentage of part glycoforms**

| Glycoform classification | | Yervoy^{®} | Antibody 1 | Antibody 2 |
|---|---|---|---|---|
| Sialylated glycoform | G1S1 | / | / | 0.04 |
| | G1FS1 | 0.54 | 0.4 | / |
| | G2S1 | / | / | 0.17 |
| | G2S1' | / | / | 0.1 |
| | G2FS1 | 0.65 | 0.42 | / |
| | G2FS1' | 0.56 | 0.14 | / |
| | G2S2 | / | / | 0.14 |
| | G2FS2 | 0.84 | 0.19 | / |
| High mannose glycoform | Man5 | 2.48 | 0.56 | 1.08 |
| | Man6 | 0.91 | 0.14 | / |
| | Man8 | 0.11 | 0.14 | 0.1 |
| Fucosylation level | G0F-GN | 3.34 | 0.44 | / |
| | G0F | 59.35 | 53.34 | / |
| | G1F | 16.53 | 25.18 | / |
| | GIF' | 6.2 | 9.07 | / |
| | G1FS1 | 0.54 | 0.4 | / |
| | G2F | 4.17 | 4.16 | / |
| | G2FS1 | 0.65 | 0.42 | / |
| | G2FS1' | 0.56 | 0.14 | / |

Glycoforms affecting the risk of immunogenicity of antibodies comprised sialylated glycoform: the antibodies with the sialylated glycoform contents from high to low were sequentially Yervoy^{®}, the antibody 1 and the antibody 2, and the low sialylated glycoform in the antibody 2 of the present invention was favorable for reducing the risk of immunogenicity. The antibodies with the high mannose glycoform contents from high to low were sequentially Yervoy^{®}, antibody 2 and antibody 1, and the low high mannose glycoform in the antibody 2 was favorable for prolonging the half-life of the antibody.

### Example 2. In Vitro Binding Activity of Antibody 2 Protein to CTLA-4 Antigen

The plasmid expressing the extracellular region of CTLA-4 antigen and the nucleotide sequence of human IgG1 antibody heavy chain Fc fragment (SEQ ID NO: 5, the amino acid sequence thereof was set forth in SEQ ID NO: 6) was transiently transfected into 293F cells.

The 293F cells were cultured with CD 293 TGE Medium (BPM Cell Culture, Cat.# CM-1156), and when the cell viability was ≥ 95%, the density of cells was sub-cultured to 80× 10⁵ cells/mL to 100×10⁵ cells/mL with fresh medium, and transfection could be performed when the cell density was approximately 150× 10⁵ cells/mL to 200× 10⁵ cells/mL after 24 h. The plasmid was first subjected to a water bath for 30 minutes at 65 °C with 1 µg plasmid per 100× 10⁵ cells and 3 µL PEI (Polysciences, Cat.# 24765-2) per 1 µg plasmid. The plasmid and PEI were each diluted with medium (the volume of the two solutions added together was 5% of a total volume) and left to stand for 5 minutes at room temperature. The PEI solution was then added into the plasmid solution, mixed well and left to stand for 20 minutes at room temperature. The mixed solution was added into the cells, and after the cells were cultured on shakers for 24 h at 37 °C, CD Feed X Supplement (BPM Cell Culture, Cat.# CF-1116) with a total volume ratio of 10% was added into the cells. The cell supernatant was harvested after 5 days and purified by Protein A to obtain CTLA-4-ECD-Fc antigen protein.

The ELISA plate was coated with the CTLA-4-ECD-Fc antigen protein overnight at 4 °C. The blocking was performed with 5% BSA blocking solution for 2 h at 37 °C on the next day. After the plate was washed, the antibody 2 protein and the antibody 1 protein were added, which were 3-fold gradient diluted from a concentration of 1 µg/mL for a total of 12 concentrations (the last one was 0), 3 replicate wells were made for each concentration, and incubation was performed for 2 h at 37 °C. After the plate was washed, the 1:10000 diluted goat-anti-human Kappa light chain-secondary antibody (Sigma, Cat.# A7164) conjugating with peroxidase was added and incubated for 0.5 h at 37 °C. After the plate was washed, a TMB single-component color developing solution was added for color development in the dark for 10-15 minutes, the reaction was terminated with 0.1 M sulfuric acid, reading was performed at a wavelength of 450 nm, and the ELISAbinding curves of the antibody 2 and the antibody 1 to the CTLA-4 antigen are shown in FIG. 2.

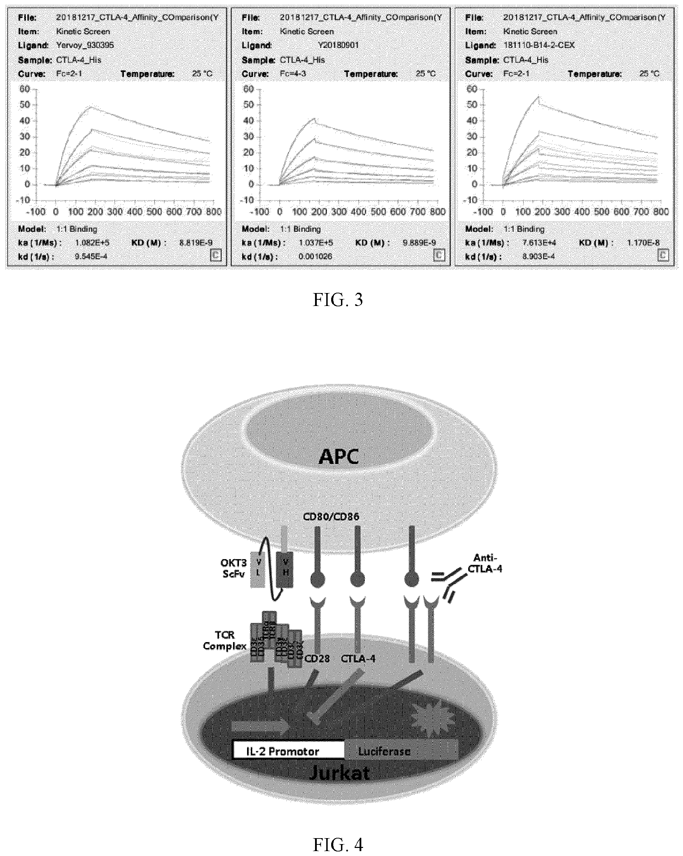

Meanwhile, the *in vitro* binding activity of the antibody 2 protein and the antibody 1 protein to CTLA-4-His (Sino Biological, 11159-H08H) antigen was also detected on BIAcore (T200, GE Healthcare). The concentration of the test antibodies (the antibody 2, the antibody 1 and Yervoy^{®}) was duilted to 5 µg/mL by HBS-EP (0.01 M HEPES, 0.15 M NaCl, 3 mM EDTA, 0.005% P20, pH 7.4), and the concentration of the antigen CTLA-4-His was diluted to 100 nM by HBS-EP, which was taken as an initial concentration for two-fold gradient dilution to obtain CTLA-4-His diluents at concentrations of 100 nM, 50 nM, 25 nM, 12.5 nM, 6.25 nM and 3.125 nM. Detection was preformed using a Protein A chip (GE Healthcare), and 5 µg/mL of drug diluents was passed through the experimental flow paths (Fc2 and Fc4) at a flow rate of 10 µL/min for 15s so that the capture amount was about 600 RU; then the flow rate was adjusted to 30 µL/min, CTLA-4-His dilutions with different concentrations of the analyte (0 nM, 3.125 nM, 6.25 nM, 12.5 nM, 25 nM, 50 nM and 100 nM) were sequentially added and simultaneously passed through the surfaces of experimental flow paths (Fc2 and Fc4) and reference flow paths (Fc1 and Fc3) with an association time of 180 s and a dissociation time of 600 s, and finally the chip was subjected to regeneration by the Glycine 1.5 buffer for 60 s and entered the next cycle. Experiment results were analyzed using data analysis software Evaluation software 3.1, the sensing signals acquired by the sample experiment flow path were double-subtracted for reference flow path and samples blank, and fitting was preformed using a kinetic "1:1" model to obtain the kinetic parameters of the affinity association of each antibody sample with the CTLA-4, wherein the kinetic parameters are shown in Table 2. Biacore curve fitting (wherein Yervoy_930395 was Yervoy^{®}, Y20180901 was antibody 1, and 181110-B14-2-CEX was antibody 2) is shown in FIG. 3.

**Table 2. Kinetic parameters of affinity association of antibody 2, antibody 1 and Yervoy^{®} with CTLA-4**

| **Samples** | **ka (1/Ms)** | **kd (1/s)** | **KD (M)** |
|---|---|---|---|
| Yervoy^{®} | 1.08e+05 | 9.54e-04 | 8.82e-09 |
| Antibody 1 | 1.04e+05 | 1.03e-03 | 9.89e-09 |
| Antibody 2 | 7.61e+04 | 8.9e-04 | 1.17e-08 |

| | | | |
|---|---|---|---|
| Note: ka: association rate; kd: dissociation rate; KD: association-dissociation equilibrium constant, i.e., affinity. Conclusion: Antibody 2 protein and antibody 1 protein binding to CTLA-4 antigen showed similar binding activity on both ELISA and BIAcore. It indicates that ADCC enhancement on anti-CTLA-4 antibody did not change its binding capacity to CTLA-4 antigen. | | | |

### Example 3. In Vitro Biological Activity of Antibody 2 Protein and Antibody 1 Protein

A schematic diagram of a model *of in vitro* biological activity of the anti-CTLA-4 antibody is shown in FIG. 4.

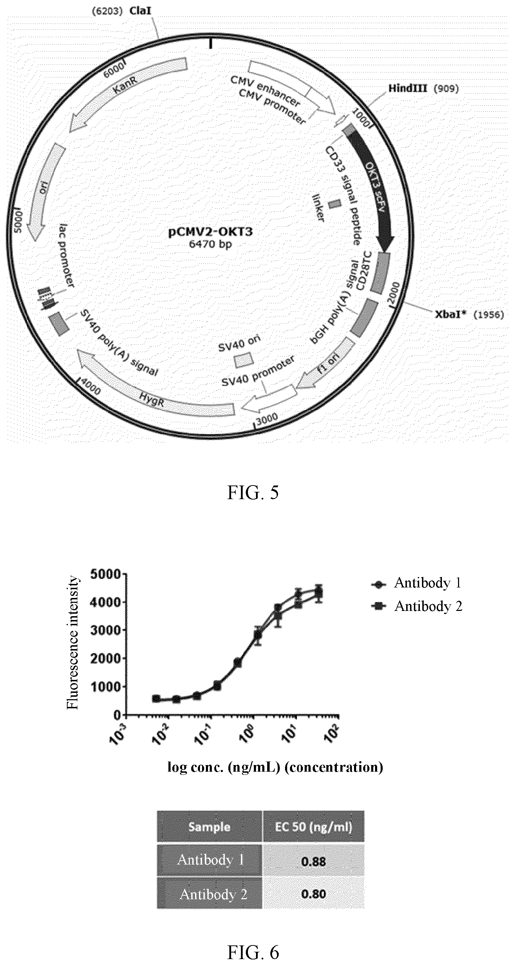

The pCMV2-OKT3 (in which the OKT3 part was in a form of ScFv of the antibody, the nucleotide sequence thereof was set forth in SEQ ID NO: 7, the heavy chain amino acid sequence of OKT3 was set forth in SEQ ID NO: 8, and the light chain amino acid sequence was set forth in SEQ ID NO: 9) plasmid was constructed, and the plasmid pattern is shown in FIG. 5.

The pCMV2-OKT3 was transfected into Raji cells by electrotransfection, then subjected to pressurized screening by hygromycin, mixed with Jurkat-IL2-luciferase cells and stimulated for 6 h, a luciferase substrate was added, and an enzyme reader was used for detecting autofluorescence, so that positive monoclonals capable of stimulating Jurkat-IL2-luciferase cells (Promega, JA 3005) to generate autofluorescence were screened and named Raji-OKT3 cells.

The density of Raji-OKT3 cells and Jurkat-IL2-luciferase-CTLA4FL cells (Promega, JA 3005) were diluted to 400×10⁵ cells/mL using 1640 medium containing 1% FBS as Buffer in this example, and 25 µL of Raji-OKT3 and 25 µL of Jurkat-IL2-luciferase-CTLA4FL were added to each well of a 96-well white plate (Corning, Cat.# 3917). The concentration of the antibody 2 and the antibody 1 was diluted to 100 µg/mL using Buffer, which was taken as an initial concentration for 3-fold gradient dilution for a total of 10 concentrations (the last one was 0). Antibody solutions with different concentrations were added into the above wells which have been added with the two cells (25 µL for each well), and 3 replicate wells were made for each concentration. At this time, 75 µL of the cell antibody mixture was presented in each well in total. The white plate was incubated in a 37°C incubator for 6 h, and then the white plate was taken out and left at room temperature for 10 minutes. 75 µL of firefly luciferase substrate (Promega) equilibrated to room temperature was added into each well, the white plate was placed in the microplate reader and subjected to shaking for 5 s, and the fluorescence value was read after 10 minutes. The results are shown in FIG. 6.

Conclusion: Both antibody 2 and antibody 1 showed similar activity in enhancing T cell function in an activity model. It indicates that ADCC enhancement on anti-CTLA-4 antibody did not change its *in vitro* biological activity with CTLA-4 antigen.

### Example 4. Binding of Antibody 2 Protein and Antibody 1 Protein to FcγRIIIa

The SA sensor (Fertebo) was pre-wetted in PBS for 10 minutes for use, the concentration of biotin labeled-FcγRIIIa 158V (ACRO Biosystems) and biotin labeled-FcyRIIIa 158F (ACRO Biosystems) was diluted to 1 µg/mL with PBST (hereinafter referred to as dilution buffer) containing 0.5% BSA at pH 6.8, and the SA sensor was immobilized in FcγRIIIa 158-Biotin to a signal of about 0.6 nm. The concentration of test antibody 2 and 1 was diluted to 500 nM with dilution buffer, which was taken as an initial concentration for 2-fold gradient dilution, and a total of 7 concentration points (500 nM, 250 nM, 125 nM, 62.5 nM, 31.25 nM, 15.62 nM and 7.812 nM) were prepared. A dilution buffer, a drug antibody diluent at the gradient concentration, a regeneration buffer (1M MgCl₂ at pH 8.4) and a neutralization buffer (PBST) were sequentially added into the corresponding columns of the 96-well plate, and the following steps were performed: (1) Baseline: baseline detection for 60 seconds in the dilution buffer; (2) Association: association for 90 seconds in the antibody gradient diluent and sample blanks (dilution buffer); (3) Dissociation: dissociation for 90 seconds in the dilution buffer; (4) Regeneration: regeneration for 5 seconds in the regeneration buffer; (5)Neutralization: neutralization for 5 seconds in the neutralization buffer; (6) Regeneration-Neutralization cycle for 3 times.

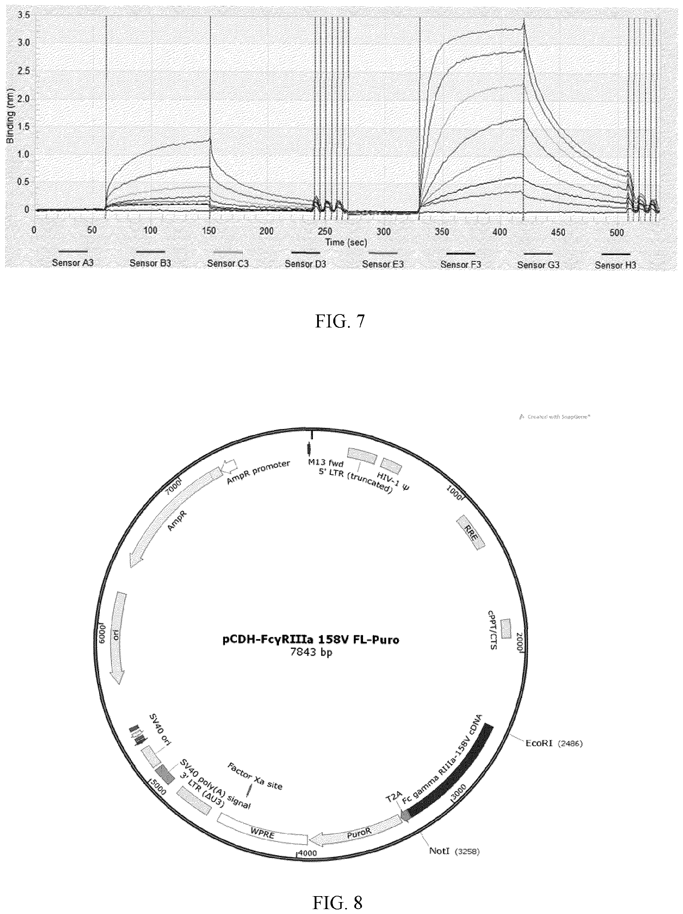

The data was processed and analyzed using Data Analysis 8.2, and the sample data was fitted after reference (sample blank) signal subtraction to obtain an affinity constant (KD). The results of affinity for FcγRIIIa 158F are shown in Table 3, and the results of affinity for FcγRIIIa 158V are shown in Table 4, wherein the association-dissociation sensorgram of antibody 2 and antibody 1 with FcγRIIIa 158V is shown in FIG. 7 (0-240 s represents the association-dissociation of antibody 1 with FcγRIIIa 158V, and 270-510 s represents the association-dissociation of antibody 2 with FcγRIIIa 158V).

**Table 3. Kinetic parameters of affinity association of antibody 2, antibody 1 with FcγRIIIa 158F**

| **Groups** | **FcγRIIIa 158F** | | |
|---|---|---|---|
| | **kon(1/Ms)** | **kdis(1/s)** | **KD (M)** |
| Antibody 1 | 1.19e+05 | 3.06e-02 | 2.58e-07 |
| Antibody 2 | 2.95e+05 | 1.97e-02 | 6.68e-08 |

| | | | |
|---|---|---|---|
| Note: kon: association rate; kdis: dissociation rate; KD: dissociation equilibrium constant, i.e., affinity. | | | |

**Table 4. Kinetic parameters of affinity association of antibody 2, antibody 1 with FcγRIIIa 158V**

| **Groups** | **FcγRIIIa 158V** | | |
|---|---|---|---|
| | **kon(1/Ms)** | **kdis(1/s)** | **KD (M)** |
| Antibody 1 | 3.63e+05 | 1.36e-02 | 3.73e-08 |
| Antibody 2 | 4.93e+05 | 8.98e-03 | 1.82e-08 |

| | | | |
|---|---|---|---|
| Note: kon: association rate; kdis: dissociation rate; KD: dissociation equilibrium constant, i.e., affinity. Conclusion: The results showed that antibody 2 enhanced by ADCC has a stronger affinity for FcγRIIIa than antibody 1. | | | |

### Example 5. In Vitro Biological Activity of Antibody 2 Protein and Antibody 1 Protein on ADCC Reporter Gene Model

The pCDH-FcγRIIIa 158V FL-Puro plasmid was constructed and the plasmid pattern is shown in FIG. 8. The construction process was as follows: the cDNA sequence of FcγRIIIa 158V FL set forth in SEQ ID NO: 10 was synthesized, placed in pUC-57 vector and named Puc-57-FcγRIIIa 158V FL cDNA, and the amino acid sequence was set forth in SEQ ID NO: 11. Forward and reverse PCR primers set forth in SEQ ID NO: 20 and SEQ ID NO: 21 were synthesized (for amplification of FcγRIIIa 158V FL cDNA fragment with EcoRI and NotI enzymatic digestion site at both ends of the fragment). PCR was performed using the forward and reverse primers described above with Puc-57-FcγRIIIa 158V FL cDNA as a template to obtain an amplified FcγRIIIa 158V FL cDNA fragment. EcoRI and NotI were used to perform digestion on pCDH-EF1-MCS-T2A-Puro plasmid (conventional plasmid, such as the one commercially available from Youbio, Cat.# VT1482) and FcγRIIIa 158V FL cDNA after primer amplification, the digested product was subjected to gel extraction, the gel-extracted product was subjected to ligation, *E. coli* transformation and sequencing, and the correct clone was named pCDH-FcγRIIIa 158V FL-Puro.

Packaging of the virus was performed using the lentivirus packaging systems pLP1, pLP2 and pLP/VSV-G (e.g., commercially available from Invitrogen) and pCDH-FcγRIIIa 158V FL-Puro, followed by infection of Jurkat cells. After 48 h, 0.3 µg/mL puromycin was added as a screening pressure. Two weeks after transfection, after the cells grew up, positive monoclonals were screened by detecting the expression of FcγRIIIa 158V by a flow cytometry using FITC-labeled anti-FcγRIIIa antibody (Invitrogen, Cat.# MHCD1601).
pGL4.30-luc2P-NFAT-RE-Hygro plasmid (commercially available from Promega) was transfected into the above-mentioned Jurkat monoclonal cells positive for FcγRIIIa 158V expression by electrotransfection, and pressure-screened with hygromycin. Positive monoclonals capable of emitting autofluorescence by stimulation of PMA and ionomycin were screened by stimulating the cells for 6 h by the PMA (50 µg/mL) and the ionomycin (1 µg/mL) and adding a luciferase substrate as well as a method for detecting the autofluorescence using a microplate reader, and were named Jurkat-NFAT-luciferase-FcγRIIIa 158V cells.

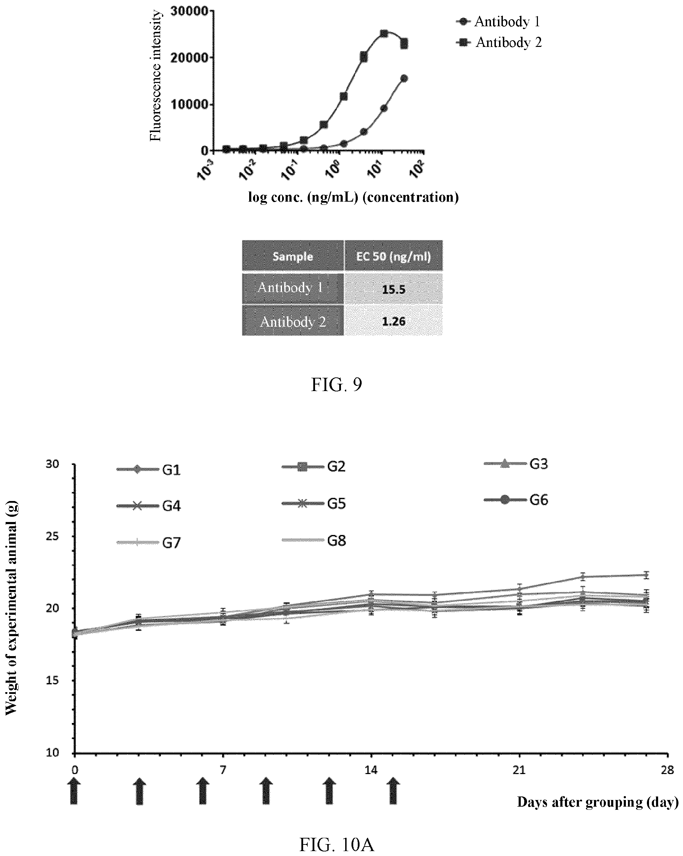

The density of Jurkat-NFAT-luciferase-FcγRIIIa 158V cells and Jurkat-IL2-luciferase-CTLA4FL cells was diluted to 200×10⁵ cells/mL using 1640 medium containing 1% FBS as the buffer in this example, and 25 µL of Jurkat-NFAT-luciferase-FcγRIIIa 158V and 25 µL of Jurkat-IL2-luciferase-CTLA4FL were added to each well of a 96-well white plate (Corning, Cat.# 3917). The concentration of the antibody 2 and the antibody 1 was diluted to 100 µg/mL using buffer, which was taken as an initial concentration for 3-fold gradient dilution for a total of 10 concentrations (the last one was 0). Antibody solutions with different concentrations were added into the above wells which have been added with the two cells (25 µL for each well), and 3 replicate wells were made for each concentration. At this time, 75 µL of the cell antibody mixture was presented in each well in total. The white plate was incubated in a 37 °C incubator for 6 h, and then the white plate was taken out and left at room temperature for 10 minutes. 75 µL of firefly luciferase substrate equilibrated to room temperature was added into each well, the white plate was placed in the microplate reader and was subjected to shaking for 5 s, and the fluorescence value was read after 10 minutes. The results are shown in FIG. 9.

Conclusion: In the ADCC reporter gene model, antibody 2 showed stronger *in vitro* ADCC activity than antibody 1, and the ADCC activity of antibody 2 in the *in vitro* model was about 12 times that of antibody 1 in terms of ECso value.

### Example 6. Anti-tumor Efficacy of Antibody 2 Protein and Antibody 1 Protein in Mice

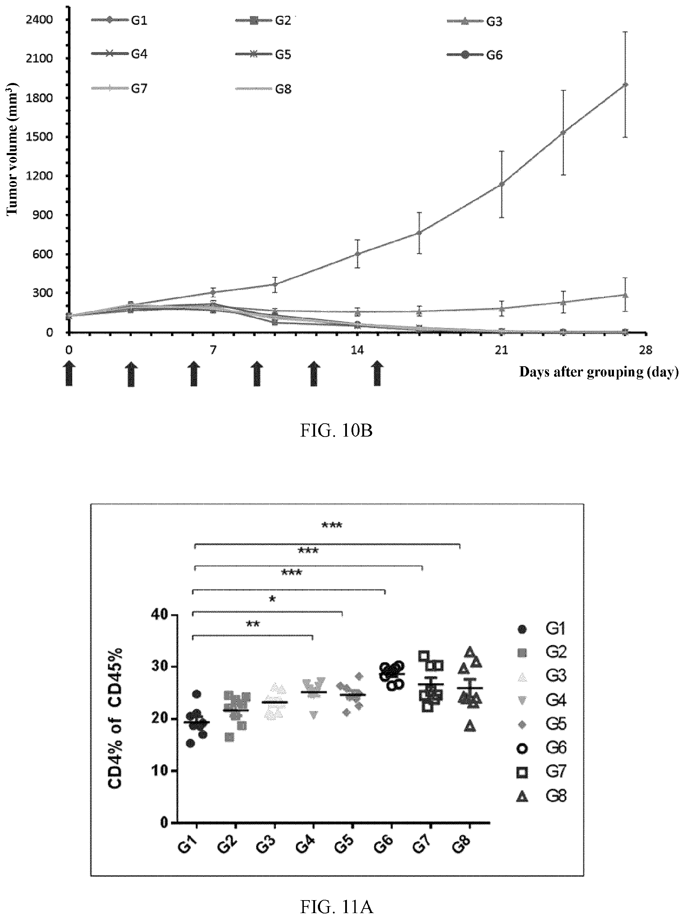

MC38 cells were inoculated subcutaneously into the right side of B-hCTLA4 humanized female mice at a concentration of 5×10⁵ cells/0.1 mL, and when tumors grew to about 125 mm³, the mice were randomly grouped, by tumor volume, into 8 groups (8 mice per group), namely control group G1 (0.9% sodium chloride injection solvent), group G2 (Yervoy^{®}, 1 mg/kg), group G3 (antibody 1, 0.3 mg/kg), group G4 (antibody 1, 1 mg/kg), group G5 (antibody 1, 3 mg/kg), group G6 (antibody 2, 0.3 mg/kg), group G7 (antibody 2, 1 mg/kg) and group G8 (antibody 2, 3 mg/kg). All groups were subjected to administration by intraperitoneal injection once every 3 days for 6 times, and the experiment ended 12 days after the last administration. Tumor volume and body weight of mice were measured and recorded twice a week. At the end of the experiment, the mice were euthanized, tumors were taken, weighed, and photographed, and the relative tumor inhibition (TGI%) was calculated. During the whole experiment, all experimental animals were well active and fed during administration, and the body weight of the experimental animals increased to some extent. No mice died during the experiment. The effect of antibody 2 and antibody 1 on the body weight of MC38 cell-transplanted B-hCTLA4 mice is shown in Table 5, and the body weight change trend of the animals after administration is shown in FIG. 10A. The effect of antibody 2 and antibody 1 on the tumor volume of MC38 cell-transplanted B-hCTLA4 mice is shown in Table 6, and the tumor volume increase trend after administration is shown in FIG. 10B.

**Table 5. Effect of antibody 2 and antibody 1 on body weight of MC38 cell-transplanted B-hCTLA4 mice**

| **Groups** | **Test drug** | **Body weight (g) ^{a}** | | | **27 days after administration Body weight change (g)** |
|---|---|---|---|---|---|
| | | **Before administration** | **27 days after the first administration** | ***P*^{b}** | |
| G1 | 0.9% sodium chloride injection | 18.4±0.3 | 22.3±0.2 | - | +3.9 |
| G2 | Yervoy^{®} | 18.2±0.2 | 20.4±0.3 | 0.000 | +2.2 |
| G3 | Antibody 1-low dose | 18.3±0.2 | 20.9±0.4 | 0.009 | +2.6 |
| G4 | Antibody 1-medium dose | 18.4±0.2 | 20.5±0.4 | 0.001 | +2.1 |
| G5 | Antibody 1-high dose | 18.3±0.3 | 20.2±0.5 | 0.001 | +1.9 |
| G6 | Antibody 2-low dose | 18.4±0.2 | 20.5±0.4 | 0.003 | +2.1 |
| G7 | Antibody 2-medium dose | 18.2±0.3 | 20.3±0.4 | 0.001 | +2.1 |
| G8 | Antibody 2-high dose | 18.3±0.3 | 20.8±0.3 | 0.001 | +2.5 |

| | | | | | |
|---|---|---|---|---|---|
| Note: a: mean ± standard error; b: the statistical comparison of the body weight of the administration groups with the body weight of the solvent control group at 27 days after the administration, t-test. | | | | | |

**Table 6. Effect of antibody 2 and antibody 1 on tumor volume of MC38 cell-transplanted B-hCTLA4 mice**

| **Groups** | **Test drug** | **Tumor volume (mm³) ^{a}** | | | ***P*^{b}** |
|---|---|---|---|---|---|
| | | **Before administration** | **27 days after the first administration** | **TGI (%)** | |
| G1 | 0.9% sodium chloride injection | 125±6 | 1901±404 | - | - |
| G2 | Yervoy^{®} | 125±5 | 0±0 | 107.1 | 0.000 |
| G3 | Antibody 1-low dose | 125±6 | 288±130 | 90.8 | 0.002 |
| G4 | Antibody 1-medium dose | 125±6 | 0±0 | 107.1 | 0.000 |
| G5 | Antibody 1-high dose | 125±5 | 0±0 | 107.1 | 0.000 |
| G6 | Antibody 2-low dose | 125±6 | 0±0 | 106.8 | 0.000 |
| G7 | Antibody 2-medium dose | 125±5 | 0±0 | 107.1 | 0.000 |
| G8 | Antibody 2-high dose | 125±6 | 0±0 | 107.0 | 0.000 |

| | | | | | |
|---|---|---|---|---|---|
| Note: a: mean ± standard error; b: the statistical comparison of the body weight of the administration groups with the body weight of the solvent control group at 27 days after the administration, t-test. | | | | | |

Conclusion: In the experimental efficacy model, the test antibody 2 and test antibody 1 both had a significant effect of inhibiting tumor growth, and the tumor inhibition effect thereof was equivalent to that of a positive control drug commercially available CTLA-4 antibody Yervoy^{®}. The test drug did not generate obvious toxic effect on animals while exerting anti-tumor growth effects, and had better safety. It is noted that the efficacy had reached to a saturation level at the medium dose and high dose groups with the same dose, so that the difference in the two groups was not significant; while at the low dose group of 0.3 mg/kg, the antibody 2 with ADCC enhancement had significantly better tumor inhibition effect than the antibody 1 without ADCC enhancement.

### Example 7. Effect of Antibody 2 on CD4^{÷}, CD8⁺ and Treg cells in Blood of Mice

In the efficacy study mentioned in Example 6, after 21 days of administration, 150 µL of anticoagulation was collected from the orbital angular vein plexus of each mouse, and CD4, CD8 positive cells and Treg cells were detected by flow assay, and the proportion of the above cells in CD45 positive cells was counted. The antibodies used herein include anti-CD45 (Biolegged, Cat.# 103138), anti-CD4 (Biolegged, Cat.# 100438), anti-CD8 (Biolegged, Cat.# 100759), and anti-Foxp3 (eBioscience, Cat.# 17-5773-82).

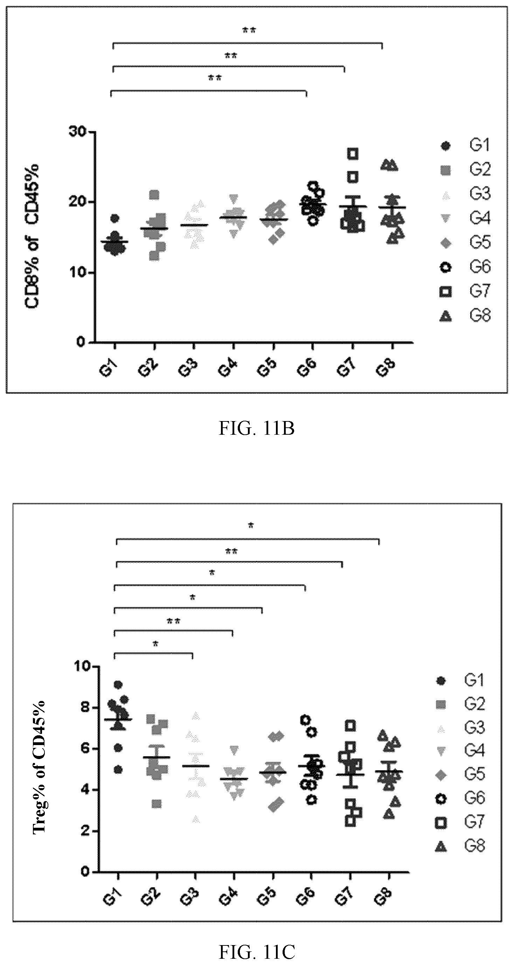

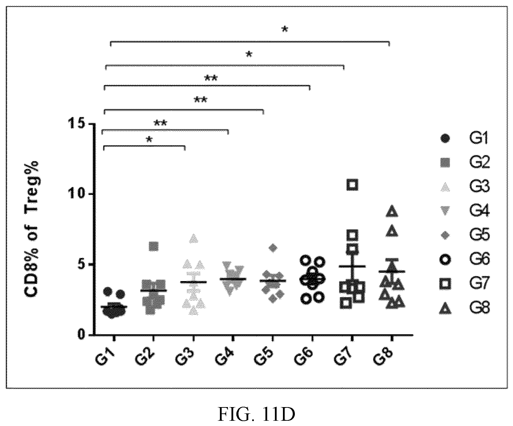

The results showed that the proportion of CD4 positive cells in the blood of mice in groups G4 and G5 significantly increased (P < 0.05, P < 0.01), and the proportion of CD4 positive cells in the blood of mice in groups G6, G7 and G8 extremely significantly increased (P < 0.01) 21 days after administration; the proportion of CD8 positive cells in the blood of mice of each antibody 1 administration group did not significantly change, while the proportion of CD8 positive cells in the blood of mice in groups G6, G7 and G8 extremely significantly increased (P < 0.01); the proportion of Treg cells in the blood of mice in groups G3, G4, G5, G6, G7 and G8 significantly reduced (P < 0.05, P < 0.01); the ratio of CD8 cells to Treg cells in the blood of mice in groups G3, G4, and G5 significantly increased (P < 0.05, P < 0.01). Schematic diagrams of comparison among each group are shown in FIGs. 11A-11D.

Conclusion: The proportion of CD4 and CD8 positive cells of the ADCC-enhanced antibody 2 significantly increased, the proportion of Treg cells thereof significantly reduced, and the ratio of CD8 cells to Treg cells significantly increased. It is suggested that the ADCC-enhanced CTLA-4 antibody resulted in stronger T cell activation, and thus stronger anti-tumor efficacy on the CTLA-4 humanized tumor-bearing mice.

### Example 8. Pharmacokinetic Study

### 1) Single intravenous infusion

Twenty-four cynomolgus monkeys, half male and half female, were selected and randomly divided into 3 groups according to their gender: low, medium and high dose groups, and the above three dose groups were administered 1 mg/kg antibody 2, 3 mg/kg antibody 2 and 10 mg/kg antibody 2, respectively, by a single intravenous infusion. The whole blood was collected before administration, immediately after the end of administration, and 1 h, 6 h, 24 h, 48 h, 3 days, 4 days, 5 days, 7 days, 9 days, 11 days, 14 days, 17 days, 21 days and 28 days after the start of administration, and serum was separated. The concentration of the drug in each sample was analyzed by a validated ELISA method, and the lower limit of the quantification was 15 ng/mL.

During the experiment, all animals had no abnormal clinical performance, and the pharmacokinetic parameters in the serum of each group of animals are shown in Table 7; the results showed that the change trend of drug concentration in the serum of each group of animals with different gender was basically consistent, and the drug concentration of the animal serum in the low, medium and high dose groups was positively correlated with the administration dose; each group had no gender difference in pharmacokinetic parameters (p > 0.05).

**Table 7. Pharmacokinetic parameters for single administration**

| **Groups** | **Gender** | | **t_{1/2} (h)** | **Cₘₐₓ (ug/mL)** | **AUCₗₐₛₜ (h*mg/mL)** | **AUC_{INF_obs} (h*mg/mL)** | **Vz_{_obs} (mL/kg)** | **Cl_{_obs} (mL/h/kg)** | **MRTₗₐₛₜ (h)** |
|---|---|---|---|---|---|---|---|---|---|
| Low dose group | Male | Mean | 180.07 | 19.78 | 2.30 | 2.48 | 107.20 | 0.42 | 185.41 |
| | | SD | 17.36 | 2.53 | 0.52 | 0.61 | 15.18 | 0.09 | 12.52 |
| | Female | Mean | 237.92 | 16.63 | 2.37 | 2.74 | 125.50 | 0.37 | 209.69 |
| | | SD | 35.88 | 1.25 | 0.20 | 0.29 | 14.37 | 0.04 | 9.30 |
| | Overall | Mean | 209.00 | 18.21 | 2.33 | 2.61 | 116.35 | 0.39 | 197.55 |
| | | SD | 40.49 | 2.48 | 0.36 | 0.45 | 16.59 | 0.07 | 16.56 |
| Medium dose group | Male | Mean | 173.10 | 62.01 | 7.75 | 8.39 | 86.09 | 0.37 | 178.99 |
| | | SD | 78.59 | 7.35 | 1.05 | 1.54 | 28.73 | 0.07 | 26.62 |
| | Female | Mean | 222.46 | 52.12 | 7.38 | 8.29 | 116.06 | 0.36 | 208.72 |
| | | SD | 19.97 | 7.06 | 0.35 | 0.57 | 2.63 | 0.03 | 7.45 |
| | Overall | Mean | 197.78 | 57.07 | 7.56 | 8.34 | 101.07 | 0.36 | 193.86 |
| | | SD | 57.98 | 8.42 | 0.73 | 1.04 | 24.54 | 0.05 | 23.89 |
| High dose group | Male | Mean | 133.70 | 187.34 | 18.46 | 19.03 | 101.73 | 0.53 | 164.50 |
| | | SD | 23.21 | 10.63 | 1.26 | 1.35 | 18.94 | 0.04 | 5.98 |
| | Female | Mean | 150.76 | 170.38 | 21.27 | 22.62 | 95.20 | 0.46 | 185.18 |
| | | SD | 50.44 | 17.40 | 4.06 | 5.03 | 22.51 | 0.10 | 22.39 |
| | Overall | Mean | 142.23 | 178.86 | 19.86 | 20.82 | 98.46 | 0.49 | 174.84 |
| | | SD | 36.34 | 15.89 | 3.10 | 3.84 | 18.95 | 0.08 | 18.52 |

### 2) Multiple intravenous infusions

Forty cynomolgus monkeys were selected and randomly divided into 4 groups according to their gender, with 10 monkeys in each group, half male and half female; the 4 groups were a vehicle control group, a low dose group (antibody 2, 0.2 mg/kg), a medium dose group (antibody 2, 0.6 mg/kg) and a high dose group (antibody 2, 2 mg/kg); the test sample or the vehicle control sample was administered by intravenous infusion once every 3 weeks for 13 weeks, i.e., 5 times on days 1, 22, 43, 64 and 85; the administration rate was about 0.2 mL/min/kg. The blood sampling time points for the control group were as follows: before administration and 2 h after the start of the administration on days 1 and 64; the blood sampling time points for the administration groups were as follow: the blood samples were collected and serum was separated before administration, immediately after the end of administration (±1 min), and 2 h, 6 h, 24 h, 72 h, 120 h, 168 h, 336 h, and 504 h after the start of administration on days 1 and 64. The concentration of test samples in serum was detected by employing a validated ELISA method, and its lower limit of the quantification was 15 ng/mL. The toxicokinetic parameters of test samples were calculated by using WinNonlin 8.0 Noncompartmental Analysis (NCA).

Within a range of 0.2-2 mg/kg, the change trend of the exposure level of the serum drugs was basically consistent with the change trend of time; exposure level in each dose group after the 4^{th} dose all increased to some extent.

**Table 8. Pharmacokinetic parameters for multiple administrations**

| **Dose** | **Gender** | | **First dose** | | **Fourth dose** | | |
|---|---|---|---|---|---|---|---|
| | | | **Cₘₐₓ (µg/mL)** | **AUCₗₐₛₜ (h·mg/mL)** | **Cₘₐₓ (µg/mL)** | **AUCₗₐₛₜ (h·mg/mL)** | **AF** |
| Low dose group | Male | Mean | 4.00 | 0.57 | 6.56 | 0.88 | 1.53 |
| | | SD | 1.13 | 0.03 | 0.72 | 0.14 | 0.19 |
| | Female | Mean | 4.62 | 0.55 | 6.53 | 0.86 | 1.57 |
| | | SD | 0.80 | 0.07 | 0.50 | 0.09 | 0.19 |
| | Overall | Mean | 4.31 | 0.56 | 6.54 | 0.87 | 1.55 |
| | | SD | 0.98 | 0.05 | 0.58 | 0.11 | 0.18 |
| Medium dose group | Male | Mean | 13.28 | 1.82 | 18.81 | 3.09 | 1.69 |
| | | SD | 1.24 | 0.13 | 1.74 | 0.46 | 0.14 |
| | Female | Mean | 13.38 | 1.68 | 16.77 | 2.43 | 1.37 |
| | | SD | 0.84 | 0.28 | 1.93 | 0.72 | 0.27 |
| | Overall | Mean | 13.33 | 1.75 | 17.90 | 2.80 | 1.55 |
| | | SD | 1.00 | 0.22 | 2.02 | 0.65 | 0.26 |
| High dose group | Male | Mean | 45.95 | 5.99 | 65.37 | 8.77 | 1.47 |
| | | SD | 3.43 | 0.62 | 6.34 | 1.09 | 0.20 |
| | Female | Mean | 42.42 | 4.99 | 60.87 | 7.93 | 1.53 |
| | | SD | 5.23 | 0.89 | 4.52 | 1.19 | 0.19 |
| | Overall | Mean | 44.19 | 5.49 | 63.37 | 8.40 | 1.50 |
| | | SD | 4.57 | 0.90 | 5.78 | 1.15 | 0.19 |

### Example 9. Toxicological Study

### 1) Acute toxicity experiment by single intravenous infusion

Eight cynomolgus monkeys were randomly divided into 4 groups (1/gender/group), group 1 was the vehicle control group, and groups 2, 3, and 4 were 10, 30, and 60 mg/kg test sample groups (antibody 2), respectively. Animals were administrated with a single intravenous infusion on day 1 at an administration capacity of 6 mL/kg and an infusion rate of 0.5 mL/min/kg.

The results showed that there were no death or dying animals across all groups during the experiment, and there was no abnormal change which was definitely related to the test sample (antibody 2) in the body temperature, the electrocardio, the blood biochemistry and the urine of each group of animals. Under the conditions of this experiment, 30 mg/kg and 60 mg/kg dose groups showed mild to severe atrophy of the thymus with a maximum tolerated dose (MTD) of 60 mg/kg.

### 2) Toxicity experiment by repeated intravenous infusions

Forty cynomolgus monkeys (20/gender) were randomly divided into 4 groups, with 10 animals in each group, half male and half female; group 1 was a vehicle control group, and animals in groups 2, 3 and 4 were administered with 0.2, 0.6 and 2 mg/kg of the test sample (antibody 2), respectively; administration was performed once every 3 weeks for 13 weeks (5 doses in total), and intravenous infusion was adopted with an administration capacity of 2 mL/kg and an infusion rate of about 0.2 mL/min/kg; the recovery period was 4 weeks.

Gastrointestinal reaction was seen in animals of high-dose group, recovery or recovery tendency was seen after drug withdrawal, toxic target organs were not seen in animals of each dose group, and the no-observed-adverse-effect level (NOAEL) was 2 mg/kg.

### 3) Immunotoxicity experiment by repeated intravenous infusions

The experiment was performed by combining with repeated intravenous infusions and long-term toxicology study with 4 weeks of recovery period, and mainly studied the effect of test sample (antibody 2) on T lymphocyte subpopulation, complement, cytokines and immunoglobulin of the cynomolgus monkey under the experiment condition; wherein group 1 was a solvent control group, and the animals in groups 2, 3 and 4 were administered with 0.2 mg/kg, 0.6 mg/kg and 2 mg/kg of the test sample (antibody 2), respectively, once every 3 weeks for 13 weeks (5 doses in total). During the experiment, cytokines (TNF-α, IFN-γ, IL-2, IL-4, IL-5 and IL-6) of the cynomolgus monkey in 3 dose groups had no abnormal change related to the antibody 2; compared with a control group, the immunoglobulin (IgG, IgM and IgA) of animals in each dose group had no statistical change; after repeated intravenous infusions of antibody 2 in cynomolgus monkeys, ADA (anti-drug antibody) was detected positive in the low, medium and high dose groups of 0/10, 1/10, 3/10.

### Example 10. In Vitro Hemolysis Experiment

The sodium chloride injection and the test sample (antibody 2) were added into a glass tube comprising 2% rabbit erythrocyte suspension, the glass tube was incubated in an electrical thermostat incubator for 3 h at 37 °C, and the dissolution or agglutination of the erythrocytes was observed. The experiment results showed that the test samples with concentrations of 10.1 mg/mL and 1 mg/mL did not cause hemolysis of rabbit erythrocyte *in vitro,* had no agglutination, and were suitable for safe clinical injection.

### Example 11. Clinical Study on Antibody 2

The study is a multi-center, open-label and dose escalation phase I clinical trial for evaluating safety, tolerability, and pharmacokinetic characteristics of antibody 2 injection for treating patients with advanced solid tumors, explores the maximum tolerated dose (MTD) and provides recommended doses for subsequent clinical trial; and the study preliminarily evaluates the immunogenicity and the anti-tumor efficacy of the antibody 2 injection.

In the first stage, a safe dose range was explored by adopting "3+3" dose escalation rule, wherein the doses were 0.1 mg/kg, 0.2 mg/kg, 0.5 mg/kg, 1 mg/kg and 2 mg/kg; the 5 dose groups were sequentially administered with the antibody 2 injection, and only after all subjects in the same dose group had completed DLT assessment, and it was determined that the maximum tolerated dose (MTD) was not explored, the exploration of the next higher dose group could be carried out until the MTD was explored or until the tolerability study in the highest dose group was completed. In the second phase, after dose escalation was completed, 1-2 tolerated doses were selected for melanoma (20-40 cases) for extension study, providing recommended doses for subsequent clinical trial.

The administration was made by intravenous infusion once every 3 weeks for 4 treatment cycles, with administration on day 1 of each cycle. During the study, use with caution/as appropriate was made in the following cases: 1) prevention of infusion reactions: if the n^{th} subject experienced the infusion reactions, prophylactic administration, limited to single or combined regimens of steroid hormone, analgesic and antihistamine, could be performed on the n^{th}, n+1^{th}, n+2^{th} ... subjects as appropriate in subsequent administrations; 2) treatment of infusion reactions: if infusion reactions occurred, steroid hormone and other drugs could be used as appropriate; 3) treatment of adverse events: researchers could use infliximab, methoxyimidazole, carmidazole, levothyroxine, steroid hormones and the like as appropriate to treat adverse events occurring during the study; 4) local treatment of tumors, such as palliative radiotherapy to bone pain. DLT evaluation period: day 21, i.e., treatment cycle 1. After the patient completed the end-of-study visit of the 4^{th} treatment cycle, the study was completed by continuing the observation visit for 6 weeks, and the safety, tolerability, pharmacokinetic characteristics, immunogenicity and preliminary efficacy of the antibody 2 injection were analyzed and summarized according to data collected by the trial.

The safety indexes include: vital signs, physical examination, laboratory tests (routine blood test, blood biochemistry, thyroid function, coagulation, urinalysis, stool routine (with occult blood test)), electrocardiogram, cardiac colour doppler, adverse events (including immune-related adverse events) and the like.

Subjects in all dose groups were required for blood sample collection at specific time points during treatment, 2 mL of blood samples were scheduled to be collected at each time point, the concentration level of antibody 2 in serum was detected, and the pharmacokinetic (PK) characteristics of the antibody 2 injection were studied. PK blood samples were collected from subjects before and after each administration in cycles 1 to 4. Intensive sampling was made in cycle 1 and cycle 3, and the PK characteristics of single and multiple administration steady state studies were studied. Specific PK parameters include: single administration: Cmax, Tₘₐₓ, T_{1/2}, CL, Vd, Ke, MRT, AUC_{(0-τ)} and AUC_{(0-∞)}; multiple administrations: C_{max, ss}, C_{avg,ss}, C_{min,ss}, AUC_{(0-τ),ss}, AUC_{(0-∞),ss}, T_{max,ss}, T_{1/2,ss}, CL, Vₛₛ, Ke, MRT, accumulation index (Rac) and fluctuation index (DF).

Subjects in all dose groups were required for blood sample collection at specific time points during treatment, 3.5 mL of blood samples were scheduled to be collected at each time point, anti-drug antibodies and neutralizing antibodies in serum were detected, and blood samples for immunogenicity assay were collected before each administration in cycles 1 to 4 and 21 days after the end of the last administration in cycle 4. The immunogenicity evaluation indexes include: the sample positive rate and individual positive rate of the anti-drug antibody (ADA), the titer of the ADA positive sample, and whether the ADA positive sample is a neutralizing antibody (Nab).

The anti-tumor efficacy of antibody 2 was assessed by using RECIST1.1 and iRECIST1.1 throughout the trial. Tumor imaging examinations (CT or MRI on chest, abdomen, pelvis and the like) were performed 28 days before the first administration, and the same imaging examination technique was used on the same part of the same patient throughout the trial. Tumor assessment after administration would be performed at weeks 6, 12, 18. The clinical efficacy evaluation include:
Objective response rate (ORR): a proportion of subjects in complete response (CR) and partial response (PR), and ORR at weeks 6, 12, and 18 are ORR6w, ORR12w and ORR18w, respectively;
Best overall response rate (BORR): the best ORR appeared in the tumor efficacy assessments at weeks 6, 12, and 18;

Duration of mitigation (DOR): the time from first assessment of objective response to first assessment of PD (progressive disease) or any-cause mortality before PD, reflecting the duration of ORR; and Progression-free survival (PFS): the time from the first dose to the appearance of objective progression or all-cause mortality of the tumor (whichever occurred first).

Dose-limiting toxicity (DLT) is defined as follows: the following events associated with the study drug that occurred in the patient from day 1 to day 21 after administration in cycle 1:
■ ≥ grade 2 of ocular AEs (adverse events) not falling to grade 1 within 2 weeks of receiving local treatment; or ≥ grade 2 of ocular AEs needing to be treated systemically;
■ ≥ grade 3 of non-haematologic toxicities (nausea, vomiting and the like can be relieved within 3 days of supportive treatment, except for ≥ grade 3 of infusion reaction occurred without prophylactic administration);
■ ≥ grade 4 of haematologic toxicities (including ≥ grade 3 of neutropenia with fever; however, ≥ grade 4 of neutropenia requires more than 7 days of administration to determine DLT); ≥ grade 4 of thrombocytopenia or grade 3 thrombocytopenia with bleeding.

### Example 12: Preparation of Antibody 2 Formulation

The preparation of antibody formulation comprises: preparing a buffer, performing UF/DF ultrafiltration, performing buffer exchange by ultrafiltration on an antibody solution by adopting the filtrated buffer, and then concentrating to obtain an antibody concentrate; preparing an excipients mother liquor containing a buffer and/or a stabilizer and/or a surfactant and/or a chelating agent according to the formula proportion, adjusting to a suitable pH; and adding an appropriate amount of the excipients mother liquor subjected to sterile filtration into an appropriate amount of the antibody concentrate to obtain a liquid formulation. The solvent used in this example was water for injection.

### 1) Buffer screening

Antibody stability study was performed using various buffer systems. In view of the properties of anti-CTLA-4 antibodies, the present invention, after initial screening, identified the following various buffer systems: His, HAc, CB, Sua and Tris.

The meanings of the above abbreviations are as follow:
His: 20 mM histidine buffer, pH 5.8;
HAc: 20 mM acetate buffer, pH 5.8;
CB: 20 mM citrate buffer, pH 5.8;
Sua: 20 mM succinate buffer, pH 5.8; and
Tris: 20 mM tris(hydroxymethyl)aminomethane, pH 7.0.

In the antibody-buffer system for stability experiment, the antibody 2 contained in His, HAc, CB and Sua was 10 mg/mL, the buffer was 20 mM, and the pH of the buffer was 5.8. The change trends of the monomer purity (SEC-HPLC, SEC for short) and the charge isomer (IEC-HPLC, IEC for short) were studied for the various formulas after standing for 4 weeks at a high temperature of 40 °C, and the change trends are shown in Table 9.

**Table 9. Buffer screening**

| Buffer type | His | HAc | CB | Sua | Tris |
|---|---|---|---|---|---|
| Buffer concentration (mM) | 20 | 20 | 20 | 20 | 20 |
| pH | 5.8 | 5.8 | 5.8 | 5.8 | 7.0 |
| Antibody 2 concentration (mg/mL) | 10 | 10 | 10 | 10 | 5 |
| SEC monomer purity (%) Day 0 | 99.67 | 99.64 | 99.59 | 99.64 | 99.57 |
| SEC monomer purity (%) Week 4 | 99.18 | 98.95 | 98.87 | 98.26 | 98.80 |
| SEC polymer (%) Day 0 | 0.29 | 0.33 | 0.38 | 0.36 | 0.39 |
| SEC polymer (%) Week 4 | 0.30 | 0.61 | 0.62 | 1.18 | 0.65 |
| IEC main peak content (%) Day 0 | 67.30 | 67.32 | 67.23 | 67.15 | 66.78 |
| IEC main peak content (%) Week 4 | 51.48 | 50.00 | 44.95 | 46.14 | 45.45 |
| IEC acidic variant content (%) Day 0 | 26.45 | 26.39 | 26.41 | 26.51 | 27.30 |
| IEC acidic variant content (%) Week 4 | 42.49 | 44.57 | 50.01 | 48.87 | 51.14 |

According to the above experimental results, histidine buffer was the optimal buffer, acetate buffer was the suboptimal, and Tris buffer was poor: the SEC-HPLC monomer purity and IEC-HPLC main peak content in histidine buffer were higher than those in other buffer systems. It can be seen that histidine buffer was optimal for protection of antibody 2.

### 2) Preliminary screening of pH range

The antibodies and buffers contained in the system for pH range screening are shown in Table 10.

**Table 10. pH range screening**

| Grouping | 1 | 2 | 3 | 4 | 5 | 6 | 7 |
|---|---|---|---|---|---|---|---|
| His buffer (mM) | 20 | 20 | 20 | 0 | 0 | 0 | 0 |
| HAc buffer (mM) | 0 | 0 | 0 | 20 | 20 | 20 | 0 |
| Tris buffer (mM) | 0 | 0 | 0 | 0 | 0 | 0 | 20 |
| pH | 5.5 | 6.0 | 6.5 | 4.5 | 5.0 | 5.5 | 7.0 |
| Antibody 2's concentration (mg/mL) | 10 | 10 | 10 | 10 | 10 | 10 | 10 |
| SEC monomer purity (%) Day 0 | 98.95 | 98.77 | 98.73 | 98.98 | 99.04 | 98.91 | 97.91 |
| SEC monomer purity (%) Week 4 | 95.43 | 95.65 | 95.50 | 94.38 | 94.93 | 94.77 | 94.75 |
| SEC polymer (%) Day 0 | 1.05 | 1.23 | 1.27 | 1.02 | 0.96 | 1.09 | 2.09 |
| SEC polymer (%) Week 4 | 1.17 | 1.22 | 1.25 | 1.29 | 1.57 | 2.10 | 2.41 |
| IEC main peak content (%) Day 0 | 67.59 | 67.38 | 67.52 | 67.38 | 67.24 | 66.84 | 66.52 |
| IEC main peak content (%) Week 4 | 49.18 | 49.40 | 44.17 | 43.41 | 44.56 | 46.69 | 46.51 |
| IEC acidic variant content (%) Day 0 | 25.01 | 25.06 | 25.38 | 24.96 | 25.01 | 24.97 | 25.39 |
| IEC acidic variant content (%) Week 4 | 41.60 | 43.02 | 49.94 | 44.89 | 46.19 | 45.26 | 47.19 |

According to the above results, when the antibody was added to the buffer in the high temperature (40 °C) test (no other components were added to the buffer), and when the buffer system was histidine buffer at pH 5.5-6.5 (excluding 6.5), the SEC-HPLC polymer and IEC-HPLC acidic variant contents were relatively low, and the SEC-HPLC monomer purity and IEC-HPLC main peak contents were relatively high.

### 3) pH fine screening

Fine screening of pH range was performed in histidine buffer system, the antibody, the buffer, the stabilizer, the metal ion chelating agent and the surfactant contained in each system are shown in Table 11; the molar concentration of sucrose at 82 mg/mL was 240 mM.

**Table 11. pH fine screening**

| Grouping | 1 | 2 | 3 | 4 | 5 | 6 | 7 |
|---|---|---|---|---|---|---|---|
| His buffer (mM) | 20 | 20 | 20 | 20 | 20 | 20 | 20 |
| pH | 5.2 | 5.4 | 5.6 | 5.8 | 6.0 | 6.2 | 6.4 |
| Antibody 2 (mg/mL) | 10 | 10 | 10 | 10 | 10 | 10 | 10 |
| Sucrose (mg/mL) | 82 | 82 | 82 | 82 | 82 | 82 | 82 |
| Disodium edetate dihydrate (mg/mL) | 0.037 | 0.037 | 0.037 | 0.037 | 0.037 | 0.037 | 0.037 |
| TW80 (mg/mL) | 0.2 | 0.2 | 0.2 | 0.2 | 0.2 | 0.2 | 0.2 |
| SEC monomer purity (%) Day 0 | 99.17 | 99.59 | 99.64 | 99.65 | 99.67 | 99.66 | 99.68 |
| SEC monomer purity (%) Week 4 | 99.10 | 99.19 | 99.23 | 99.27 | 99.27 | 99.26 | 99.24 |
| SEC polymer (%) Day 0 | 0.78 | 0.38 | 0.33 | 0.32 | 0.30 | 0.30 | 0.30 |
| SEC polymer (%) Week 4 | 0.27 | 0.28 | 0.28 | 0.28 | 0.28 | 0.29 | 0.31 |
| IEC main peak content (%) Day 0 | 69.17 | 69.06 | 69.15 | 69.12 | 69.05 | 69.05 | 69.08 |
| IEC main peak content (%) Week 4 | 49.16 | 50.06 | 50.73 | 51.26 | 51.08 | 50.53 | 47.75 |
| IEC acidic variant content (%) Day 0 | 23.17 | 23.22 | 23.30 | 23.32 | 23.41 | 23.33 | 23.51 |
| IEC acidic variant content (%) Week 4 | 41.66 | 41.82 | 41.66 | 42.09 | 42.76 | 43.74 | 47.37 |

According to the above results, when the buffer added into the antibody 2 were added with the stabilizer, the metal ion chelating agent and the surfactant, and when the buffer system was the histidine buffer at pH 5.4-6.2, the stability of the antibody 2 was good, and better when the pH value was 5.8.

### 4) Surfactant shaking stability study

The antibody, buffer, stabilizer, metal ion chelating agent and surfactant contained in the system for surfactant screening are shown in Table 12.

In an antibody formulation, aggregation was likely to occur and insoluble particles were easily generated under vigorous shaking, which could be prevented by adding a certain amount of the surfactant. In this experiment, 0.1 mg/mL polysorbate 20, 0.2 mg/mL polysorbate 20, 0.4 mg/mL polysorbate 20, 0.1 mg/mL polysorbate 80, 0.2 mg/mL polysorbate 80 and 0.4 mg/mL polysorbate 80 were added to the formula containing the buffer, stabilizer, and metal ion chelating agent, and the mass and turbidity of the sample after shaking for 72 h were observed. The experimental conditions were as follows: the formulation of the combination of different surfactants was laid flat at 200 rpm, and shaken for 72 h at room temperature, and the solution properties, SEC, IEC and turbidity were detected at 0 h and 72 h of shaking, and the results are shown in Table 12.

**Table 12. Surfactant screening**

| Grouping | 1 | 2 | 3 | 4 | 5 | 6 | 7 |
|---|---|---|---|---|---|---|---|
| His buffer (mM) | 20 | 20 | 20 | 20 | 20 | 20 | 20 |
| pH | 5.8 | 5.8 | 5.8 | 5.8 | 5.8 | 5.8 | 5.8 |
| Antibody 2 (mg/mL) | 10 | 10 | 10 | 10 | 10 | 10 | 10 |
| Sucrose (mg/mL) | 82 | 82 | 82 | 82 | 82 | 82 | 82 |
| Disodium edetate dihydrate (mg/mL) | 0.037 | 0.037 | 0.037 | 0.037 | 0.037 | 0.037 | 0.037 |
| Polysorbate 80 (mg/mL) | 0.1 | 0.2 | 0.4 | 0 | 0 | 0 | 0 |
| Polysorbate 20 (mg/mL) | 0 | 0 | 0 | 0.1 | 0.2 | 0.4 | 0 |
| Solution property | Transparent No particles | Transparent No particles | Transparent No particles | Transparent No particles | Transparent No particles | Transparent No particles | Transparent No particles |
| SEC monomer purity (%) 0 h | 99.18 | 99.17 | 99.16 | 99.14 | 99.11 | 99.10 | 99.11 |
| SEC monomer purity (%) 72 h | 99.03 | 99.10 | 99.05 | 99.01 | 99.04 | 99.04 | 98.72 |
| SEC polymer (%) 0 h | 0.35 | 0.34 | 0.34 | 0.33 | 0.35 | 0.34 | 0.33 |
| SEC polymer (%) 72 h | 0.44 | 0.38 | 0.40 | 0.41 | 0.35 | 0.39 | 0.65 |
| IEC main peak content (%) 0 h | 64.43 | 64.51 | 64.80 | 64.87 | 64.93 | 64.93 | 64.79 |
| IEC main peak content (%) 72 h | 65.23 | 65.39 | 65.34 | 65.37 | 65.21 | 65.24 | 65.86 |
| IEC acidic variant content (%) 0 h | 19.31 | 19.27 | 19.22 | 19.43 | 19.55 | 19.61 | 19.69 |
| IEC acidic variant content (%) 72 h | 20.02 | 20.11 | 20.17 | 20.21 | 20.33 | 20.39 | 20.11 |
| Turbidity OD340 0 h | 0.037 | 0.038 | 0.038 | 0.036 | 0.032 | 0.038 | 0.040 |
| Turbidity OD340 72 h | 0.042 | 0.042 | 0.039 | 0.040 | 0.040 | 0.040 | 0.901 |

The results showed that the added 0.1-0.4 mg/mL surfactant (polysorbate 20 or polysorbate 80) protected the antibody protein during the shaking process, and the turbidity was not obviously changed.

### 5) Formula of liquid formulation

The liquid formulation of the present invention could stably store the active ingredient antibody 2, and the formulas of the formulation with better stability (such as liquid formulations A, B, C, D, E, F, G, H, I of the present invention, wherein formulation J was used as a comparative formulation) are shown in Tables below.

**Table 13A. Formula of liquid formulation A**

| Components | Formula amount | 5 mL of the formulation |
|---|---|---|
| Antibody 2 | 10 mg/mL | 50 mg |
| L-histidine | 1.24 mg/mL (8mM) | 6.2 mg |
| L-histidine hydrochloride monohydrate | 2.52 mg/mL (12 mM) | 12.6 mg |
| Trehalose dihydrate | 85 mg/mL (225 mM) | 425 mg |
| Disodium edetate dihydrate | 0.037 mg/mL | 0.185 mg |
| Polysorbate 80 | 0.2 mg/mL | 1 mg |
| pH | 5.8 | |

**Table 13B. Formula of liquid formulation B**

| Components | Formula amount | 5 mL of the formulation |
|---|---|---|
| Antibody 2 | 10 mg/mL | 50 mg |
| L-histidine | 1.24 mg/mL | 6.2 mg |
| L-histidine hydrochloride monohydrate | 2.52 mg/mL | 12.6 mg |
| Sucrose | 82 mg/mL (240 mM) | 410 mg |
| Disodium edetate dihydrate | 0.037 mg/mL | 0.185 mg |
| Polysorbate 80 | 0.2 mg/mL | 1 mg |
| pH | 5.8 | |

**Table 13C. Formula of liquid formulation C**

| Components | Formula amount | 5 mL of the formulation |
|---|---|---|
| Antibody 2 | 10 mg/mL | 50 mg |
| L-histidine | 1.24 mg/mL | 6.2 mg |
| L-histidine hydrochloride monohydrate | 2.52 mg/mL | 12.6 mg |
| Mannitol | 45 mg/mL (247 mM) | 225 mg |
| Disodium edetate dihydrate | 0.037 mg/mL | 0.185 mg |
| Polysorbate 80 | 0.2 mg/mL | 1 mg |
| pH | 5.8 | |

**Table 13D. Formula of liquid formulation D**

| Components | Formula amount | 5 mL of the formulation |
|---|---|---|
| Antibody 2 | 10 mg/mL | 50 mg |
| Acetate buffer | 20 mM | 0.1 moL |
| Trehalose dihydrate | 85 mg/mL (225 mM) | 425 mg |
| Disodium edetate dihydrate | 0.037 mg/mL | 0.185 mg |
| Polysorbate 80 | 0.2 mg/mL | 1 mg |
| pH | 5.8 | |

**Table 13E. Formula of liquid formulation E**

| Components | Formula amount | 5 mL of the formulation |
|---|---|---|
| Antibody 2 | 10 mg/mL | 50 mg |
| Acetate buffer | 20 mM | 0.1 moL |
| Sucrose | 82 mg/mL | 410 mg |
| Disodium edetate dihydrate | 0.037 mg/mL | 0.185 mg |
| Polysorbate 80 | 0.2 mg/mL | 1 mg |
| pH | 5.8 | |

**Table 13F. Formula of liquid formulation F**

| Components | Formula amount | 5 mL of the formulation |
|---|---|---|
| Antibody 2 | 10 mg/mL | 50 mg |
| Acetate buffer | 20 mM | 0.1 moL |
| Mannitol | 45 mg/mL (247 mM) | 225 mg |
| Disodium edetate dihydrate | 0.037 mg/mL | 0.185 mg |
| Polysorbate 80 | 0.2 mg/mL | 1 mg |
| pH | 5.8 | |

**Table 13G. Formula of liquid formulation G**

| Components | Formula amount | 5 mL of the formulation |
|---|---|---|
| Antibody 2 | 10 mg/mL | 50 mg |
| L-histidine | 0.62 mg/mL (4 mM) | 3.1 mg |
| L-histidine hydrochloride monohydrate | 1.26 mg/mL (6 mM) | 6.3 mg |
| Acetate buffer | 10 mM | 0.05 moL |
| Trehalose dihydrate | 85 mg/mL | 425 mg |
| Disodium edetate dihydrate | 0.037 mg/mL | 0.185 mg |
| Polysorbate 80 | 0.2 mg/mL | 1 mg |
| pH | 5.8 | |

**Table 13H. Formula of liquid formulation H**

| Components | Formula amount | 5 mL of the formulation |
|---|---|---|
| Antibody 2 | 10 mg/mL | 50 mg |
| L-histidine | 0.62 mg/mL | 3.1 mg |
| L-histidine hydrochloride monohydrate | 1.26 mg/mL | 6.3 mg |
| Acetate buffer | 10 mM | 0.05 moL |
| Sucrose | 82 mg/mL | 410 mg |
| Disodium edetate dihydrate | 0.037 mg/mL | 0.185 mg |
| Polysorbate 80 | 0.2 mg/mL | 1 mg |
| pH | 5.8 | |

**Table 13I. Formula of liquid formulation I**

| Components | Formula amount | 5 mL of the formulation |
|---|---|---|
| Antibody 2 | 10 mg/mL | 50 mg |
| L-histidine | 0.62 mg/mL | 3.1 mg |
| L-histidine hydrochloride monohydrate | 1.26 mg/mL | 6.3 mg |
| Acetate buffer | 10 mM | 0.05 moL |
| Mannitol | 45 mg/mL | 225 mg |
| Disodium edetate dihydrate | 0.037 mg/mL | 0.185 mg |
| Polysorbate 80 | 0.2 mg/mL | 1 mg |
| pH | 5.8 | |

**Table 13J. Formula of liquid formulation J**

| Components | Formula amount | 10 mL of the formulation |
|---|---|---|
| Antibody 2 | 5 mg/mL | 50 mg |
| Tris hydroxymethyl aminomethane hydrochloride | 3.15 mg/mL | 31.5 mg |
| Mannitol | 10 mg/mL | 100 mg |
| Sodium chloride | 5.85 mg/mL | 58.5 mg |
| DTPA | 0.04 mg/mL | 0.4 mg |
| Polysorbate 80 | 0.1 mg/mL | 1 mg |
| pH | 7.0 | |

### 6) Freeze-thaw experiment

Insoluble particles were easily generated in the antibody formulation when the antibody formulation was subjected to a freeze/thaw cycle, and the antibody could be stabilized by adding the stabilizer and the surfactant. The results of repeated freeze-thaw of the liquid formulation B of the present invention are shown in Table 14.

**Table 14. Freeze-thaw experimental results for liquid formulation B**

| Experimental conditions | | One freeze-thaw cycle: freezing at -80 °C for 24 h, thawing at 37 °C, and standing for 24 h | | |
|---|---|---|---|---|
| Test items | | Freeze-thaw 0 | Freeze-thaw 3 | Freeze-thaw 5 |
| Solution property | | Transparent No particles | Transparent No particles | Transparent No particles |
| SEC-HPLC (%) | Polymer | 0.83 | 0.41 | 0.42 |
| | Monomer purity | 95.22 | 95.75 | 95.66 |
| | Fragment | 3.95 | 3.84 | 3.92 |
| IEC-HPLC (%) | Acidic variant | 26.83 | 27.50 | 27.66 |
| | Main peak | 66.06 | 65.20 | 65.08 |
| | Base variant | 7.11 | 7.30 | 7.27 |
| Insoluble microparticles | ≥ 10 µm | 1.25 | 9.58 | 0.83 |
| | ≥25 µm | 1.25 | 9.17 | 0.42 |

The results showed that the liquid formulation B was frozen at -80 °C, thawed at 37 °C, and subjected to five repeated freeze-thaw cycles, and the clarity, the color, the insoluble particles, SEC-HPLC and IEC-HPLC had no obvious change, which indicates that the formulation had stable property in repeated freeze-thaw experiments, had no precipitate, and did not affect the property of the antibody 2.

### 7) Accelerated stability study

The formulation A, the formulation B, the formulation C, the formulation D, the formulation E, the formulation F, the formulation G, the formulation H, the formulation I and the formulation J were placed in a thermostat incubator for 6 months at 25 ± 2 °C, and sampling was performed at the end of months 0, 2, 3 and 6. The monomer purity (SEC-HPLC), charge isomer (IEC-HPLC) and nonreduced capillary gel electrophoresis (CE-SDS (NR)) of the antibody 2 were observed according to the stability focused observation items, and the results are shown in Tables 15 and 16.

**Table 15. SEC-HPLC results of accelerated stability study**

| Monomer purity by SEC-HPLC (%) | | | | |
|---|---|---|---|---|
| Formulation | Month 0 | Month 2 | Month 3 | Month 6 |
| Formulation A | 98.97 | 98.95 | 98.91 | 99.03 |
| Formulation B | 99.00 | 98.90 | 98.92 | 99.07 |
| Formulation C | 99.06 | 98.96 | 98.88 | 99.00 |
| Formulation D | 99.05 | 98.88 | 98.83 | 98.92 |
| Formulation E | 99.02 | 98.88 | 98.83 | 98.92 |
| Formulation F | 98.97 | 98.86 | 98.81 | 98.93 |
| Formulation G | 98.99 | 98.96 | 98.90 | 99.02 |
| Formulation H | 98.90 | 98.95 | 98.88 | 99.05 |
| Formulation I | 98.98 | 98.91 | 98.78 | 98.95 |
| Formulation J | 98.87 | 98.63 | 98.58 | 98.62 |

| SEC-HPLC polymer (%) | | | | |
|---|---|---|---|---|
| Formulation A | 1.03 | 0.90 | 0.87 | 0.97 |
| Formulation B | 1.00 | 0.94 | 0.87 | 0.93 |
| Formulation C | 0.94 | 0.88 | 0.91 | 1.00 |
| Formulation D | 0.95 | 0.96 | 0.96 | 1.08 |
| Formulation E | 0.99 | 0.96 | 0.96 | 1.08 |
| Formulation F | 1.02 | 0.98 | 0.99 | 1.07 |
| Formulation G | 1.01 | 0.88 | 0.91 | 0.98 |
| Formulation H | 1.10 | 0.89 | 0.91 | 0.95 |
| Formulation I | 1.02 | 0.93 | 0.99 | 1.05 |
| Formulation J | 1.13 | 1.20 | 1.20 | 1.38 |

**Table 16. IEC-HPLC results of accelerated stability study**

| IEC-HPLC main peak content (%) | | | | |
|---|---|---|---|---|
| Formulation | Month 0 | Month 2 | Month 3 | Month 6 |
| Formulation A | 67.33 | 65.81 | 64.69 | 59.02 |
| Formulation B | 67.34 | 66.01 | 64.46 | 59.29 |
| Formulation C | 67.41 | 65.58 | 64.37 | 58.95 |
| Formulation D | 67.05 | 65.45 | 64.04 | 58.49 |
| Formulation E | 66.93 | 65.57 | 64.13 | 58.59 |
| Formulation F | 67.09 | 65.6 | 64.18 | 58.52 |
| Formulation G | 67.08 | 65.57 | 64.23 | 58.48 |
| Formulation H | 67.14 | 65.64 | 64.29 | 58.77 |
| Formulation I | 66.75 | 65.54 | 64.02 | 58.46 |
| Formulation J | 66.65 | 61.34 | 59.04 | 49.57 |

| IEC-HPLC acidic variant content (%) | | | | |
|---|---|---|---|---|
| Formulation A | 25.00 | 28.80 | 30.30 | 34.45 |
| Formulation B | 24.99 | 28.53 | 30.43 | 34.09 |
| Formulation C | 25.03 | 28.99 | 30.62 | 34.53 |
| Formulation D | 25.32 | 29.11 | 30.93 | 34.92 |
| Formulation E | 25.34 | 29.02 | 30.84 | 34.74 |
| Formulation F | 25.28 | 29.05 | 30.75 | 34.93 |
| Formulation G | 25.26 | 29.26 | 30.96 | 35.10 |
| Formulation H | 25.19 | 29.16 | 30.93 | 34.80 |
| Formulation I | 25.42 | 29.31 | 31.22 | 35.20 |
| Formulation J | 25.96 | 34.29 | 37.19 | 45.84 |

The results showed that the monomer of the formulation J reduced faster than other formulations in SEC-HPLC, which is shown as the increase of monomer; the IEC-HPLC main peak reduced rapidly, which is shown as the rapid increase of the acidic variant; this means that comparative formulation J could not stably store antibody 2, while the formulations A-I of the present invention could stably preserve antibody 2, especially the formulation B of the present invention.

### 8) Compatibility testing

Through accelerated stability experiments and long-term stability experiments, the liquid formulation of the present invention had good compatibility with vials and rubber stoppers, and met the packaging requirements.
SEQ ID NO: 1, heavy chain amino acid sequence of anti-CTLA-4 antibody 
QVQLVESGGGVVQPGRSLRLSCAASGFTFSSYTMHWVRQAPGKGLEWVTFISYDGNNKY
YADSVKGRFTISRDNSKNTLYLQMNSLRAEDTAIYYCARTGWLGPFDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCS
VMHEALHNHYTQKSLSLSPGK
SEQ ID NO: 2, light chain amino acid sequence of anti-CTLA-4 antibody 
EIVLTQSPGTLSLSPGERATLSCRASQSVGSSYLAWYQQKPGQAPRLLIYGAFSRATGIPDRF
SGSGSGTDFTLTISRLEPEDFAVYYCQQYGSSPWTFGQGTKVEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC
SEQ ID NO: 3, heavy chain nucleic acid sequence of anti-CTLA-4 antibody 
CAGGTCCAGCTGGTCGAATCTGGCGGCGGCGTCGTCCAGCCTGGAAGATCTCTGAGACT
GTCTTGCGCTGCTTCTGGCTTTACCTTCTCTTCTTACACAATGCATTGGGTCAGACAGGC
TCCTGGTAAAGGTCTGGAGTGGGTCACATTCATCTCTTACGACGGCAACAACAAGTACT
ACGCCGATTCTGTCAAGGGCAGATTCACCATCTCCAGAGACAACTCCAAGAACACCCTG
TACCTGCAGATGAACTCTCTGAGAGCTGAGGATACCGCTATCTACTATTGCGCTAGAACA
GGCTGGCTGGGACCTTTTGATTACTGGGGACAGGGAACACTGGTCACAGTCTCTTCTGC
TTCTACCAAAGGACCTTCTGTCTTTCCTCTGGCTCCTTCTTCTAAGTCTACCTCTGGCGG
AACAGCTGCTCTGGGTTGTCTGGTCAAGGATTACTTCCCTGAACCGGTGACAGTGTCTT
GGAATTCTGGAGCTCTGACCTCAGGAGTCCATACATTTCCTGCTGTCCTGCAGTCTTCTG
GCCTGTATTCTCTGTCCTCTGTGGTGACAGTCCCTTCTTCTTCTCTGGGAACACAGACCT
ACATCTGCAACGTCAACCACAAGCCTTCCAACACCAAGGTCGACAAGAGAGTGGAGCC
TAAGTCTTGCGACAAGACACATACATGTCCACCGTGCCCAGCACCTGAACTCCTGGGGG
GACCGTCAGTCTTCCTCTTCCCCCCAAAACCCAAGGACACCCTCATGATCTCCCGGACC
CCTGAGGTCACATGCGTGGTGGTGGACGTGAGCCACGAAGACCCTGAGGTCAAGTTCA
ACTGGTACGTGGACGGCGTGGAGGTGCATAATGCCAAGACAAAGCCGCGGGAGGAGCA
GTACAACAGCACGTACCGTGTGGTCAGCGTCCTCACCGTCCTGCACCAGGACTGGCTGA
ATGGCAAGGAGTACAAGTGCAAGGTCTCCAACAAAGCCCTCCCAGCCCCCATCGAGAA
AACCATCTCCAAAGCCAAAGGGCAGCCCCGAGAACCACAGGTGTACACCCTGCCCCCA
TCCCGGGATGAGCTGACCAAGAACCAGGTCAGCCTGACCTGCCTGGTCAAAGGCTTCT
ATCCCAGCGACATCGCCGTGGAGTGGGAGAGCAATGGGCAGCCGGAGAACAACTACAA
GACCACGCCTCCCGTGCTGGACTCCGACGGCTCCTTCTTCCTCTACAGCAAGCTCACCG
TGGACAAGAGCAGGTGGCAGCAGGGGAACGTCTTCTCATGCTCCGTGATGCATGAGGC
TCTGCACAACCACTACACGCAGAAGAGCCTCTCCCTGTCTCCGGGTAAA
SEQ ID NO: 4, light chain nucleic acid sequence of anti-CTLA-4 antibody 
GAAATTGTCCTGACACAGTCTCCTGGAACACTGTCTCTGTCTCCTGGAGAGAGAGCTAC
ACTGTCTTGTAGGGCTTCTCAGTCTGTGGATCTTCTTACCTGGCTTGGTATCAGCAGAA
ACCTGGACAGGCTCCTAGACTGCTGATCTACGGCGCTTTTCTAGAGCTACCGGAATCCC
TGACAGATTCTCTGGATCTGGCTCTGGAACCGATTTCACCCTGACCATCTCTAGACTGGA
ACCTGAGGACTTTGCTGTCTACTACTGTCAGCAGTACGGCTCTTCTCCTTGGACATTGG
ACAGGGAACCAAGGTCGAGATCAAGCGTACGGTGGCTGCACCATCTGTCTTCATCTTCC
CGCCATCTGATGAGCAGTTGAAATCTGGAACTGCCTCTGTTGTGTGCCTGCTGAATAACT
TCTATCCCAGAGAGGCCAAAGTACAGTGGAAGGTGGATAACGCCCTCCAATCGGGTAAC
TCCCAGGAGAGTGTCACAGAGCAGGACAGCAAGGACAGCACCTACAGCCTCAGCAGC
ACCCTGACGCTGAGCAAAGCAGACTACGAGAAACACAAAGTCTACGCCTGCGAAGTCA
CCCATCAGGGCCTGAGCTCGCCCGTCACAAAGAGCTTCAACAGGGGAGAGTGT
SEQ ID NO: 5, nucleic acid sequence of CTLA-4-ECD-Fc 
ATGCACGTGGCTCAGCCAGCAGTGGTCCTGGCTTCTTCTAGAGGAATCGCTTCTTTCGTC
TGCGAATACGCTTCTCCAGGAAAGGCTACAGAAGTGAGAGTGACAGTGCTGAGACAGG
CAGATTCTCAGGTGACAGAGGTCTGCGCAGCTACATACATGATGGGAAACGAGCTGACC
TTTCTGGACGATTCTATTTGTACCGGCACATCTTCCGGCAATCAGGTGAATCTGACCATTC
AGGGACTGAGAGCTATGGACACAGGCCTGTACATTTGCAAGGTGGAGCTGATGTACCCT
CCTCCTTATTACCTGGGAATCGGAAACGGAACACAGATCTACGTGATCGACCCAGAACC
TTGTCCAGATTCCGATCAGGAACCTAAGTCTTCCGACAAGACCCATACATCTCCTCCTTC
TCCAGCTCCAGAACTGCTGGGAGGATCTTCAGTGTTTCTGTTCCCTCCTAAGCCTAAGG
ATACCCTGATGATCTCCAGAACACCAGAAGTGACTTGCGTGGTGGTGGACGTGTCTCAC
GAAGATCCAGAAGTGAAGTTCAATTGGTACGTGGACGGAGTGGAAGTGCATAACGCTA
AGACCAAGCCTAGAGAGGAGCAGTACAACTCCACCTATAGAGTGGTGTCCGTGCTGAC
AGTGCTGCATCAGGATTGGCTGAACGGAAAGGAGTACAAGTGCAAGGTGTCCAACAAA
GCTCTGCCAGCTCCTATCGAGAAGACAATCTCCAAGGCTAAGGGACAGCCTAGAGAACC
TCAGGTGTATACACTGCCTCCTTCTAGAGACGAGCTGACAAAGAACCAGGTGTCTCTGA
CTTGTCTGGTGAAGGGCTTCTATCCTTCTGATATCGCCGTCGAGTGGGAATCTAACGGAC
AGCCAGAGAACAACTACAAGACCACACCTCCAGTGCTGGATTCAGACGGATCTTTCTTC
CTGTACTCCAAGCTGACCGTGGACAAAAGTAGATGGCAGCAGGGAAACGTGTTCTCTT
GTAGCGTGATGCACGAAGCTCTGCATAACCACTACACCCAGAAGTCTCTGTCTCTGTCTC
CAGGAAAA
SEQ ID NO: 6, amino acid sequence of CTLA-4-ECD-Fc
SEQ ID NO: 7, nucleic acid sequence of OKT3 ScFv 
CAGGTCCAGCTGCAGCAGTCTGGGGCTGAACTGGCAAGACCTGGGGCCTCAGTGAAGA
TGTCCTGCAAGGCTTCTGGCTACACCTTTACTAGGTACACGATGCACTGGGTAAAACAG
AGGCCTGGACAGGGTCTGGAATGGATTGGATACATTAATCCTAGCCGTGGTTATACTAAT
TACAATCAGAAGTTCAAGGACAAGGCCACATTGACTACAGACAAATCCTCCAGCACAG
CCTACATGCAACTGAGCAGCCTGACATCTGAGGACTCTGCAGTCTATTACTGTGCAAGAT
ATTATGATGATCATTACTGCCTTGACTACTGGGGCCAAGGCACCACGGTGACCGTGAGCG
CCGGGGGAGGTGGCAGCGGGGGAGGTGGCAGCGGCGGCGGGAGCTCCCAAATTGTTC
TCACCCAGTCTCCAGCAATCATGTCTGCATCTCCAGGGGAGAAGGTCACCATGACCTGC
AGTGCCAGCTCAAGTGTAAGTTACATGAACTGGTACCAGCAGAAGTCAGGCACCTCCCC
CAAAAGATGGATTTATGACACATCCAAACTGGCTTCTGGAGTCCCTGCTCACTTCAGGG
 
GCAGTGGGTCTGGGACCTCTTACTCTCACAATCAGCGGCATGGAGGCTGAAGATGCT
GCCACTTATTACTGCCAGCAGTGGAGTAGTAACCCATTCACGTTCGGCTCGGGGACAAA
GTTGGAAATAAACCGGGCGGTGGGGATCCC
SEQ ID NO: 8, heavy chain amino acid sequence of OKT3 
QVQLQQSGAELARPGASVKMSCKASGYTFTRYTMHWVKQRPGQGLEWIGYINPSRGYTN
YNQKFKDKATLTTDKSSSTAYMQLSSLTSEDSAVYYCARYYDDHYCLDYWGQGTTVTVSA
SEQ ID NO: 9, light chain amino acid sequence of OKT3 
QIVLTQSPAIMSASPGEKVTMTCSASSSVSYMNWYQQKSGTSPKRWIYDTSKLASGVPAHF
RGSGSGTSYSLTISGMEAEDAATYYCQQWSSNPFTFGSGTKLEINRGGGDP
SEQ ID NO: 10, nucleic acid sequence of FcyRIIIa 158V FL cDNA 
ATGTGGCAGCTGCTCCTCCCAACTGCTCTGCTACTTCTAGTTTCAGCTGGCATGCGGACT
GAAGATCTCCCAAAGGCTGTGGTGTTCCTGGAGCCTCAATGGTACAGGGTGCTCGAGAA
GGACAGTGTGACTCTGAAGTGCCAGGGAGCCTACTCCCCTGAGGACAATTCCACACAG
TGGTTTCACAATGAGAGCCTCATCTCAAGCCAGGCCTCGAGCTACTTCATTGACGCTGC
CACAGTCGACGACAGTGGAGAGTACAGGTGCCAGACAAACCTCTCCACCCTCAGTGAC
CCGGTGCAGCTAGAAGTCCATATCGGCTGGCTGTTGCTCCAGGCCCCTCGGTGGGTGTT
CAAGGAGGAAGACCCTATTCACCTGAGGTGTCACAGCTGGAAGAACACTGCTCTGCAT
AAGGTCACATATTTACAGAATGGCAAAGGCAGGAAGTATTTCATCATAATTCTGACTTC
TACATTCCAAAAGCCACACTCAAAGACAGCGGCTCCTACTTCTGCAGGGGGCTTGTTGG
GAGTAAAAATGTGTCTTCAGAGACTGTGAACATCACCATCACTCAAGGTTTGGCAGTGT
CAACCATCTCATCATTCTTTCCACCTGGGTACCAAGTCTCTTTCTGCTTGGTGATGGTACT
CCTTTTTGCAGTGGACACAGGACTATATTTCTCTGTGAAGACAAACATTCGAAGCTCAA
CAAGAGACTGGAAGGACCATAAATTTAAATGGAGAAAGGACCCTCAAGACAAATGA
SEQ ID NO: 11, amino acid sequence of FcyRIIIa 158V FL 
MWQLLLPTALLLLVSAGMRTEDLPKAVVFLEPQWYRVLEKDSVTLKCQGAYSPEDNSTQW
FHNESLISSQASSYFIDAATVDDSGEYRCQTNLSTLSDPVQLEVHIGWLLLQAPRWVFKEED
PIHLRCHSWKNTALHKVTYLQNGKGRKYFHHNSDFYIPKATLKDSGSYFCRGLVGSKNVSS
ETVNITITQGLAVSTISSFFPPGYQVSFCLVMVLLFAVDTGLYFSVKTNIRSSTRDWKDHKFK
WRKDPQDK
SEQ ID NO: 12, amino acid sequence of heavy chain CDR1 of anti-CTLA-4 antibody
   SYTMH
SEQ ID NO: 13, amino acid sequence of heavy chain CDR2 of anti-CTLA-4 antibody
   FISYDGNNKYYADSVKG
SEQ ID NO: 14, amino acid sequence of heavy chain CDR3 of anti-CTLA-4 antibody
   TGWLGPFDY
SEQ ID NO: 15, amino acid sequence of light chain CDR1 of anti-CTLA-4 antibody
   RASQSVGSSYLA
SEQ ID NO: 16, amino acid sequence of light chain CDR2 of anti-CTLA-4 antibody
   GAFSRAT
SEQ ID NO: 17, amino acid sequence of light chain CDR3 of anti-CTLA-4 antibody
   QQYGSSPWT
SEQ ID NO: 18, amino acid sequence of heavy chain variable region of anti-CTLA-4 antibody 
QVQLVESGGGVVQPGRSLRLSCAASGFTFSSYTMHWVRQAPGKGLEWVTFISYDGNNKY
YADSVKGRFTISRDNSKNTLYLQMNSLRAEDTAIYYCARTGWLGPFDYWGQGTLVTVSS
SEQ ID NO: 19, amino acid sequence of light chain variable region of anti-CTLA-4 antibody 
EIVLTQSPGTLSLSPGERATLSCRASQSVGSSYLAWYQQKPGQAPRLLIYGAFSRATGIPDRF
SGSGSGTDFTLTISRLEPEDFAVYYCQQYGSSPWTFGQGTKVEIK
SEQ ID NO: 20, forward primer of FcγRIIIa 158V FL cDNA (the underlined part is a restriction endonuclease cutting site)
   5'-GCGAATTCATGTGGCAGCTGCTCCTCCC-3'
SEQ ID NO: 21, reverse primer of FcγRIIIa 158V FL cDNA (the underlined part is a restriction endonuclease cutting site)
   5'- CAGCGGCCGCTCATTTGTCTTGAGGGTCCTTTCTC-3'

## Claims

1. A monoclonal antibody against CTLA-4, comprising:
(1) a heavy chain HCDR1 comprising or consisting of an amino acid sequence set forth in SEQ ID NO: 12,
a heavy chain HCDR2 comprising or consisting of an amino acid sequence set forth in SEQ ID NO: 13,
a heavy chain HCDR3 comprising or consisting of an amino acid sequence set forth in SEQ ID NO: 14,
a light chain LCDR1 comprising or consisting of an amino acid sequence set forth in SEQ ID NO: 15,
a light chain LCDR2 comprising or consisting of an amino acid sequence set forth in SEQ ID NO: 16, and
a light chain LCDR3 comprising or consisting of an amino acid sequence set forth in SEQ ID NO: 17;
or
(2) a heavy chain variable region comprising or consisting of an amino acid sequence set forth in SEQ ID NO: 18, an amino acid sequence having at least 80%, 90%, preferably 91%, 92%, 93%, 94%, 95%, 96%, 97%, 98% or 99% sequence identity to SEQ ID NO: 18, or an amino acid sequence having one or more conservative amino acid mutations compared to SEQ ID NO: 18, and
a light chain variable region comprising or consisting of an amino acid sequence set forth in SEQ ID NO: 19, an amino acid sequence having at least 80%, 90%, preferably 91%, 92%, 93%, 94%, 95%, 96%, 97%, 98% or 99% sequence identity to SEQ ID NO: 19, or an amino acid sequence having one or more conservative amino acid mutations compared to SEQ ID NO: 19;
wherein an Fc region of the monoclonal antibody has high mannose glycoforms with a total amount of < 5% and/or sialylated glycoforms with a total amount of < 3%.

2. The monoclonal antibody according to claim 1 comprising a heavy chain and a light chain, wherein the heavy chain comprises or consists of an amino acid sequence set forth in SEQ ID NO: 1, or an amino acid sequence having at least 80%, at least 81%, 82%, 83%, 84%, 85%, 86%, 87%, 88%, 89%, 90%, 91%, 92%, 93%, 94%, 95%, 96%, 97%, 98% or 99% homology to the amino acid sequence set forth in SEQ ID NO: 1;
the light chain comprises or consists of an amino acid sequence set forth in SEQ ID NO: 2, or an amino acid sequence having at least 80%, at least 81%, 82%, 83%, 84%, 85%, 86%, 87%, 88%, 89%, 90%, 91%, 92%, 93%, 94%, 95%, 96%, 97%, 98% or 99% homology to the amino acid sequence set forth in SEQ ID NO: 2.

3. The monoclonal antibody according to claim 1 or 2, wherein the heavy chain is encoded by a nucleotide sequence set forth in SEQ ID NO: 3 or a nucleotide sequence having at least 80%, at least 81%, 82%, 83%, 84%, 85%, 86%, 87%, 88%, 89%, 90%, 91%, 92%, 93%, 94%, 95%, 96%, 97%, 98% or 99% homology to the nucleotide sequence set forth in SEQ ID NO: 3, and the light chain is encoded by a nucleotide sequence set forth in SEQ ID NO: 4 or a nucleotide sequence having at least 80%, at least 81%, 82%, 83%, 84%, 85%, 86%, 87%, 88%, 89%, 90%, 91%, 92%, 93%, 94%, 95%, 96%, 97%, 98% or 99% homology to the nucleotide sequence set forth in SEQ ID NO: 4.

4. The monoclonal antibody according to any one of claims 1-3, wherein the Fc region of the monoclonal antibody has a fucosylation level of 0%-5%.

5. The monoclonal antibody according to any one of claims 1-4, wherein the monoclonal antibody is expressed by a cell in which an α-(1,6)-fucosytransferase gene is knocked out.

6. A pharmaceutical composition or fusion protein comprising the monoclonal antibody according to any one of claims 1-5.

7. A multispecific antibody (e.g., a bispecific antibody) comprising the monoclonal antibody according to any one of claims 1-5, and an antibody or an antigen-binding fragment against another antigen and/or another antigenic epitope.

8. A kit comprising the monoclonal antibody according to any one of claims 1-5, the multispecific antibody according to claim 7 or the fusion protein according to claim 6.

9. The kit according to claim 8, wherein the kit further comprises a second antibody, a cytotoxic agent, a chemotherapeutic agent or a radiotherapeutic agent, wherein the second antibody specifically recognizes the monoclonal antibody; optionally, the second antibody further comprises a detectable label, such as fluorescein, a metal ion, biotin, a radioisotope, a chemiluminescent substance, an enzyme or polyethylene glycol.

10. Use of the monoclonal antibody according to any one of claims 1-5, the fusion protein according to claim 6 or the multispecific antibody according to claim 7 in the preparation of a medicament for treating a cancer or for rejecting a transplantable tumor, such as melanoma, non-small cell lung cancer, bladder cancer, liver cancer, colon cancer, prostate cancer, lymphoma or renal cancer.

11. The use according to claim 10, wherein the medicament is formulated for administration of the anti-CTLA-4 antibody at a dose of 7 mg to 180 mg in each treatment cycle.

12. The use according to claim 11, wherein one treatment cycle is 1 week, 2 weeks, 3 weeks or 4 weeks.

13. The use according to claim 10, wherein the medicament is formulated for administration of 0.1 mg/kg to 2.5 mg/kg of the anti-CTLA-4 antibody per dose.

14. The use according to claim 13, wherein the administration is performed once every 1 week, 2 weeks, 3 weeks or 4 weeks.

15. The use according to any one of claims 10-14, wherein the medicament is formulated for intravenous injection or subcutaneous injection.

16. A liquid formulation comprising the following components:
(1) 5-40 mg/mL anti-CTLA-4 antibody or fragment thereof,
(2) 10-30 mM buffer,
(3) 200-260 mM stabilizer,
(4) 0.02-0.2 mg/mL chelating agent,
(5) 0.1-0.4 mg/mL surfactant, and
(6) water;
wherein the liquid formulation has a pH value of 5.4-6.2.

17. The formulation according to claim 16, wherein the buffer is selected from at least one of a histidine buffer and an acetate buffer, the stabilizer is selected from at least one of trehalose, sucrose and mannitol, the chelating agent comprises disodium edetate, and the surfactant is selected from at least one of polysorbate 20 and polysorbate 80.

18. The liquid formulation according to claim 16 or 17, wherein the anti-CTLA-4 antibody or fragment thereof comprises HCDR1 set forth in SEQ ID NO: 12, HCDR2 set forth in SEQ ID NO: 13, HCDR3 set forth in SEQ ID NO: 14, LCDR1 set forth in SEQ ID NO: 15, LCDR2 set forth in SEQ ID NO: 16 and LCDR3 set forth in SEQ ID NO: 17; or
wherein a heavy chain variable region of the anti-CTLA-4 antibody or fragment thereof comprises an amino acid sequence set forth in SEQ ID NO: 18 or an amino acid sequence having at least about 90% identity to the sequence set forth in the SEQ ID NO: 18, and a light chain variable region of the anti-CTLA-4 antibody or fragment thereof comprises an amino acid sequence set forth in SEQ ID NO: 19 or an amino acid sequence having at least about 90% identity to the sequence set forth in the SEQ ID NO: 19.

19. The formulation according to any one of claims 16-18, wherein a heavy chain of the anti-CTLA-4 antibody comprises an amino acid sequence sequence set forth in SEQ ID NO: 1, a sequence having at least 80% identity to the sequence set forth in SEQ ID NO: 1, or an amino acid sequence having one or more conservative amino acid substitutions compared to the sequence set forth in SEQ ID NO: 1; and/or
a light chain of the anti-CTLA-4 antibody comprises an amino acid sequence sequence set forth in SEQ ID NO: 2, a sequence having at least 80% identity to the sequence set forth in SEQ ID NO: 2, or an amino acid sequence having one or more conservative amino acid substitutions compared to the sequence set forth in SEQ ID NO: 2.

20. The formulation according to any one of claims 16-19, wherein the anti-CTLA-4 antibody or fragment thereof is a monoclonal antibody, and the anti-CTLA-4 antibody or fragment thereof has a fucosylation level of < 5%, high mannose glycoforms with a total amount of < 5%, and sialylated glycoforms with a total amount of < 3%.

21. The formulation according to any one of claims 16-20, wherein the anti-CTLA-4 antibody is expressed by a cell in which an α-(1,6)-fucosytransferase gene is knocked out.

22. The liquid formulation according to any one of claims 16-21, wherein the liquid formulation comprises 7-15 mg/mL anti-CTLA-4 antibody or fragment thereof.

23. The liquid formulation according to any one of claims 16-22, wherein the liquid formulation comprises 17-23 mM histidine buffer.

24. The liquid formulation according to any one of claims 16-23, wherein the liquid formulation comprises 220-250 mM sucrose.

25. The liquid formulation according to any one of claims 16-24, wherein the liquid formulation comprises 0.02-0.05 mg/mL disodium edetate.

26. The liquid formulation according to any one of claims 16-25, wherein the liquid formulation comprises 0.1-0.3 mg/mL polysorbate 80.

27. A liquid formulation comprising the following components:
(1) 10 mg/mL anti-CTLA-4 antibody or fragment thereof,
(2) 20 mM histidine buffer,
(3) 240 mM sucrose,
(4) 0.033 mg/mL disodium edetate,
(5) 0.2 mg/mL polysorbate 80, and
(6) water;
wherein the liquid formulation has a pH value of 5.8.

28. A method for treating a tumor or cancer, comprising: administering to a patient in need thereof an effective amount of an anti-CTLA-4 antibody;
wherein the anti-CTLA-4 antibody comprises HCDR1 set forth in SEQ ID NO: 12, HCDR2 set forth in SEQ ID NO: 13, HCDR3 set forth in SEQ ID NO: 14, LCDR1 set forth in SEQ ID NO: 15, LCDR2 set forth in SEQ ID NO: 16 and LCDR3 set forth in SEQ ID NO: 17.

29. The method according to claim 28, wherein a heavy chain of the anti-CTLA-4 antibody comprises an amino acid sequence sequence set forth in SEQ ID NO: 1, an amino acid sequence sequence having at least 80% identity to the sequence set forth in SEQ ID NO: 1, or an amino acid sequence having one or more conservative amino acid substitutions compared to the sequence set forth in SEQ ID NO: 1; and/or
a light chain of the anti-CTLA-4 antibody comprises an amino acid sequence sequence set forth in SEQ ID NO: 2, an amino acid sequence sequence having at least 80% identity to the sequence set forth in SEQ ID NO: 2, or an amino acid sequence having one or more conservative amino acid substitutions compared to the sequence set forth in SEQ ID NO: 2.

30. The method according to claim 28 or 29, wherein the anti-CTLA-4 antibody has a fucosylation level of 0%-10%.

31. The method according to claim 28 or 29, wherein the anti-CTLA-4 antibody is expressed by a cell in which an α-(1,6)-fucosyltransferase gene is knocked out.

32. The method according to any one of claims 28-31, wherein the tumor or cancer comprises acute lymphocytic leukemia, acute myelogenous leukemia, chronic lymphocytic leukemia, chronic myelogenous leukemia, myeloproliferative disorder/tumor, Hodgkin's lymphoma, indolent and aggressive non-Hodgkin's lymphoma, Burkitt's lymphoma, follicular lymphoma, multiple myeloma, giant cell myeloma, heavy chain myeloma, light chain or Bense-Jones myeloma, breast cancer, ovarian cancer, pancreatic cancer, prostate cancer, melanoma, colorectal cancer, lung cancer, head and neck cancer, bladder cancer, esophageal cancer, liver cancer and renal cancer.

33. The method according to any one of claims 28-31, wherein the effective amount of the anti-CTLA-4 antibody administered in each treatment cycle is 7 mg to 180 mg.

34. The method according to any one of claims 28-31, wherein one treatment cycle is 1 week, 2 weeks, 3 weeks or 4 weeks.

35. The method according to any one of claims 28-31, wherein a dose of the anti-CTLA-4 antibody for each administration is 0.1 mg/kg to 2.5 mg/kg.

36. The method according to any one of claims 28-31, wherein an administration route is intravenous injection or subcutaneous injection.
